# EUROPEAN PATENT APPLICATION

(11) **EP 4 620 390 A1**
(43) Date of publication of application: **24.09.2025**
(21) Application number: 23912081.9
(22) Date of filing: 25.12.2023
(51) Int. Cl.: A61B 5/11, A61B 5/00, A61B 5/08, A61B 5/16, A61B 5/107, A61B 5/113, A61B 5/0245, A61B 5/372

(54) **HEALTH RISK DETERMINATION SYSTEM, AUTONOMIC NERVE DETERMINATION SYSTEM, LIFE IMPROVEMENT SYSTEM, SLEEPING POSTURE DETERMINATION SYSTEM, SLEEPING POSTURE DETERMINATION PROGRAM, BRUXISM DETECTION SYSTEM, AND BRUXISM DETECTION PROGRAM**

(30) Priority: 28.12.2022 JP 2022212408; 16.01.2023 JP 2023004346; 15.02.2023 JP 2023021847; 16.03.2023 JP 2023042106; 23.03.2023 JP 2023046741
(71) Applicant: NISHIKAWA CO., LTD., Tokyo 103-0006 (JP)
(72) Inventor: NISHIKAWA, Yasuyuki, Tokyo 103-0006 (JP); NONOMURA, Takuto, Tokyo 103-0006 (JP); SHIMADA, Saki, Tokyo 103-0006 (JP); NISHINA, Midori, Tokyo 103-0006 (JP); AOKI, Mari, Tokyo 103-0006 (JP)
(74) Representative: Viering, Jentschura & Partner mbB Patent- und Rechtsanwälte
(86) International application number: PCT/JP2023/046510
(87) International publication number: WO 2024/143302

(57) **Abstract**

As one example, a health risk determination system includes a sensor unit that acquires breathing information, which is information indicating a breathing of a user, without coming into contact with the user; an abnormality detection unit that detects an abnormal breathing state of the user from the breathing information; a unit count acquisition unit that acquires the number of detections of the abnormal breathing state for each unit time period having a predetermined time span between a sleep onset time and an awakening time; an average count acquisition unit that acquires an average detection count that is a value obtained by dividing a total value of the number of detections of the abnormal breathing state between the sleep onset time and the awakening time by a time from the sleep onset time to the awakening time; a maximum count acquisition unit that acquires a maximum detection count that is a maximum value among a plurality of numbers of detections acquired for each unit time period; and a health risk determination unit that determines a health risk based on both the average detection count and the maximum detection count.

## Description

### Technical Field

The present disclosure relates to a health risk determination system, an autonomic nervous system determination system, a life improvement system, a sleeping posture determination system, a sleeping posture determination program, a bruxism detection system, and a bruxism detection program.

Priority is claimed on Japanese Patent Application No. 2022-212408, filed on December 28, 2022, Japanese Patent Application No. 2023-004346, filed on January 16, 2023, Japanese Patent Application No. 2023-021847, filed on February 15, 2023, Japanese Patent Application No. 2023-042106, filed on March 16, 2023, and Japanese Patent Application No. 2023-046741, filed on March 23, 2023, the entire contents of which are incorporated herein by reference.

### Background Art

Patent Literature 1 discloses an information processing device indicating the possibility of a blood pressure variability caused by sleep apnea syndrome. The information processing device includes a measurement terminal and a sensor that are worn on the body of a subject to acquire predetermined measurement data from the subject.

Patent Literature 2 discloses a wearable device worn on the wrist of a user. The wearable device includes a pulse sensor that calculates a pulse rate as biological information. The activity state of the autonomic nervous system is calculated based on the pulse wave signal detected by the pulse sensor.

Patent Literature 3 describes a sleep state detection system that detects the sleep state of a human. The sleep state detection system includes a radio wave transmitting and receiving device that emits microwaves toward the human body during sleep and receives reflected waves of the microwaves affected by the body pulsations of the human body, and a computer that detects the sleep state of the human by analyzing the reflected wave data of the reflected waves. The computer performs operation and stop control of an air conditioning device, a lighting device, and an audio device based on the detected sleep state.

Patent Literature 4 describes a biological information display device. The biological information display device includes a sensor sheet having a rectangular shape and a control unit attached to an end portion of the sensor sheet. The biological information display device is used while being laid on a bed. The bed is composed of a placement portion for placing laying bedding, and a backboard portion that stands upright from an end portion of the placement portion, and the biological information display device is used while being inserted under the laying bedding placed on the placement portion.

Patent Literature 5 describes a bruxism prevention device including a device body that is worn in the oral cavity. The device body includes a piezoelectric sensor that detects the state of contact between the upper teeth and the lower teeth. The bruxism prevention device includes control means for determining bruxism based on a signal from the piezoelectric sensor.

Patent Literature 6 describes a biological information detection device including vibration signal sensing means for detecting body vibration emitted by an animal. The vibration signal sensing means is composed of a vibration sensor body and at least one of a bottom plate, a cushion member, and a vibration collecting plate that are laminated above and below the vibration sensor body. The animal and the vibration sensor body are not in contact with each other. The biological information detection device separates the sound of bruxism from the signal detected by the vibration signal sensing means. More specifically, a signal from the human body is detected by the vibration signal sensing means. Then, a step of pre-processing the detected signal and a step of filtering the pre-processed signal to separate the detected signal into a plurality of signals and obtain at least two of breathing vibration, heartbeat vibration, snoring, and body movement signals are executed. In the biological information detection device, the snoring is considered to include generating sounds such as bruxism, sneezing, and sleep talking.

### Citation List

### Patent Literature

Patent Literature 1: Japanese Unexamined Patent Publication No. 2018-149173
Patent Literature 2: Japanese Unexamined Patent Publication No. 2018-543
Patent Literature 3: Japanese Unexamined Patent Publication No. 2006-87850
Patent Literature 4: Japanese Patent No. 3960298
Patent Literature 5 Japanese Unexamined Patent Publication No. 2020-75018
Patent Literature 6: Japanese Unexamined Patent Publication No. 2019-673

### Summary of Invention

### Technical Problem

In the information processing device described above, the measurement terminal and the sensor acquire measurement data from the subject in a state where the measurement terminal and the sensor are in contact with or in close proximity to a part of the body of the subject. For this reason, an object such as a sensor may be in contact with the body for a long period of time, and therefore, there is a possibility that a significant burden is placed on the subject. The burden is particularly noticeable when an attempt is made to acquire measurement data from the subject during sleep. In determining a health risk such as sleep apnea syndrome, it is required to accurately identify the health risk of the subject.

In determining the state of the autonomic nervous system during sleep, it may not be necessarily appropriate to use the same criteria as the criteria for determining the autonomic nervous system during wakefulness. For example, during wakefulness, it is desirable to have a good balance between the sympathetic nervous system and the parasympathetic nervous system. Meanwhile, during sleep, the ideal state of the autonomic nervous system may differ depending on the sleep state. Therefore, it is desirable to be able to determine the state of the autonomic nervous system according to the sleep state.

In the sleep state detection system described above, the operation and stop control of the air conditioning device, the lighting device, and the audio device is performed according to the sleep state from the time the user goes to bed until the user wakes up. However, in order to improve the life of the user, it may be required to predict the state of the user during wakefulness after the user wakes up, and provide information for improving the life of the user.

In the biological information display device described above, body position information and time-series data of a sleeping posture are displayed on a display. However, merely displaying the body position information and the sleeping posture allows a user to identify the sleeping posture, but does not allow the user to identify how to improve the sleeping posture. Therefore, it may be required to provide information that enables the sleeping posture to be improved. Furthermore, it may be required to improve the sleeping posture so as to improve the life of the user.

In the bruxism prevention device described above, in order to detect bruxism, the device body needs to be worn in the oral cavity. Therefore, particularly during sleep, having the device body inside the oral cavity places a significant burden on the subject, which is a concern.

In the biological information detection device described above, the signal from the human body is separated into the plurality of signals through pre-processing and filtering, and the separated snoring signal includes bruxism, along with sounds such as sneezing and sleep talking. Therefore, it cannot be determined whether the detected signal from the human body is caused by bruxism, sneezing, or sleep talking, which is a concern. Therefore, there is room for improvement in terms of the accuracy of detecting bruxism.

An object of the present disclosure is to provide a health risk determination system capable of accurately identifying a health risk while reducing burden, an autonomic nervous system determination system capable of determining the state of the autonomic nervous system according to a sleep state, a life improvement system capable of predicting the state of a user during wakefulness and providing information for improving the life of the user, a sleeping posture determination system and a sleeping posture determination program capable of encouraging an improvement in sleeping posture and providing useful information capable of improving the life, and a bruxism detection system and a bruxism detection program capable of detecting bruxism with high accuracy while reducing the burden on the user.

### Solution to Problem

(1) A health risk determination system according to the present disclosure is a health risk determination system that determines a health risk caused by an abnormal breathing state of a user from a sleep onset time to an awakening time. The health risk determination system includes a sensor unit that acquires breathing information, which is information indicating a breathing of the user, without coming into contact with the user; an abnormality detection unit that detects the abnormal breathing state of the user from the breathing information; a unit count acquisition unit that acquires the number of detections of the abnormal breathing state for each unit time period having a predetermined time span between the sleep onset time and the awakening time; an average count acquisition unit that acquires an average detection count that is a value obtained by dividing a total value of the number of detections of the abnormal breathing state between the sleep onset time and the awakening time by a time from the sleep onset time to the awakening time; a maximum count acquisition unit that acquires a maximum detection count that is a maximum value among a plurality of numbers of detections acquired for each unit time period; and a health risk determination unit that determines the health risk of the user based on both the average detection count and the maximum detection count.
(2) An autonomic nervous system determination system according to the present disclosure is an autonomic nervous system determination system that determines a state of an autonomic nervous system of a user from a bedtime to a wake-up time. The autonomic nervous system determination system includes a sensor unit that acquires at least one of breathing information that is information indicating a breathing of the user, body movement information that is information indicating a body movement of the user, and brainwave information that is information indicating a brainwave of the user, and heartbeat information that is information indicating a heartbeat of the user; an autonomic nervous system acquisition unit that acquires autonomic nervous system information, which is information indicating the state of the autonomic nervous system of the user, from the heartbeat information; a sleep state acquisition unit that acquires a sleep state of the user from at least one of the breathing information, the body movement information, the heartbeat information, and the brainwave information; and an autonomic nervous system determination unit that determines the state of the autonomic nervous system of the user based on both the autonomic nervous system information and the sleep state.
(3) A life improvement system according to one aspect of the present disclosure is a life improvement system for improving a life of a user. The life improvement system includes a sensor unit that acquires at least one of breathing information that is information indicating a breathing of the user, body movement information that is information indicating a body movement of the user, and heartbeat information that is information indicating a heartbeat of the user; a sleep state acquisition unit that acquires sleep state information, which is information indicating a sleep state of the user, from at least one of the breathing information, the body movement information, and the heartbeat information; an autonomic nervous system acquisition unit that acquires autonomic nervous system information, which is information indicating a state of an autonomic nervous system of the user, from the heartbeat information; a device control unit that controls an operation of a device constituting an environment around the user, based on at least one of past sleep information, which is the sleep state information of the user in the past stored in advance, and the autonomic nervous system information; a prediction information generation unit that generates prediction information that is information indicating a predicted state of the user during wakefulness, based on at least one of the past sleep information and the autonomic nervous system information; and an improvement information generation unit that generates improvement information that is information that improves the life of the user, based on the prediction information.
(4) A life improvement system according to another aspect of the present disclosure includes a sensor unit that acquires heartbeat information that is information indicating a heartbeat of a user; an autonomic nervous system acquisition unit that acquires autonomic nervous system information, which is information indicating a state of an autonomic nervous system of the user, from the heartbeat information; a prediction information generation unit that generates prediction information that is information indicating a predicted state of the user during wakefulness, based on the autonomic nervous system information; and an improvement information generation unit that generates improvement information that is information that improves a life of the user, based on the prediction information.
(5) A sleeping posture determination system according to the present disclosure includes a sensor unit that is mounted on bedding and that receives a load of a body of a user of the bedding placed on the bedding and outputs a waveform corresponding to the load; a sleeping posture determination unit that determines a sleeping posture of the user based on the waveform output by the sensor unit; and an advice generation unit that generates advice for improving the sleeping posture of the user based on the sleeping posture determined by the sleeping posture determination unit.
(6) There is provided a non-transitory computer-readable storage medium storing a sleeping posture determination program according to the present disclosure, the program causing a computer to execute: a step of determining a sleeping posture of a user of bedding based on a waveform indicating a load of the user output by a sensor unit mounted on the bedding; and a step of generating advice for improving the sleeping posture of the user based on the sleeping posture determined by the step of determining.
(7) A bruxism detection system according to the present disclosure is a bruxism detection system that detects bruxism of a user from a sleep onset time to an awakening time. The bruxism detection system includes a sensor unit that acquires vibration information, which is information indicating vibration of the user, without coming into contact with the user; and a detection unit that extracts sign information indicating a sign occurring before on an onset of the bruxism from the vibration information, and that detects the bruxism from the vibration information and the sign information.
(8) There is provided a non-transitory computer-readable storage medium storing a bruxism detection program according to the present disclosure, the program causing a computer to execute: a step of extracting sign information indicating a sign occurring before an onset of bruxism from vibration information indicating vibration of a user acquired by a sensor unit; and a step of detecting the bruxism from the vibration information and the sign information.

### Advantageous Effects of Invention

According to the present disclosure, it is possible to provide the health risk determination system capable of accurately identifying a health risk while reducing burden, the autonomic nervous system determination system capable of determining the state of the autonomic nervous system according to a sleep state, the life improvement system capable of predicting the state of the user during wakefulness and providing the information for improving the life of the user, the sleeping posture determination system and the sleeping posture determination program capable of encouraging an improvement in sleeping posture and providing useful information capable of improving the life, and the bruxism detection system and the bruxism detection program capable of detecting bruxism with high accuracy while reducing the burden on the user.

### Brief Description of Drawings

[FIG. 1] FIG. 1 is a block diagram showing an example of a health risk determination system.
[FIG. 2] (a) in FIG. 2 is a perspective view showing a cushion body to which a sensor unit is attached. (b) in FIG. 2 is a perspective view showing a core material constituting the cushion body shown in (a) of FIG. 2.
[FIG. 3] FIG. 3 is a flowchart showing one example of the operation of the health risk determination system.
[FIG. 4] FIG. 4 is a graph showing one example of time-series data of the number of detections of an abnormal breathing state.
[FIG. 5] FIG. 5 is a view showing one example of criteria for determining a health risk.
[FIG. 6] FIG. 6 is a view showing one example of a display of health risk.
[FIG. 7] FIG. 7 is a block diagram showing an example of an autonomic nervous system determination system.
[FIG. 8] (a) in FIG. 8 is a perspective view showing a cushion body to which a sensor unit is attached. (b) in FIG. 8 is a perspective view showing a core material constituting the cushion body shown in (a) of FIG. 8.
[FIG. 9] FIG. 9 is a flowchart showing one example of the operation of the autonomic nervous system determination system.
[FIG. 10] FIG. 10 is a table showing one example of a support database stored in a server.
[FIG. 11] FIG. 11 is a view showing one example of a display of the determination result of the autonomic nervous system at sleep onset, and one example of a display of the determination result of the autonomic nervous system upon waking up.
[FIG. 12] FIG. 12 is a view showing another example of a display of the determination result of the autonomic nervous system.
[FIG. 13] FIG. 13 is a table showing one example of an autonomic nervous system database stored in a server.
[FIG. 14] FIG. 14 is a block diagram showing an example of a life improvement system.
[FIG. 15] FIG. 15 is a view showing an example of an input screen of subjective information that is displayed on an information terminal shown in FIG. 14.
[FIG. 16] (a) in FIG. 16 is a perspective view showing a cushion body to which a sensor sheet is attached. (b) in FIG. 16 is a perspective view showing a core material constituting the cushion body shown in (a) of FIG. 16.
[FIG. 17] FIG. 17 is a perspective view showing an example of a core material of a cushion to which a cushion sensor is attached.
[FIG. 18] FIG. 18 is a view showing an example of an output screen of prediction information and improvement information that are displayed on the information terminal shown in FIG. 14.
[FIG. 19] FIG. 19 is a view showing an example of an output screen of feedback information that is displayed on the information terminal shown in FIG. 14.
[FIG. 20] FIG. 20 is a flowchart showing one example of the operation of the life improvement system.
[FIG. 21] (a) in FIG. 21 is a perspective view showing an example of bedding and a sensor unit. (b) in FIG. 21 is a perspective view showing a core material of the bedding in (a) of FIG. 21.
[FIG. 22] FIG. 22 is a plan view showing the sensor unit in (a) of FIG. 21.
[FIG. 23] FIG. 23 is a perspective view showing an example of an attachment structure of the sensor unit to the bedding in (a) of FIG. 21.
[FIG. 24] FIG. 24 is a perspective view showing a detailed structure of the sensor unit in FIG. 22.
[FIG. 25] FIG. 25 is a block diagram showing an example of a sleeping posture determination system and a sleeping posture determination program.
[FIG. 26] (a), (b), and (c) in FIG. 26 are views showing examples of waveforms acquired by the sensor unit in (a) of FIG. 21.
[FIG. 27] FIG. 27 is a view showing an example of a screen displayed by a display unit of the sleeping posture determination system in FIG. 25.
[FIG. 28] FIG. 28 is a view showing an example of a screen displayed by the display unit of the sleeping posture determination system in FIG. 25.
[FIG. 29] FIG. 29 is a view showing an example of a screen displayed by the display unit of the sleeping posture determination system in FIG. 25.
[FIG. 30] FIG. 30 is a view showing an example of a screen displayed by the display unit of the sleeping posture determination system in FIG. 25.
[FIG. 31] FIG. 31 is a view showing an example of a screen displayed by the display unit of the sleeping posture determination system in FIG. 25.
[FIG. 32] FIG. 32 is a view showing an example of a screen displayed by the display unit of the sleeping posture determination system in FIG. 25.
[FIG. 33] FIG. 33 is a view showing an example of a screen displayed by the display unit of the sleeping posture determination system in FIG. 25.
[FIG. 34] FIG. 34 is a block diagram showing an example of a bruxism detection system.
[FIG. 35] (a) in FIG. 35 is a perspective view showing a cushion body to which a sensor unit is attached. (b) in FIG. 35 is a perspective view showing a core material constituting the cushion body shown in (a) of FIG. 35.
[FIG. 36] (a) in FIG. 36 is a schematic waveform diagram of bruxism. (b) in FIG. 36 is a schematic waveform diagram of turning over during sleep. (c) in FIG. 36 is a schematic waveform diagram at rest.
[FIG. 37] FIG. 37 is a schematic waveform diagram of a waveform indicating sign information caused by an increase in heart rate before the onset of bruxism.
[FIG. 38] FIG. 38 is a schematic view showing one example of criteria for the level of occurrence frequency of bruxism.
[FIG. 39] FIG. 39 is a flowchart showing one example of the operation of the bruxism detection system.

### Description of Embodiments

Hereinafter, specific examples of a health risk determination system, an autonomic nervous system determination system, a life improvement system, a sleeping posture determination system, a sleeping posture determination program, a bruxism detection system, and a bruxism detection program according to the present disclosure will be described with reference to the drawings. The health risk determination system, the autonomic nervous system determination system, the life improvement system, the sleeping posture determination system, the sleeping posture determination program, the bruxism detection system, and the bruxism detection program can be combined with each other. In the description of the drawings, the same or corresponding elements are denoted by the same reference signs, and duplicate descriptions will be omitted as appropriate. For ease of understanding, the drawings may be depicted in a partially simplified or exaggerated manner, and dimensional ratios and the like are not limited to those shown in the drawings.

First, an example of the health risk determination system will be described. As one example, the health risk determination system determines a health risk caused by the abnormal breathing state of a user. The health risk determination system determines the health risk of the user from sleep onset time to awakening time. For example, the health risk determination system may be used for personal or household purposes, or may be used in research institutes or the like for testing and research purposes. In addition, the health risk determination system may be used in hospitals or the like for treatment. The "user" refers to a person who is the subject of health risk determination by the health risk determination system. The user is, for example, a person who has a health risk caused by an abnormal breathing state, a person who receives sleep treatment at a hospital or the like, or a person who wishes to determine his or her own health risk.

The "health risk" refers to, for example, a risk such as heart failure associated with sleep apnea syndrome (SAS). The "abnormal breathing state" refers to the state of breathing during sleep that has the possibility of posing a health risk to the user. The abnormal breathing state includes, for example, an apnea state and a hypopnea state. The "apnea state" refers to, for example, a state where the breathing of the user stops for 10 seconds or more. The "hypopnea state" refers to a state where a state where the amplitude of an airflow in the breathing of the user decreases by 30% or more and the arterial oxygen saturation (SpO₂) decreases by 4% or more lasts for 10 seconds or more.

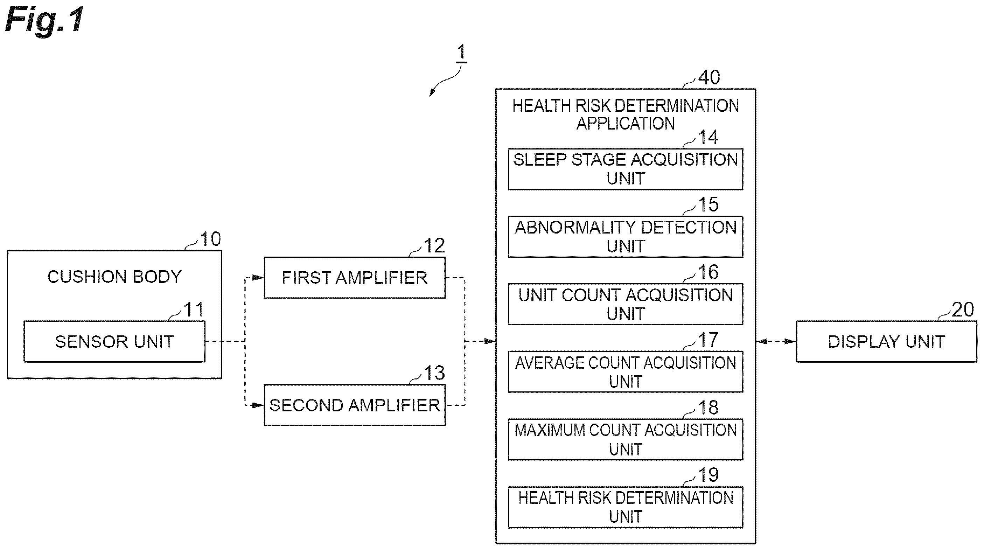

FIG. 1 is a block diagram showing a health risk determination system 1 as one example. The health risk determination system 1 is accessible, for example, from an information terminal such as a computer, a tablet terminal, a smartphone, a wristwatch, or a wearable terminal. The health risk determination system 1 includes, for example, a sensor unit 11 and a health risk determination application 40.

The health risk determination application 40 is, for example, an application program executed on the information terminal. The health risk determination application 40 may be an application downloaded to the information terminal and executed on the information terminal. Hereinafter, an example in which the health risk determination application 40 is an application downloaded to the information terminal and the functions of the health risk determination application 40 is executed on the information terminal will be described.

The "information terminal" is, for example, a "mobile terminal". The "mobile terminal" refers to, for example, a portable information terminal such as a mobile phone including a smartphone, a tablet, or a notebook computer. The "information terminal" may be a terminal other than a mobile terminal, or may be, for example, a desktop computer. The "information terminal" includes, as one example, a processor (for example, a CPU) that executes an operating system, software (application), and the like; a main storage unit composed of a ROM and a RAM; an auxiliary storage unit composed of a flash memory and the like; a communication control unit composed of a wireless communication module and the like; an input device; and an output device such as a display. However, the configuration of the "information terminal" is not limited to the above-described configuration, and can be changed as appropriate.

Each function of the health risk determination application 40 is realized by loading predetermined software into the processor or the main storage unit and executing the software. The processor operates the communication control unit, the input device, or the output device described above according to the software, and reads and writes data from and to the main storage unit or the auxiliary storage unit. The data or database required to execute the functions of the health risk determination application 40 is stored in the main storage unit or the auxiliary storage unit.

Each functional element of the "information terminal" is realized by loading predetermined software into the processor or the storage unit (for example, the main storage unit or the auxiliary storage unit described above) and executing the software. The processor operates the communication control unit, the input device, or the output device described above according to the software, and reads and writes data from and to the storage unit. The data or database used for the processing of the "information terminal" is stored in the storage unit.

The health risk determination application 40 may be a distributed processing system composed of a plurality of computers, or may be a client-server system or a cloud system. As one example, the health risk determination application 40 includes, for example, a main module, a data acquisition module, a determination module, and an output module. The data acquisition module, the determination module, and the output module are executed, thereby causing each functional element of the health risk determination application 40 to function. As one example, the health risk determination application 40 may be provided in a state where the health risk determination application 40 is permanently recorded on a tangible storage medium such as a CD-ROM, a DVD-ROM, or a semiconductor memory. The health risk determination application 40 may be provided via a communication network as a data signal superimposed on a carrier wave.

The sensor unit 11 acquires information about the body of the user. As one example, the sensor unit 11 acquires breathing information, body movement information, and heartbeat information. The breathing information is information indicating the breathing of the user. The breathing information may include, for example, the number of breaths per unit time (hereinafter, simply referred to as a "breathing rate"). The unit time may be changeable. The body movement information is information indicating the body movement of the user. The heartbeat information is information indicating the heartbeat of the user. The contents of the information acquired by the sensor unit 11 can be changed as appropriate. The sensor unit 11 may acquire, for example, information indicating blood pressure.

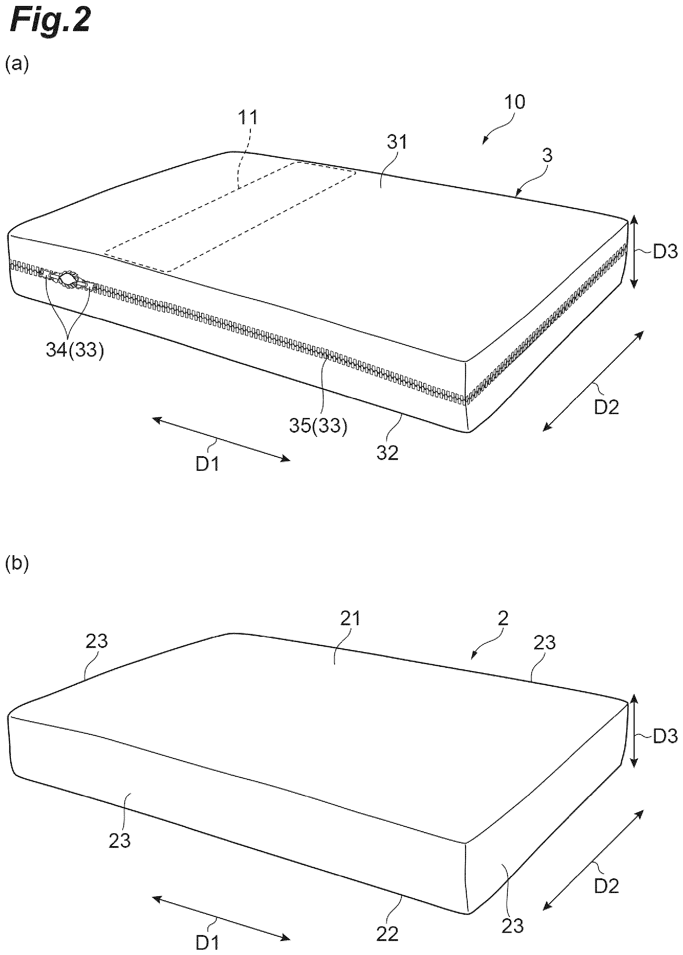

(a) in FIG. 2 is a perspective view showing a cushion body 10 to which the sensor unit 11 is attached. (b) in FIG. 2 is a perspective view showing a core material 2 constituting the cushion body 10 shown in (a) of FIG. 2. As shown in FIG. 1 and (a) and (b) of FIG. 2, the sensor unit 11 is, for example, a sensor sheet that is attachable to and detachable from the cushion body 10. The sensor unit 11 acquires vital data of the user lying on the cushion body 10. As one example, the sensor unit 11 may acquire an electrocardiogram. The breathing information, the body movement information, and the heartbeat information described above are included, for example, in the vital data.

For example, the sensor unit 11 includes a sheet-shaped fabric on which a thread-shaped sensor is embroidered, and the position of the sheet-shaped fabric with respect to the cushion body 10 can be changed. The thread-shaped sensor of the sensor unit 11 is fixed, for example, by being embroidered such that the thread-shaped sensor spreads out two-dimensionally on the sheet-shaped fabric. The thread-shaped sensor is, as one example, a piezoelectric sensor. In this case, the piezoelectric sensor of the sensor unit 11 generates electrical signals corresponding to the applied load of the body of the user based on the load, and the sensor unit 11 acquires the electrical signals as the breathing information, the body movement information, and the heartbeat information described above. The electrical signals acquired as the breathing information, the body movement information, and the heartbeat information may be referred to as "signals" below.

The sensor unit 11 includes, for example, the thread-shaped sensor (as one example, a piezoelectric sensor), and a communication unit that outputs the breathing information, the body movement information, and the heartbeat information as electrical signals generated by the thread-shaped sensor to the outside of the sensor unit 11. An example in which the sensor unit 11 includes a piezoelectric sensor has been described above. However, the type of the sensor of the sensor unit 11 is not limited to a piezoelectric sensor, and is not particularly limited. For example, the sensor unit 11 may include an acceleration sensor.

The breathing information includes a signal indicating body movement associated with the breathing of the user. The "body movement associated with breathing" refers to the movement of the body associated with breathing. The sensor unit 11 detects the body movement associated with the breathing of the user. The sensor unit 11 outputs the signal indicating body movement associated with breathing to the outside of the sensor unit 11. The heartbeat information includes a signal indicating body movement associated with the heartbeat of the user. The "body movement associated with heartbeat" refers to the movement of the body associated with heartbeat. The sensor unit 11 detects the body movement associated with the heartbeat of the user. The sensor unit 11 outputs the signal indicating body movement associated with heartbeat to the outside of the sensor unit 11. The body movement information includes a signal indicating body movement not associated with the breathing and heartbeat of the user. The "body movement not associated with breathing and heartbeat" refers to the movement of the body that is not related to breathing and heartbeat, for example, the movement of the body caused by turning over during sleep. The sensor unit 11 detects the body movement not associated with the breathing and heartbeat of the user. The sensor unit 11 outputs the signal indicating body movement not associated with breathing and heartbeat to the outside of the sensor unit 11.

For example, the sensor unit 11 is used while being attached to the cushion body 10 on which the body of the user is placed. The cushion body 10 has a rectangular shape in a plan view. The cushion body 10 extends in a longitudinal direction D1 and a lateral direction D2 orthogonal to the longitudinal direction D1. The cushion body 10 has a thickness in a thickness direction D3 orthogonal to both the longitudinal direction D1 and the lateral direction D2.

A length of the cushion body 10 in the longitudinal direction D1 is, for example, 120 cm or more and 210 cm or less (as one example, 195 cm). A length of the cushion body 10 in the lateral direction D2 is, for example, 70 cm or more and 180 cm or less (as one example, 97 cm). A length of the cushion body 10 in the thickness direction D3 is, for example, 3 cm or more and 40 cm or less (as one example, 9 cm).

As one example, the cushion body 10 is a mattress. The user of the cushion body 10 places his or her own body on the cushion body 10. At this time, a direction in which the body of the lying user extends (namely, a direction in which the head and legs of the user are connected to each other) coincides with, for example, the longitudinal direction D1 of the cushion body 10.

As shown in (b) of FIG. 2, the cushion body 10 includes the core material 2 housed inside a cover fabric 3 to be described later. As one example, the core material 2 has a rectangular shape in a plan view. The core material 2 includes, for example, a content and a bag that houses the content. The content is, for example, urethane foam or polyester. The material of the bag is, for example, cotton or polyester.

The core material 2 has a rectangular parallelepiped shape. The core material 2 has an upper surface 21 on which the body of the user is placed, and a lower surface 22 facing opposite the upper surface 21. The upper surface 21 is a surface facing one side (the upper side in (b) of FIG. 2) in the thickness direction D3. The lower surface 22 is a surface facing the other side (the lower side in (b) of FIG. 2) in the thickness direction D3. In addition, the core material 2 has a plurality of side surfaces 23 connecting the upper surface 21 and the lower surface 22.

As shown in (a) of FIG. 2, the cushion body 10 includes the cover fabric 3. As one example, the cover fabric 3 has a bag shape, and has a rectangular shape in a plan view. The cover fabric 3 includes, for example, a front fabric 31 covering the upper surface 21 of the core material 2; a back fabric 32 covering the lower surface 22 of the core material 2; and an opening and closing member 33 connecting the front fabric 31 and the back fabric 32 to each other. The opening and closing member 33 is provided at a position on the cover fabric 3 which corresponds to the side surfaces 23 of the core material 2. When viewed in the thickness direction D3, the opening and closing member 33 is located outside the side surfaces 23 of the core material 2. The cover fabric 3 is attachable to and detachable from the core material 2. The term "being attachable to and detachable from the core material" includes a case in which the cover fabric is turned over with respect to the core material, and a case in which the cover fabric is turned over to expose at least a part of the core material.

The opening and closing member 33 is, as one example, a so-called double slider. Namely, the opening and closing member 33 includes two slide members 34 and an opening and closing portion 35 that opens and closes the front fabric 31 and the back fabric 32 upon sliding of the slide members 34.

In this case, the front fabric 31 and the back fabric 32 can be opened by sliding one slide member 34 along the opening and closing portion 35 in one direction (a clockwise direction in (a) of FIG. 2) and sliding the other slide member 34 along the opening and closing portion 35 in the other direction (a counterclockwise direction in (a) of FIG. 2).

In addition, the front fabric 31 and the back fabric 32 can be closed by sliding the one slide member 34 along the opening and closing portion 35 in the other direction and sliding the other slide member 34 along the opening and closing portion 35 in the one direction. The opening and closing member 33 may be a so-called single slider. In this case, the opening and closing member 33 includes one slide member 34 and the opening and closing portion 35.

The sensor unit 11 is disposed, for example, inside the cover fabric 3 having a bag shape. More specifically, the sensor unit 11 is disposed between the core material 2 and the cover fabric 3. When the sensor unit 11 is attached to the cushion body 10, for example, the sensor unit 11 is disposed to extend in the lateral direction D2 of the cushion body 10. In this case, the sensor unit 11 is located on the side opposite the body of the user when viewed from the cover fabric 3 of the cushion body 10. Therefore, when the body of the user is placed on the cushion body 10, the sensor unit 11 does not come into contact with the user. The sensor unit 11 acquires the breathing information, the body movement information, and the heartbeat information of the user in a non-contact manner.

The sensor unit 11 is attached, for example, at a position corresponding to the heart of the user in the longitudinal direction D1. As one example, the sensor unit 11 is attached at any position between the position where the head of the user is placed and a position spaced apart in the longitudinal direction D1 of the cushion body 10 (a position on the lower right side in (a) of FIG. 2). The attachment position of the sensor unit 11 in the longitudinal direction D1 can be changed. A distance from the position where the head of the user is placed to the position where the sensor unit 11 is attached may be, for example, 40 cm or more and 50 cm or less (as one example, 50 cm).

The health risk determination application 40 acquires, for example, the sleep onset time and the awakening time of the user from the breathing information, the body movement information, and the heartbeat information. The sleep onset time refers to the time the user falls asleep. The awakening time refers to the time the user awakens. For example, the health risk determination application 40 may determine the sleep onset time based on the body movement information acquired by the sensor unit 11 and the movement of the bedding detected by the acceleration sensor of the sensor unit 11, and acquire the sleep onset time. The same applies to the acquisition of the awakening time. Unlike the above-described example, the user may operate the health risk determination application 40 on the information terminal, and the sleep onset time and the awakening time may be acquired through the operation of the user.

The health risk determination system 1 shown in FIG. 1 includes a first amplifier 12 and a second amplifier 13. The first amplifier 12 and the second amplifier 13 amplify the signal indicating body movement associated with breathing, the signal indicating body movement associated with heartbeat, and the signal indicating body movement not associated with breathing and heartbeat that are output from the sensor unit 11. The first amplifier 12 and the second amplifier 13 output the amplified signals (each of the signal indicating body movement associated with breathing, the signal indicating body movement associated with heartbeat, and the signal indicating body movement not associated with breathing and heartbeat) to the health risk determination application 40. For example, the first amplifier 12 and the second amplifier 13 output the amplified signals to a sleep stage acquisition unit 14 to be described later.

As one example, a gain of the first amplifier 12 is larger than a gain of the second amplifier 13. However, the gain of the first amplifier 12 and the gain of the second amplifier 13 should be different from each other. For example, the gain of the second amplifier 13 may be larger than the gain of the first amplifier 12.

The health risk determination application 40 includes, for example, the sleep stage acquisition unit 14, an abnormality detection unit 15, a unit count acquisition unit 16, an average count acquisition unit 17, a maximum count acquisition unit 18, and a health risk determination unit 19.

The sleep stage acquisition unit 14 acquires the sleep stage of the user. The sleep stage is a stage indicating the depth of sleep of the user. The sleep stages are classified into, for example, wakefulness, REM sleep, and non-REM sleep. The non-REM sleep is classified into four types of sleep stages. The sleep stage acquisition unit 14 acquires the sleep stage from the breathing information, the body movement information, and the heartbeat information acquired by the sensor unit 11. The sleep stage acquisition unit 14 acquires the sleep state using one of the signal amplified by the first amplifier 12 and the signal amplified by the second amplifier 13. For example, when the breathing of the user has suddenly increased, there is a possibility that the signal amplified by the first amplifier 12 exceeds the limit and becomes saturated. At this time, the sleep stage acquisition unit 14 acquires the signal amplified by the second amplifier 13 having a gain smaller than the gain of the first amplifier 12.

The abnormality detection unit 15 detects the abnormal breathing state of the user. The abnormality detection unit 15 detects the abnormal breathing state from the breathing information acquired by the sensor unit 11. The abnormality detection unit 15 detects, for example, the abnormal breathing state of the user from the signal indicating body movement associated with breathing, the signal indicating body movement associated with heartbeat, and the signal indicating body movement not associated with breathing and heartbeat that are output from the sensor unit 11. The abnormality detection unit 15 detects, for example, the abnormal breathing state from the breathing rate of the user.

The abnormality detection unit 15 may store abnormal breathing data and normal breathing data in advance. The abnormal breathing data is, for example, information indicating body movement associated with breathing, information indicating body movement associated with heartbeat, and information indicating body movement not associated with breathing and heartbeat while a person suffering from SAS sleeps. The normal breathing data is, for example, information indicating body movement associated with breathing, information indicating body movement associated with heartbeat, and information indicating body movement not associated with breathing and heartbeat while a person not suffering from SAS sleeps. The abnormality detection unit 15 may store, in advance, an algorithm for calculating the breathing rate of the user based on the breathing information, the abnormal breathing data, and the normal breathing data. In this case, the abnormality detection unit 15 can detect the abnormal breathing state from the breathing rate of the user.

Each of the abnormal breathing data and the normal breathing data may be generated, for example, by detecting body movement of persons during sleep using the sensor unit 11. The number of the persons may be one or may be plural (as one example, 12 persons). The abnormality detection unit 15 may refer to the abnormal breathing data and the normal breathing data, and determine the breathing rate of the user using the above-described algorithm based on the breathing information acquired by the sensor unit 11.

By the way, when the breathing state changes from the abnormal breathing state to a normal breathing state that is not the abnormal breathing state, the body movement of the user tends to occur. As one example, the abnormality detection unit 15 detects the abnormal breathing state from the body movement information acquired by the sensor unit 11. The abnormality detection unit 15 detects, for example, the abnormal breathing state of the user from the signal indicating body movement associated with breathing, the signal indicating body movement associated with heartbeat, and the signal indicating body movement not associated with breathing and heartbeat that are output from the sensor unit 11. The abnormality detection unit 15 detects, for example, the abnormal breathing state from the body movement of the user when the breathing state changes from the abnormal breathing state to the normal breathing state.

In addition, when the breathing state changes from the abnormal breathing state to the normal breathing state, the heart rate of the user tends to suddenly increase. As one example, the abnormality detection unit 15 detects the abnormal breathing state from the heartbeat information acquired by the sensor unit 11. The abnormality detection unit 15 detects, for example, the abnormal breathing state of the user from the signal indicating body movement associated with heartbeat that is output from the sensor unit 11. The abnormality detection unit 15 detects, for example, the abnormal breathing state from an increase in the heart rate of the user when the breathing state changes from the abnormal breathing state to the normal breathing state.

As one example, the abnormality detection unit 15 detects the abnormal breathing state of the user from the breathing rate of the user obtained from the breathing information, the body movement of the user when the breathing state changes from the abnormal breathing state to the normal breathing state, which is obtained from the body movement information, and an increase in the heart rate of the user when the breathing state changes from the abnormal breathing state to the normal breathing state, which is obtained from the heartbeat information. In this manner, the abnormality detection unit 15 may detect the abnormal breathing state of the user by taking into account not only the breathing information but also the body movement information and the heartbeat information.

The unit count acquisition unit 16 acquires the number of detections of the abnormal breathing state detected by the abnormality detection unit 15 for each unit time period between the sleep onset time and the awakening time. The unit time period is a time period having a predetermined time span. The time span of the unit time period is, as one example, one hour. For example, the time span of the unit time period can be changed, and may be set in advance by a user. The user is, for example, the user himself or herself, a person related to the user including the family of the user, or a doctor who performs treatment on the user. The smaller the time span of the unit time period is, the more detailed the sleep of the user can be examined, so that the health risk can be determined with even higher accuracy. Meanwhile, the larger the time span of the unit time period is, the more the processing load of the health risk determination system 1 can be reduced.

The average count acquisition unit 17 acquires an average detection count from the detection result of the abnormality detection unit 15. The average detection count refers to a value obtained by dividing the total value of the number of detections of the abnormal breathing state between the sleep onset time and the awakening time by the time from the sleep onset time to the awakening time. When the time span of the unit time period is one hour, the average detection count corresponds to a so-called apnea hypopnea index (AHI).

The maximum count acquisition unit 18 acquires a maximum detection count from the detection result of the abnormality detection unit 15. The maximum detection count refers to a maximum value among a plurality of the number of detections of the abnormal breathing states acquired for each unit time period. For example, the maximum count acquisition unit 18 acquires the number of detections of the abnormal breathing state for each unit time period. Then, the maximum count acquisition unit 18 acquires, as the maximum detection count, the number of detections of the abnormal breathing state in the unit time period having the largest number of detections.

The health risk determination unit 19 determines the health risk of the user. The health risk determination unit 19 determines the health risk of the user based on both the average detection count acquired by the average count acquisition unit 17 and the maximum detection count acquired by the maximum count acquisition unit 18. The health risk determination unit 19 determines, for example, the level of health risk based on both the average detection count and the maximum detection count. For example, the health risk determination unit 19 determines the health risk in three stages: "high", "medium", and "low".

As one example, the health risk determination unit 19 determines the health risk based on the detection result of the abnormality detection unit 15 and the sleep stage acquired by the sleep stage acquisition unit 14. The health risk determination unit 19 determines, for example, the health risk based on the time the abnormality detection unit 15 detects the abnormal breathing state and the sleep stage acquired by the sleep stage acquisition unit 14 at that time.

The health risk determination system 1 includes a display unit 20. The display unit 20 displays, for example, information generated by the health risk determination application 40 on the display of the information terminal. The display unit 20 may display, for example, the information on the display of a computer, a tablet terminal, a smartphone, or a wearable terminal that is owned by a user and that is an example of the information terminal.

The display unit 20 displays the health risk determined by the health risk determination unit 19. The display unit 20 displays the health risk on the display of the information terminal. The display unit 20 displays, for example, information indicating the magnitude of long-term risk and information indicating the magnitude of short-term risk. The long-term risk refers to the health risk of the user that is determined using the number of detections of the abnormal breathing state between the sleep onset time and the awakening time. The short-term risk refers to the health risk of the user that is determined using the number of detections of the abnormal breathing state acquired for each unit time period. The display unit 20 calculates, for example, the long-term risk based on the average detection count acquired by the average count acquisition unit 17. The display unit 20 calculates, for example, the short-term risk based on the maximum detection count acquired by the maximum count acquisition unit 18.

As one example, the display unit 20 displays information indicating the level of health risk. The display unit 20 displays, for example, the level of health risk determined by the health risk determination unit 19. The display unit 20 acquires, for example, information indicating a stage representing the level of health risk of the user from the health risk determination unit 19. For example, the display unit 20 displays the health risk in three stages: "high", "medium", and "low".

As one example, the display unit 20 displays information indicating the relationship between the occurrence of the abnormal breathing state and the sleep stage. The display unit 20 displays, based on the detection time of the abnormal breathing state detected by the abnormality detection unit 15 and the sleep stage acquired by the sleep stage acquisition unit 14 at the detection time, the information indicating the relationship between the occurrence of the abnormal breathing state and the sleep stage. The information indicating the magnitude of the long-term risk, the information indicating the magnitude of the short-term risk, the information indicating the level of health risk, and the information indicating the relationship between the occurrence of the abnormal breathing state and the sleep stage described above will be described in detail later.

Communication between the sensor unit 11 and both the first amplifier 12 and the second amplifier 13, communication between both the first amplifier 12 and the second amplifier 13 and the health risk determination application 40, and communication between the health risk determination application 40 and the display unit 20 may be realized, for example, by a wireless communication interface such as a wireless Local Area Network (LAN) or Bluetooth (registered trademark). In addition, these communications may be realized by wire.

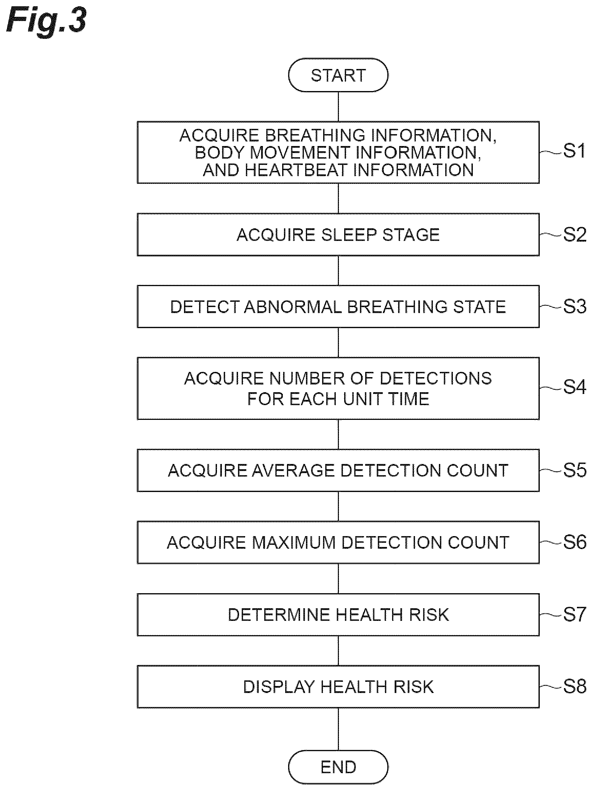

Subsequently, one example of the operation of the health risk determination system 1 will be described. FIG. 3 is a flowchart showing one example of the operation of the health risk determination system 1. Before the health risk determination system 1 is operated, first, the sensor unit 11 is attached to the cushion body 10. The sensor unit 11 is attached to the cushion body 10 such that the sensor unit 11 does not come into contact with a user when the body of the user is placed on the cushion body 10. For example, the sensor unit 11 is disposed between the core material 2 and the cover fabric 3. Next, the body of the user is laid on the cushion body 10 to which the sensor unit 11 is attached. For example, the body of the user is placed on the cushion body 10 so as to come into contact with the front fabric 31 of the cover fabric 3.

Subsequently, the sensor unit 11 acquires the breathing information, the body movement information, and the heartbeat information from body movement associated with the breathing of the user, body movement associated with heartbeat, and body movement not associated with breathing and heartbeat (step S1). In step S1, the sensor unit 11 detects body movement associated with the breathing of the user, body movement associated with heartbeat, and body movement not associated with breathing and heartbeat. The sensor unit 11 outputs a signal indicating the body movement associated with breathing, a signal indicating the body movement associated with heartbeat, and a signal indicating the body movement not associated with breathing and heartbeat to the outside of the sensor unit 11.

Next, the first amplifier 12 and the second amplifier 13 amplify the signal indicating body movement associated with breathing, the signal indicating body movement associated with heartbeat, and the signal indicating body movement not associated with breathing and heartbeat that are output from the sensor unit 11. Then, the amplified signals (each of the signal indicating body movement associated with breathing, the signal indicating body movement associated with heartbeat, and the signal indicating body movement not associated with breathing and heartbeat) are output to the health risk determination application 40. The first amplifier 12 and the second amplifier 13 output, for example, the amplified signals to the sleep stage acquisition unit 14.

Subsequently, the sleep stage acquisition unit 14 acquires the sleep stage of the user from the breathing information, the body movement information, and the heartbeat information acquired by the sensor unit 11 (step S2). The sleep stage acquisition unit 14 acquires, for example, the sleep stage from one of the signal amplified by the first amplifier 12 and the signal amplified by the second amplifier 13.

Then, the abnormality detection unit 15 detects the abnormal breathing state from the breathing information, the body movement information, and the heartbeat information acquired by the sensor unit 11 (step S3). As one example, the abnormality detection unit 15 detects the abnormal breathing state of the user from the breathing rate of the user obtained from the breathing information, the body movement of the user when the breathing state changes from the abnormal breathing state to the normal breathing state, which is obtained from the body movement information, and an increase in the heart rate of the user when the breathing state changes from the abnormal breathing state to the normal breathing state, which is obtained from the heartbeat information.

Subsequently, the unit count acquisition unit 16 acquires the number of detections of the abnormal breathing state detected by the abnormality detection unit 15 for each unit time period between the sleep onset time and the awakening time (step S4). Next, the average count acquisition unit 17 acquires the average detection count from the detection result of the abnormality detection unit 15 (step S5). Subsequently, the maximum count acquisition unit 18 acquires the maximum detection count from the detection result of the abnormality detection unit 15 (step S6). In other words, in step S6, the maximum count acquisition unit 18 acquires the number of detections of the abnormal breathing state for each unit time period from the detection result of the abnormality detection unit 15. Then, the maximum count acquisition unit 18 acquires, as the maximum detection count, the number of detections of the abnormal breathing state in the unit time period having the largest number of detections.

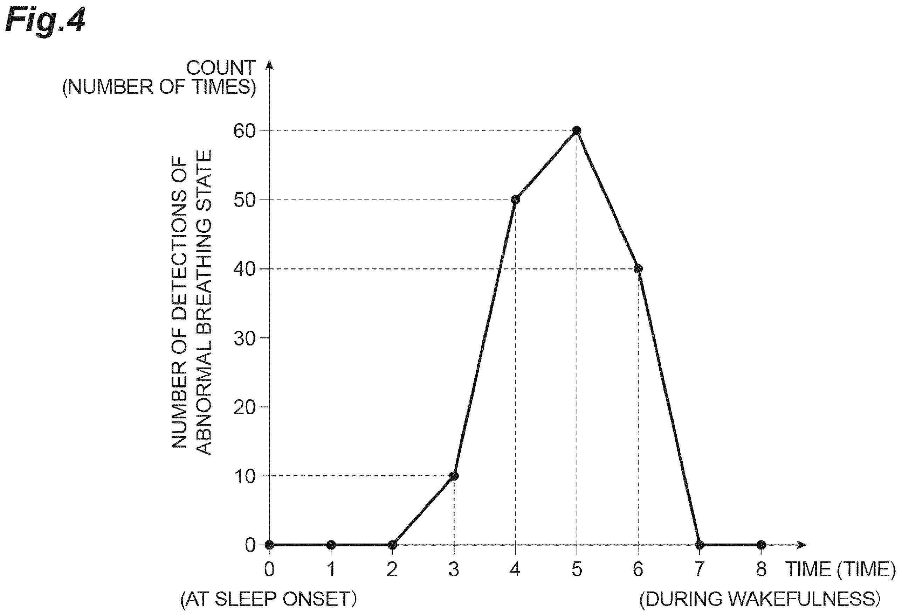

A specific example of the operations in step S4 to S6 will be described with reference to FIG. 4. FIG. 4 is a graph showing one example of time-series data of the number of detections of the abnormal breathing state. The vertical axis shown in FIG. 4 represents the number of detections of the abnormal breathing state detected by the abnormality detection unit 15, and the horizontal axis represents the time from the sleep onset time. In the example of FIG. 4, the time from the sleep onset time to the awakening time (namely, the sleep time of the user) is eight hours. In addition, the time span of the unit time period is one hour.

In the unit time period up to one hour after the sleep onset time, the number of detections is 0. In the unit time period from one hour to two hours after the sleep onset time, the number of detections is 0. In the unit time period from two hours to three hours after the sleep onset time, the number of detections is 10. In the unit time period from three hours to four hours after the sleep onset time, the number of detections is 50.

In the unit time period from four hours to five hours after the sleep onset time, the number of detections is 60. In the unit time period from five hours to six hours after the sleep onset time, the number of detections is 40. In the unit time period from six hours to seven hours after the sleep onset time, the number of detections is 0. In the unit time period from seven hours to eight hours after the sleep onset time, the number of detections is 0.

The total value of the number of detections of the abnormal breathing state between the sleep onset time and the awakening time is 160. The time from the sleep onset time to the awakening time is eight hours. Therefore, in the example of FIG. 4, the average count acquisition unit 17 acquires 20 as the average detection count. In addition, among the plurality of unit time periods described above, the unit time period from four hours to five hours after the sleep onset time has the largest number of detections. Therefore, in the example of FIG. 4, the maximum count acquisition unit 18 acquires 60 as the maximum detection count.

Next, as shown in FIG. 3, the health risk determination unit 19 determines the health risk of the user based on both the average detection count acquired by the average count acquisition unit 17 and the maximum detection count acquired by the maximum count acquisition unit 18 (step S7). The health risk determination unit 19 determines the level of health risk based on both the average detection count and the maximum detection count. For example, the health risk determination unit 19 determines the health risk in three stages: "high", "medium", and "low".

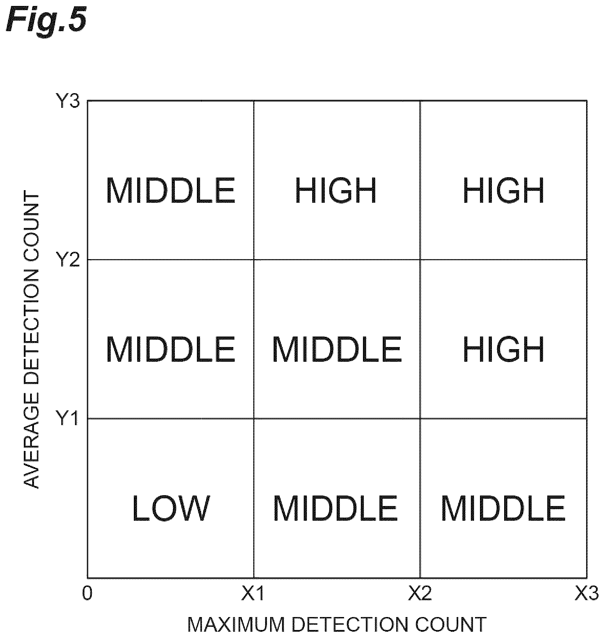

A specific example of the operation of the health risk determination unit 19 when determining the level of health risk will be described with reference to FIG. 5. FIG. 5 is a schematic view showing one example of criteria for determining a health risk. The terms "high", "medium", and "low" shown in FIG. 5 indicate the level of health risk. In addition, "X1", "X2", "X3", "Y1", "Y2", and "Y3" are predetermined natural numbers. As one example, X1 is 20, X2 is 45, and X3 is 65. As one example, Y1 is 15, Y2 is 30, and Y3 is 45. The values of X1 to X3 and Y1 to Y3 may be changeable values.

When the average detection count is 0 or more and less than Y1 and the maximum detection count is 0 or more and less than X1, the health risk determination unit 19 determines the health risk as low. At this time, the health risk determination unit 19 determines the stage representing the level of health risk as "low". When the average detection count is Y2 or more and the maximum detection count is X1 or more and less than X3, when the average detection count is Y1 or more and less than Y3 and the maximum detection count is X2 or more, and when the average detection count is Y3 or more and the maximum detection count is X3 or more, the health risk determination unit 19 determines the health risk as high. At this time, the health risk determination unit 19 determines the stage of health risk as "high". In other cases, the health risk determination unit 19 determines the health risk as approximately medium. At this time, the health risk determination unit 19 determines the stage of health risk as "medium".

In step S7, the health risk determination unit 19 determines the health risk based on the detection result of the abnormality detection unit 15 and the sleep stage acquired by the sleep stage acquisition unit 14. The health risk determination unit 19 acquires, for example, the time the abnormality detection unit 15 detects the abnormal breathing state and the sleep stage acquired by the sleep stage acquisition unit 14 at that time.

Next, the display unit 20 displays the health risk determined by the health risk determination unit 19 (step S8). As described above, the display unit 20 displays, for example, at least one of the information indicating the magnitude of the long-term risk, the information indicating the magnitude of the short-term risk, the information indicating the level of health risk, and the information indicating the relationship between the occurrence of the abnormal breathing state and the sleep stage.

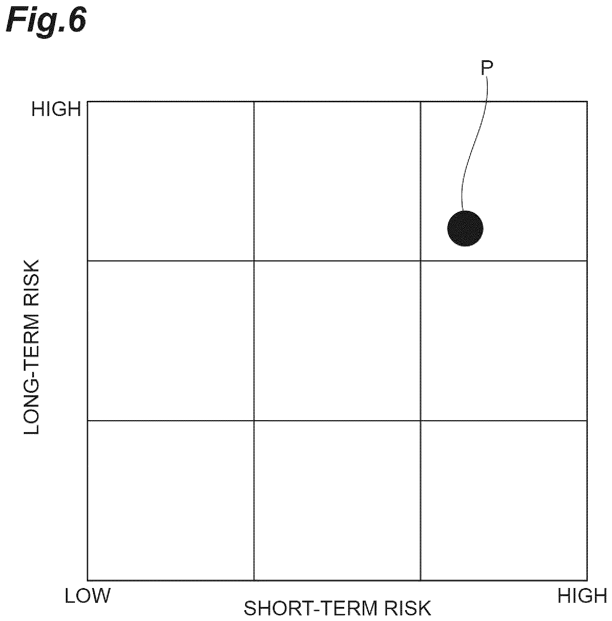

A specific example of the operation in step S8 will be described with reference to FIG. 6. FIG. 6 is a view showing one example of the display of health risk by the display unit 20. In the example of FIG. 6, the display unit 20 displays the vertical axis representing the magnitude of the long-term risk, and the horizontal axis representing the magnitude of the short-term risk. The display unit 20 displays a plurality of (as one example, nine) squares disposed in a matrix. The squires correspond to squares shown in FIG. 5. The vertical axis representing the magnitude of the long-term risk shown in FIG. 6 corresponds to the vertical axis representing the average detection count shown in FIG. 5. The horizontal axis representing the magnitude of the short-term risk shown in FIG. 6 corresponds to the horizontal axis representing the maximum detection count shown in FIG. 5.

As one example, the display unit 20 displays the determination result of the health risk by displaying a point P in one of the plurality of squares shown in FIG. 6. In this manner, the display unit 20 displays the information indicating the magnitude of the long-term risk, and the information indicating the magnitude of the short-term risk. The display unit 20 calculates, for example, the long-term risk based on the average detection count acquired by the average count acquisition unit 17. The display unit 20 determines the position of the point P in a direction along the vertical axis (an up-down direction of the drawing sheet in FIG. 6) from the average detection count acquired by the average count acquisition unit 17. The display unit 20 calculates, for example, the short-term risk based on the maximum detection count acquired by the maximum count acquisition unit 18. The display unit 20 determines the position of the point P in a direction along the horizontal axis (a left-right direction of the drawing sheet in FIG. 6) from the maximum detection count acquired by the maximum count acquisition unit 18. Then, the display unit 20 displays the point P in one of the plurality of squares based on the determined position in the direction along the vertical axis and the determined position in the direction along the horizontal axis.

FIG. 6 shows an example of a display by the display unit 20 when the average detection count is Y2 or more and less than Y3 and the maximum detection count is Y2 or more and less than Y3. Therefore, the display unit 20 displays the point P in the square located on the upper right side of FIG. 6 among the nine squares.

In step S8, the display unit 20 displays, for example, the information indicating the level of health risk determined by the health risk determination unit 19. The display unit 20 acquires, for example, information indicating a stage representing the level of health risk of the user from the health risk determination unit 19. The display unit 20 displays the level of health risk of the user in three stages: "high", "medium", and "low". In the examples of FIGS. 5 and 6, the display unit 20 displays a message that the stage representing the level of health risk of the user is "high".

In step S8, the display unit 20 displays, for example, the information indicating the relationship between the occurrence of the abnormal breathing state and the sleep stage. The display unit 20 displays, for example, based on the detection time of the abnormal breathing state acquired by the health risk determination unit 19 and the sleep stage at that time, the information indicating the relationship between the occurrence of the abnormal breathing state and the sleep stage. In this case, the display unit 20 may display the sleep stage at which the number of detections of the abnormal breathing state detected by the abnormality detection unit 15 has increased or decreased, as the information indicating the relationship between the occurrence of the abnormal breathing state and the sleep stage. For example, the display unit 20 displays a message that the number of detections of the abnormal breathing state has increased or decreased when M hours has passed since the sleep onset time (M is a natural number, as one example, four) and the sleep depth is N (N is a natural number, as one example, three).

For example, the display unit 20 may be display a possibility that the abnormal breathing state affects the quality of sleep of the user. For example, the display unit 20 may display a message that when the number of detections of the abnormal breathing state is large during non-REM sleep in which the sleep of the user is deep, there is a possibility that the quality of sleep decreases due to the abnormal breathing state, as the information indicating the relationship between the occurrence of the abnormal breathing state and the sleep stage.

One example of the operation of the health risk determination system 1 has been described above. However, the content and order of each step in the operation of the health risk determination system are not limited to the above-described example, and can be changed as appropriate.

Subsequently, actions and effects of the health risk determination system 1 will be described. In the health risk determination system 1, the sensor unit 11 acquires breathing information of the user without coming into contact with the user. The burden can be reduced compared to when the sensor unit 11 comes into contact with the user.

The health risk determination system 1 determines the health risk of the user based on both the average detection count acquired by the average count acquisition unit 17 and the maximum detection count acquired by the maximum count acquisition unit 18. For example, there is a possibility that the user intensively experiences the abnormal breathing state in a short period of time between the sleep onset time and the awakening time. In this case, when the health risk of the user is determined solely based on the average detection count, there is a possibility that the health risk cannot be accurately identified. In the health risk determination system 1, the health risk of the user is determined by taking into account the average detection count as well as the maximum detection count, and therefore, even when the user intensively experiences the abnormal breathing state, the health risk can be accurately identified.

For example, as shown in FIG. 4, there is a possibility that the user intensively experiences the abnormal breathing state in a short period of time. In the example of FIG. 4, the average detection count is 20. In the state shown in FIG. 4, when only the criteria for determining the average detection count shown in FIG. 5 are considered, the health risk of the user may be determined as approximately medium (the average detection count is Y1 or more and less than Y2). On the other hand, when the health risk is determined based on both the average detection count and the maximum detection count as in the health risk determination system 1, the health risk determination unit 19 determines the health risk of the user as high (the average detection count is Y1 or more and less than Y2 and the maximum detection count is X2 or more). In this manner, in the health risk determination system 1, the health risk of the user can be more accurately determined.

As one example, the sensor unit 11 acquires the body movement information that is information indicating the body movement of the user, and the heartbeat information that is information indicating the heartbeat of the user. The abnormality detection unit 15 detects the abnormal breathing state from the body movement information and the heartbeat information. In this case, the abnormal breathing state can be detected from the body movement information and the heartbeat information in addition to the breathing information. For example, the abnormal breathing state can be detected from the body movement of the user when the breathing state changes from the abnormal breathing state to the normal breathing state and an increase in the heart rate of the user. Therefore, the abnormal breathing state of the user can be more accurately detected.

As one example, the health risk determination system 1 further includes the sleep stage acquisition unit 14 that determines the sleep stage of the user based on the breathing information, the body movement information, and the heartbeat information. The health risk determination unit determines the health risk of the user based on the detection result of the abnormality detection unit 15 and the sleep stage. In this case, the health risk can be determined based on the sleep stage in addition to the detection result of the abnormal breathing state. For example, from the view point of the relationship between the occurrence of the abnormal breathing state and the sleep stage, the user can be advised of the health risk.

As one example, the health risk determination system 1 further includes the first amplifier 12 and the second amplifier 13 that amplify signals and output the amplified signals to the sleep stage acquisition unit 14. The gain of the first amplifier 12 is larger than the gain of the second amplifier 13.

For example, when the breathing state changes from the abnormal breathing state to the normal breathing state, the breathing of the user tends to increase. If the health risk determination system 1 includes only the first amplifier 12 having a predetermined gain, there is a possibility that the signal amplified by the first amplifier 12 when the breathing of the user has increased exceeds the limit and becomes saturated. On the other hand, since the health risk determination system 1 includes the second amplifier 13 having a gain smaller than the gain of the first amplifier 12, when the signal in the first amplifier 12 becomes saturated, the second amplifier 13 can amplify the signal and output the amplified signal to the sleep stage acquisition unit 14. Therefore, the sleep stage can be more accurately acquired.

Next, a specific example of the autonomic nervous system determination system will be described. As one example, the autonomic nervous system determination system determines the state of the autonomic nervous system of a user from a bedtime to a wake-up time. For example, the autonomic nervous system determination system may be used for personal or household purposes, or may be used in research institutes or the like for testing and research purposes. In addition, the autonomic nervous system determination system may be used in hospitals or the like for treatment. The "user" refers to a person who is the subject of autonomic nervous system determination by the autonomic nervous system determination system. The user is, for example, a person who has a health risk caused by an autonomic nervous system disturbance, a person who receives sleep treatment at a hospital or the like, or a person who wishes to determine the state of his or her own autonomic nervous system.

The "autonomic nervous system" refers to a nervous system that controls the functioning of organs such as the heart or stomach or involuntary functions such as blood circulation. As used herein, the term "involuntary" refers to, for example, a situation in which something does not proceed according to one's own wishes or cannot be controlled by one's own intention. The autonomic nervous system is composed of the sympathetic nervous system and the parasympathetic nervous system. The sympathetic nervous system and the parasympathetic nervous system are regulated while being balanced to each other. The balance between the sympathetic nervous system and the parasympathetic nervous system may hereinafter be simply referred to as the "balance of the autonomic nervous system". The term "balance of the autonomic nervous system being good" refers to, as one example, a state where the sympathetic nervous system and the parasympathetic nervous system function in an antagonistic manner during wakefulness, and a state where the parasympathetic nervous system is dominant over the sympathetic nervous system at sleep onset.

The sympathetic nervous system functions when a person is excited, tense, or stressed. When the sympathetic nervous system functions, reactions such as faster heartbeat, shallower and faster breathing, the constriction of blood vessels, and an increase in blood pressure appear in the body. The parasympathetic nervous system functions when a person is relaxed. When the parasympathetic nervous system functions, reactions such as slower heartbeat, deeper and slower breathing, the dilation of blood vessels, a decrease in blood pressure, and an enhancement of immune function appear in the body. Such reactions of the autonomic nervous system cannot be intentionally controlled by a person's intention, and are therefore used as objective indicators of emotions, feelings, fatigue, stress, and the like.

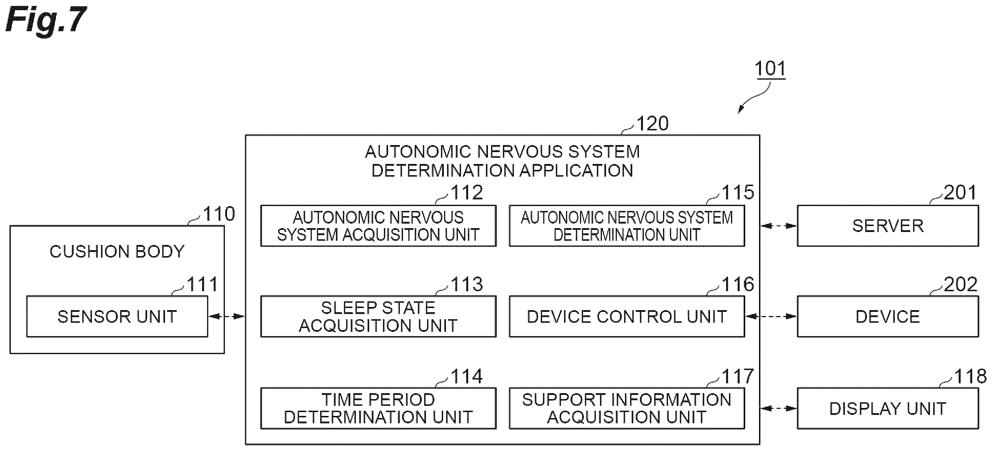

FIG. 7 is a block diagram showing the autonomic nervous system determination system as one example. The autonomic nervous system determination system 101 is accessible, for example, from an information terminal such as a computer, a tablet terminal, a smartphone, a wristwatch, or a wearable terminal. The autonomic nervous system determination system 101 is communicably connected to, for example, a server 201 external to the autonomic nervous system determination system 101. The autonomic nervous system determination system 101 includes, for example, a sensor unit 111 and an autonomic nervous system determination application 120.

The autonomic nervous system determination application 120 is, for example, an application program executed on the information terminal. The autonomic nervous system determination application 120 may be an application downloaded to the information terminal and executed on the information terminal, or may be executed on the server 201. The autonomic nervous system determination application 120 may be an application downloaded from the server 201. Hereinafter, an example in which the autonomic nervous system determination application 120 is an application downloaded to the information terminal and the functions of the autonomic nervous system determination application 120 are executed on the information terminal will be described.

Each function of the autonomic nervous system determination application 120 is realized by loading predetermined software into the processor or the main storage unit and executing the software. The data or database required to execute the functions of the autonomic nervous system determination application 120 is stored in the main storage unit or the auxiliary storage unit.

The autonomic nervous system determination application 120 may be a distributed processing system composed of a plurality of computers, or may be a client-server system or a cloud system. As one example, the autonomic nervous system determination application 120 includes, for example, a main module, a data acquisition module, a determination module, and an output module. The data acquisition module, the determination module, and the output module are executed, thereby causing each functional element of the autonomic nervous system determination application 120 to function. As one example, the autonomic nervous system determination application 120 may be provided in a state where the autonomic nervous system determination application 120 is permanently recorded on a tangible storage medium such as a CD-ROM, a DVD-ROM, or a semiconductor memory. The autonomic nervous system determination application 120 may be provided via a communication network as a data signal superimposed on a carrier wave.

The sensor unit 111 acquires information about the body of the user. The sensor unit 111 acquires at least one of breathing information, body movement information, heartbeat information, and brainwave information. As one example, the sensor unit 111 acquires the breathing information, the body movement information, and the heartbeat information. The brainwave information is information indicating the brainwave of the user. For example, the contents of the information acquired by the sensor unit 111 can be changed as appropriate. The sensor unit 111 may acquire, for example, information indicating blood pressure.

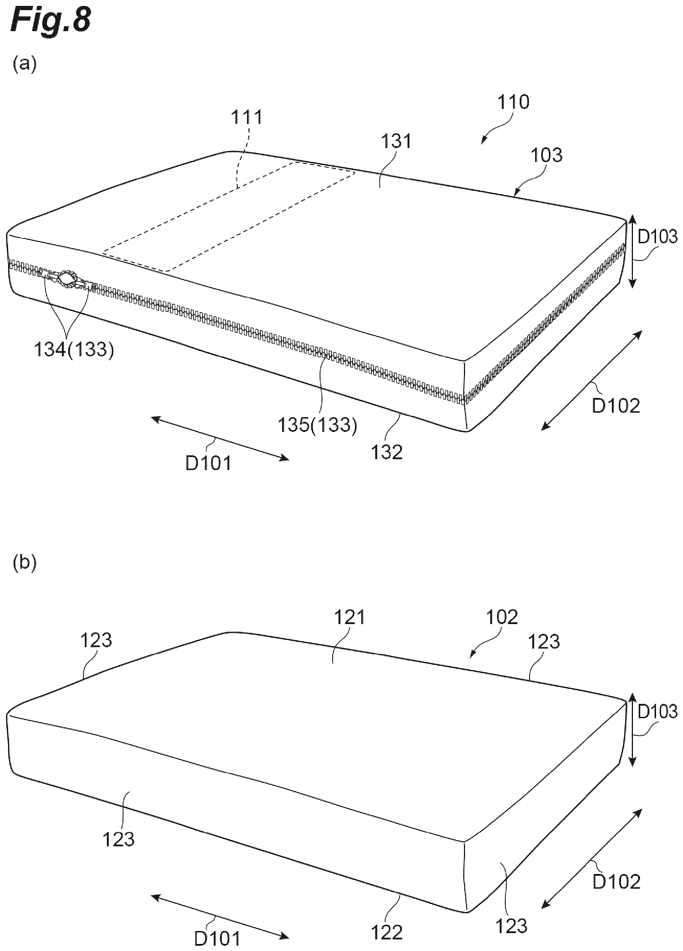

(a) in FIG. 8 is a perspective view showing a cushion body 110 to which the sensor unit 111 is attached. (b) in FIG. 8 is a perspective view showing a core material 102 constituting the cushion body 110 shown in (a) of FIG. 8. As shown in FIG. 7 and (a) and (b) of FIG. 8, the sensor unit 111 is, for example, a sensor sheet that is attachable to and detachable from the cushion body 110. The sensor unit 111 acquires vital data of the user lying on the cushion body 110. As one example, the sensor unit 111 may acquire an electrocardiogram.

For example, the sensor unit 111 includes a sheet-shaped fabric on which a thread-shaped sensor is embroidered, and the position of the sheet-shaped fabric with respect to the cushion body 110 can be changed. The thread-shaped sensor of the sensor unit 111 is fixed, for example, by being embroidered such that the thread-shaped sensor spreads out two-dimensionally on the sheet-shaped fabric. For example, a piezoelectric sensor of the sensor unit 111 generates electrical signals corresponding to the applied load of the body of the user based on the load, and the sensor unit 111 acquires the electrical signals as the breathing information, the body movement information, and the heartbeat information described above.

The sensor unit 111 includes, for example, the thread-shaped sensor (as one example, a piezoelectric sensor), and a communication unit that outputs the breathing information, the body movement information, and the heartbeat information as electrical signals generated by the thread-shaped sensor to the outside of the sensor unit 111. An example in which the sensor unit 111 includes a piezoelectric sensor has been described above. However, the type of the sensor of the sensor unit 111 is not limited to a piezoelectric sensor, and is not particularly limited. For example, the sensor unit 111 may include an acceleration sensor.

The sensor unit 111 detects body movement associated with the breathing of the user. The sensor unit 111 outputs a signal indicating the body movement associated with breathing to the outside of the sensor unit 111. The sensor unit 111 detects body movement associated with the heartbeat of the user. The sensor unit 111 outputs a signal indicating the body movement associated with heartbeat to the outside of the sensor unit 111. The sensor unit 111 detects body movement not associated with the breathing and heartbeat of the user. The sensor unit 111 outputs a signal indicating the body movement not associated with breathing and heartbeat to the outside of the sensor unit 111.

For example, the sensor unit 111 is used while being attached to the cushion body 110 on which the body of the user is placed. The cushion body 110 has a rectangular shape in a plan view. The cushion body 110 extends in a longitudinal direction D101 and a lateral direction D102 orthogonal to the longitudinal direction D101. The cushion body 110 has a thickness in a thickness direction D103 orthogonal to both the longitudinal direction D101 and the lateral direction D102.

A length of the cushion body 110 in the longitudinal direction D101 is, for example, 120 cm or more and 210 cm or less (as one example, 195 cm). A length of the cushion body 110 in the lateral direction D102 is, for example, 70 cm or more and 180 cm or less (as one example, 97 cm). A length of the cushion body 110 in the thickness direction D103 is, for example, 3 cm or more and 40 cm or less (as one example, 9 cm).

As one example, the cushion body 110 is a mattress. The user of the cushion body 110 places his or her own body on the cushion body 110. At this time, a direction in which the body of the lying user extends (namely, a direction in which the head and legs of the user are connected to each other) coincides with, for example, the longitudinal direction D101 of the cushion body 110.

As shown in (b) of FIG. 8, the cushion body 110 includes the core material 102 housed inside a cover fabric 103 to be described later. As one example, the core material 102 has a rectangular shape in a plan view. The core material 102 includes, for example, a content and a bag that houses the content. The content is, for example, urethane foam or polyester. The material of the bag is, for example, cotton or polyester.

The core material 102 has a rectangular parallelepiped shape. The core material 102 has an upper surface 121 on which the body of the user is placed, and a lower surface 122 facing opposite the upper surface 121. The upper surface 121 is a surface facing one side (the upper side in (b) of FIG. 8) in the thickness direction D103. The lower surface 122 is a surface facing the other side (the lower side in (b) of FIG. 8) in the thickness direction D103. The core material 102 has a plurality of side surfaces 123 connecting the upper surface 121 and the lower surface 122.

As shown in (a) of FIG. 8, the cushion body 110 includes the cover fabric 103. As one example, the cover fabric 103 has a bag shape, and has a rectangular shape in a plan view. The cover fabric 103 includes, for example, a front fabric 131 covering the upper surface 121 of the core material 102; a back fabric 132 covering the lower surface 122 of the core material 102; and an opening and closing member 133 connecting the front fabric 131 and the back fabric 132 to each other. The opening and closing member 133 is provided at a position on the cover fabric 103 which corresponds to the side surfaces 123 of the core material 102. When viewed in the thickness direction D103, the opening and closing member 133 is located outside the side surfaces 123 of the core material 102. The cover fabric 103 is attachable to and detachable from the core material 102.

The opening and closing member 133 is, as one example, a so-called double slider. Namely, the opening and closing member 133 includes two slide members 134 and an opening and closing portion 135 that opens and closes the front fabric 131 and the back fabric 132 upon sliding of the slide members 134.

In this case, the front fabric 131 and the back fabric 132 can be opened by sliding one slide member 134 along the opening and closing portion 135 in one direction (a clockwise direction in (a) of FIG. 8) and sliding the other slide member 134 along the opening and closing portion 135 in the other direction (a counterclockwise direction in (a) of FIG. 8).

The front fabric 131 and the back fabric 132 can be closed by sliding the one slide member 134 along the opening and closing portion 135 in the other direction and sliding the other slide member 134 along the opening and closing portion 135 in the one direction. The opening and closing member 133 may be a so-called single slider. In this case, the opening and closing member 133 includes one slide member 134 and the opening and closing portion 135.

The sensor unit 111 is disposed, for example, inside the cover fabric 103 having a bag shape. More specifically, the sensor unit 111 is disposed between the core material 102 and the cover fabric 103. When the sensor unit 111 is attached to the cushion body 110, for example, the sensor unit 111 is disposed to extend in the lateral direction D102 of the cushion body 110. In this case, the sensor unit 111 is located on the side opposite the body of the user when viewed from the cover fabric 103 of the cushion body 110. When the body of the user is placed on the cushion body 110, the sensor unit 111 does not come into contact with the user. The sensor unit 111 acquires the breathing information, the body movement information, and the heartbeat information of the user in a non-contact manner.

The sensor unit 111 is attached, for example, at a position corresponding to the heart of the user in the longitudinal direction D101. As one example, the sensor unit 111 is attached at any position between the position where the head of the user is placed and a position spaced apart in the longitudinal direction D101 of the cushion body 110 (a position on the lower right side in (a) of FIG. 8). The attachment position of the sensor unit 111 in the longitudinal direction D101 can be changed. A distance from the position where the head of the user is placed to the position where the sensor unit 111 is attached may be, for example, 40 cm or more and 50 cm or less (as one example, 50 cm).

The autonomic nervous system determination application 120 acquires, for example, the bedtime and wake-up time of the user from the breathing information, the body movement information, and the heartbeat information. The bedtime refers to the time the user goes to bed. The wake-up time refers to the time the user wakes up the next morning or the like. For example, the autonomic nervous system determination application 120 may determine the bedtime based on the body movement information acquired by the sensor unit 111 and the movement of the bedding detected by the acceleration sensor of the sensor unit 111, and acquire the bedtime. The same applies to the acquisition of the wake-up time. Unlike the above-described example, the user may operate the autonomic nervous system determination application 120 on the information terminal, and the bedtime and the wake-up time may be acquired through the operation of the user.

The autonomic nervous system determination application 120 includes, for example, an autonomic nervous system acquisition unit 112, a sleep state acquisition unit 113, and a time period determination unit 114. The autonomic nervous system acquisition unit 112 acquires autonomic nervous system information from the heartbeat information. The autonomic nervous system acquisition unit 112 may acquire the autonomic nervous system information, and calculate a stress level. For example, the autonomic nervous system acquisition unit 112 calculates the stress level based on at least one of the breathing information, the heartbeat information, and the body movement information acquired by the sensor unit 111.

The autonomic nervous system information acquired by the autonomic nervous system acquisition unit 112 is information indicating the state of the autonomic nervous system of the user. The autonomic nervous system acquisition unit 112 acquires, for example, the heart rate variability (HRV) of the user by analyzing the heartbeat information acquired by the sensor unit 111. The autonomic nervous system acquisition unit 112 acquires the autonomic nervous system information from the heart rate variability of the user. For example, the autonomic nervous system information includes information indicating periodic components contained in the heart rate variability. Incidentally, the method for acquiring the autonomic nervous system information is not limited to the above-described example, and can be changed as appropriate.

The sleep state acquisition unit 113 acquires the sleep state of the user. The sleep state includes a sleep stage indicating the depth of sleep of the user. The sleep state acquisition unit 113 acquires the sleep state from at least one of the breathing information, the body movement information, the heartbeat information, and the brainwave information acquired by the sensor unit 111. As one example, the sleep state acquisition unit 113 acquires a sleep stage as the sleep state from the breathing information, the body movement information, and the heartbeat information acquired by the sensor unit 111. The sleep state acquisition unit 113 acquires, for example, the sleep state using the signal indicating body movement associated with breathing, the signal indicating body movement associated with heartbeat, and the signal indicating body movement not associated with breathing and heartbeat that are output from the sensor unit 111.

The time period determination unit 114 determines a detection target time period based on the body movement information acquired by the sensor unit 111. The detection target time period refers to a time period during which the body movement of the user does not occur between the bedtime and the wake-up time. The "time period during which body movement does not occur" refers to, for example, the time period during which either the supine position or the lateral position is maintained. The "time period during which body movement does not occur" may be a time period other than the time the user turns over during sleep between the bedtime and the wake-up time. For example, the time period determination unit 114 determines the detection target time period based on the signal indicating body movement not associated with the breathing and heartbeat of the user that is output from the sensor unit 111.

The autonomic nervous system determination application 120 includes an autonomic nervous system determination unit 115, a device control unit 116, and a support information acquisition unit 117. The autonomic nervous system determination unit 115 determines the state of the autonomic nervous system of the user based on both the autonomic nervous system information acquired by the autonomic nervous system acquisition unit 112 and the sleep state acquired by the sleep state acquisition unit 113. As one example, the autonomic nervous system determination unit 115 determines the state of the autonomic nervous system of the user in the detection target time period determined by the time period determination unit 114. For example, the autonomic nervous system determination unit 115 performs frequency analysis on the periodic components of the heart rate variability included in the autonomic nervous system information, and determines the state of the autonomic nervous system based on the power spectrum of each frequency.

The power spectrum of the autonomic nervous system obtained as a result of the frequency analysis is divided into a low frequency (LF) component that is the integral value of the power spectrum in a low frequency band (as one example, 0.04 Hz to 0.15 Hz), and a high frequency (HF) component that is the integral value of the power spectrum in a high frequency band (as one example, 0.15 Hz to 0.4 Hz). The LF component reflects sympathetic activity and parasympathetic activity, and the HF component reflects parasympathetic activity. The autonomic nervous system determination unit 115 may use, for example, a sympathetic nervous system index as an index indicating the dominance of the sympathetic nervous system. The sympathetic nervous system index is, as one example, a value obtained by dividing the value of the LF component by the value of the HF component. In addition, the autonomic nervous system determination unit 115 may use, for example, a parasympathetic nervous system index as an index indicating the dominance of the parasympathetic nervous system. The parasympathetic nervous system index is, as one example, a value obtained by dividing the value of the HF component by the sum of the LF component and the HF component.

The autonomic nervous system determination unit 115 calculates, for example, the level of activity of the user based on the sympathetic nervous system index. The level of activity is the degree of dominance of the sympathetic nervous system over the parasympathetic nervous system. The autonomic nervous system determination unit 115 calculates, for example, the level of relaxation of the user based on the parasympathetic nervous system index. The level of relaxation is the degree of dominance of the parasympathetic nervous system over the sympathetic nervous system. The autonomic nervous system determination unit 115 classifies, for example, the state of the autonomic nervous system of the user based on the level of activity and the level of relaxation of the user. The classification of the state of the autonomic nervous system will be described later.

The autonomic nervous system determination unit 115 determines, for example, the state of the autonomic nervous system according to the sleep state of the user. For example, the autonomic nervous system tends to be disturbed more easily during REM sleep compared to during non-REM sleep. The "autonomic nervous system disturbance" refers to, as one example, a state where the activity of the amygdala involved in heart rate variability becomes active and the homeostasis of the autonomic nervous system is no longer maintained. The autonomic nervous system determination unit 115 may store, in advance, for example, information indicating the state of the autonomic nervous system of a person other than the user during REM sleep. The autonomic nervous system determination unit 115 may determine the state of the autonomic nervous system of the user by comparing the autonomic nervous system information of the user acquired by the autonomic nervous system acquisition unit 112 with the information indicating the state of the autonomic nervous system that is stored in advance.

The device control unit 116 controls the operation of a device 202 constituting an environment around the user. The device control unit 116 controls the operation of the device 202 using the determination result of the autonomic nervous system determination unit 115. The device 202 is, for example, an air conditioner, a lighting fixture, a music player, or an aroma diffuser installed in the bedroom of the user, or an electric blanket or a thermal therapy device that is placed over the body of the user. The device control unit 116 can communicate with the device 202. For example, when the device 202 is an air conditioner, the device control unit 116 may perform temperature control of a space where the user is present by controlling the operation of the device 202.

The support information acquisition unit 117 acquires support information from the server 201 using the determination result of the autonomic nervous system determination unit 115. Incidentally, the support information acquisition unit 117 may acquire, from the data, support information included in data stored in the information terminal by downloading the autonomic nervous system determination application 120, instead of acquiring support information from the server 201. The support information refers to information that supports the life of the user. The support information includes, for example, information indicating the content of a meal that is desirable for the user to have after waking up. For example, the support information acquisition unit 117 determines at least one of the ease of sleep onset, sleepiness upon waking up, fatigue recovery level, and biorhythm based on the determination result of the autonomic nervous system determination unit 115, and acquires the support information. The support information acquisition unit 117 acquires the support information using, for example, table data stored in a database stored in the server 201, and the determination result of the autonomic nervous system determination unit 115. A specific example of the support information and a specific method for acquiring the support information will be described in detail later.

The autonomic nervous system determination system 101 includes a display unit 118. The display unit 118 displays, for example, information generated by the autonomic nervous system determination application 120 on the display of the information terminal. The display unit 118 may display, for example, the information on the display of a computer, a tablet terminal, a smartphone, or a wearable terminal that is owned by a user and that is an example of the information terminal.

The display unit 118 displays the support information acquired by the support information acquisition unit 117. The display unit 118 displays the support information on the display of the information terminal. For example, the display unit 118 may display, as the support information, a comment presenting advice according to the state of the autonomic nervous system of the user during sleep based on the level of relaxation at sleep onset and the level of activity upon waking up.

As one example, the display unit 118 displays the determination result of the autonomic nervous system determination unit 115 in addition to the support information. The display unit 118 displays, for example, information indicating the evaluation of the state of the autonomic nervous system of the user based on the level of relaxation at sleep onset and the level of activity upon waking up. The display unit 118 may display, for example, information indicating the balance of the autonomic nervous system at sleep onset and upon waking up based on the autonomic nervous system information and the sleep state. Specific examples of the comment that presents advice, the information indicating evaluation, and the information indicating the balance of the autonomic nervous system described above will be described in detail later.

Communication between the sensor unit 111 and the autonomic nervous system determination application 120, communication between the autonomic nervous system determination application 120 and the server 201, and communication between the device control unit 116 and the device 202 may be realized, for example, by a wireless communication interface such as a wireless Local Area Network (LAN) or Bluetooth (registered trademark). In addition, these communications may be realized by wire.

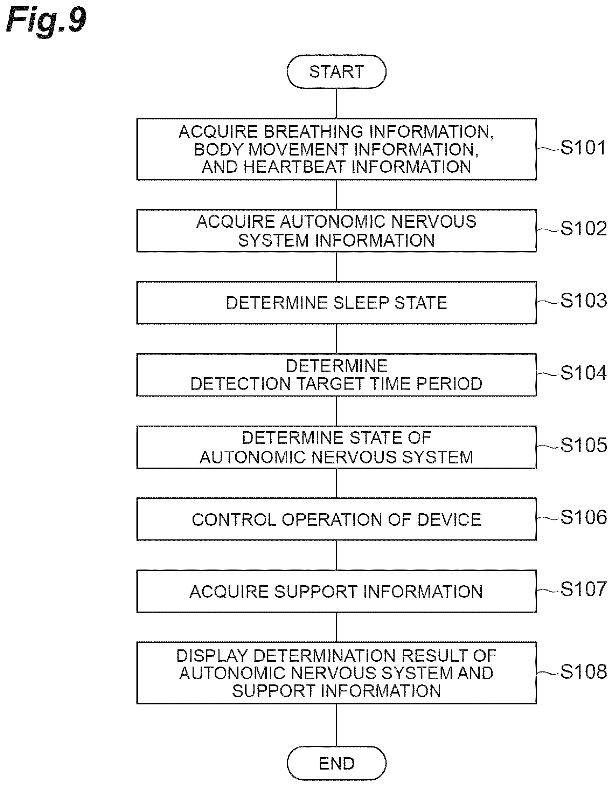

One example of the operation of the autonomic nervous system determination system 101 will be described. FIG. 9 is a flowchart showing one example of the operation of the autonomic nervous system determination system 101. Before the autonomic nervous system determination system 101 is operated, first, the sensor unit 111 is attached to the cushion body 110. The sensor unit 111 is attached to the cushion body 110 such that the sensor unit 111 does not come into contact with a user when the body of the user is placed on the cushion body 110. For example, the sensor unit 111 is disposed between the core material 102 and the cover fabric 103. Next, the body of the user is laid on the cushion body 110 to which the sensor unit 111 is attached. The body of the user is placed, for example, on the cushion body 110 so as to come into contact with the front fabric 131 of the cover fabric 103.

The sensor unit 111 acquires the breathing information, the body movement information, and the heartbeat information from body movement associated with the breathing of the user, body movement associated with heartbeat, and body movement not associated with breathing and heartbeat (step S101). In step S101, the sensor unit 111 detects body movement associated with the breathing of the user, body movement associated with heartbeat, and body movement not associated with breathing and heartbeat. The sensor unit 111 outputs a signal indicating the body movement associated with breathing, a signal indicating the body movement associated with heartbeat, and a signal indicating the body movement not associated with breathing and heartbeat to the outside of the sensor unit 111.

Next, the autonomic nervous system acquisition unit 112 acquires the autonomic nervous system information from the heartbeat information (step S102). In step S102, for example, the autonomic nervous system acquisition unit 112 acquires the heart rate variability of the user by analyzing the heartbeat information acquired by the sensor unit 111. In step S102, the autonomic nervous system acquisition unit 112 acquires the autonomic nervous system information from the heart rate variability of the user. The autonomic nervous system information includes, for example, information indicating the frequency of the R wave of heartbeat.

The sleep state acquisition unit 113 acquires the sleep state of the user from the breathing information, the body movement information, and the heartbeat information acquired by the sensor unit 111 (step S103). For example, the sleep state acquisition unit 113 acquires the sleep state from signals output from the sensor unit 111. In step S103, the sleep state acquisition unit 113 acquires, for example, the sleep stage of the user from the breathing information, the body movement information, and the heartbeat information.

The time period determination unit 114 determines a detection target time period based on the body movement information acquired by the sensor unit 111 (step S104). In step S104, the time period determination unit 114 determines the detection target time period based on the signal indicating body movement not associated with the breathing and heartbeat of the user that is acquired by the sensor unit 111. As a specific example, the time period determination unit 114 sets, as the detection target time periods, a time period during which the supine position continues for a certain period of time (as one example, one minute) or longer and a time period during which the lateral position continues for a certain period of time (as one example, one minute) or longer.

The autonomic nervous system determination unit 115 determines the state of the autonomic nervous system of the user based on both the autonomic nervous system information acquired by the autonomic nervous system acquisition unit 112 and the sleep state acquired by the sleep state acquisition unit 113 (step S105). In step S105, the autonomic nervous system determination unit 115 determines the state of the autonomic nervous system of the user in the detection target time period determined by the time period determination unit 114. For example, the autonomic nervous system determination unit 115 executes frequency analysis on the heart rate variability included in the autonomic nervous system information, and determines the state of the autonomic nervous system based on the power spectrum of each frequency. The autonomic nervous system determination unit 115 calculates, for example, a sympathetic nervous system index and a parasympathetic nervous system index obtained by the frequency analysis of the heart rate variability.

In step S105, the autonomic nervous system determination unit 115 determines, for example, the state of the autonomic nervous system according to the sleep state acquired by the sleep state acquisition unit 113. The autonomic nervous system determination unit 115 may determine the state of the autonomic nervous system of the user, for example, by comparing the autonomic nervous system information of the user acquired by the autonomic nervous system acquisition unit 112 with the information indicating the state of the autonomic nervous system that is stored in advance. The determination of the state of the autonomic nervous system by the autonomic nervous system determination unit 115 is performed, for example, in the following procedure.

The autonomic nervous system determination unit 115 calculates the level of activity of the user based on the sympathetic nervous system index. The autonomic nervous system determination unit 115 calculates the level of relaxation of the user based on the parasympathetic nervous system index. The autonomic nervous system determination unit 115 classifies the state of the autonomic nervous system of the user into a plurality of (as one example, four) zones (for example, an ideal zone, a fatigue zone, a stress zone, and an internal rhythm disturbance zone) based on the level of relaxation at sleep onset and the level of activity upon waking up.

For example, the autonomic nervous system determination unit 115 performs the above classification based on whether the level of relaxation and the level of activity are high or low. The term "level of relaxation being high" indicates, for example, that the parasympathetic nervous system index is equal to or larger than a predetermined threshold value. The term "level of relaxation being low" indicates, for example, that the parasympathetic nervous system index is less than the predetermined threshold value. The term "level of activity being high" indicates, for example, that the sympathetic nervous system index is equal to or larger than a predetermined threshold value. The term "level of activity being low" indicates, for example, that the sympathetic nervous system index is less than the predetermined threshold value. The predetermined threshold values can be changed as appropriate.

When the level of relaxation at sleep onset is high and the level of activity upon waking up is high, the autonomic nervous system determination unit 115 determines that the state of the autonomic nervous system of the user is located in the ideal zone. When the level of relaxation at sleep onset is low and the level of activity upon waking up is high, the autonomic nervous system determination unit 115 determines that the state of the autonomic nervous system of the user is located in the stress zone. When the level of relaxation at sleep onset is high and the level of activity upon waking up is low, the autonomic nervous system determination unit 115 determines that the state of the autonomic nervous system of the user is located in the fatigue zone. When the level of relaxation at sleep onset is low and the level of activity upon waking up is low, the autonomic nervous system determination unit 115 determines that the state of the autonomic nervous system of the user is located in the internal rhythm disturbance zone (autonomic nervous system disturbance zone).

The device control unit 116 controls the operation of the device 202 using the determination result of the autonomic nervous system determination unit 115 (step S106). In step S106, for example, the device control unit 116 controls the operation of an air conditioner installed in the bedroom of the user. As one example, the device control unit 116 may control the operation of the air conditioner such that the temperature in the bedroom increases (or decreases) according to the determination result of the autonomic nervous system by the autonomic nervous system determination unit 115.

In step S106, for example, the device control unit 116 may adjust the brightness of the bedroom. In this case, the device 202 is, for example, a lighting fixture such as a lamp. When the parasympathetic nervous system is dominant over the sympathetic nervous system despite the fact that it is the time the user is scheduled to wake up, the device control unit 116 may control the lighting fixture to brighten the bedroom more than usual. As a result of the bedroom becoming brighter, it can be expected that the effect of making it easier for the user to wake up is obtained and the sympathetic nervous system becomes dominant over the parasympathetic nervous system.

In step S106, for example, the device control unit 116 may control the device 202 to adjust the temperature of the bed of the user. In this case, the device 202 is, for example, an electric blanket that is placed over the user. As described above, the autonomic nervous system during REM sleep is more easily disturbed compared to the autonomic nervous system during non-REM sleep. When the sympathetic nervous system is dominant over the parasympathetic nervous system despite the fact that the sleep state of the user is the state of non-REM sleep, there is a possibility that the user is stressed. In this case, the device control unit 116 may control a heating device to increase or decrease the temperature of the bed.

The support information acquisition unit 117 acquires support information using the determination result of the autonomic nervous system determination unit 115 (step S107). For example, the support information acquisition unit 117 acquires the support information from the server 201. The support information acquisition unit 117 acquires the support information using, for example, table data stored in a database stored in the server 201, and the determination result of the autonomic nervous system determination unit 115.

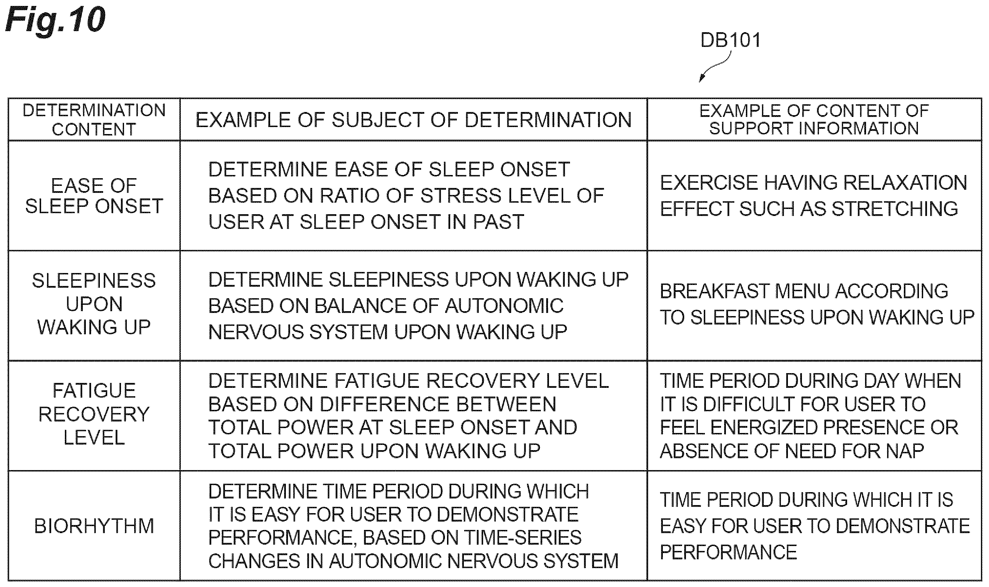

A specific example of the operation in step S107 will be described with reference to FIG. 10. FIG. 10 is a table showing one example of a table stored in a support database DB101. The support database DB101 is stored in, for example, the server 201. However, the support database DB101 may be part of data held by the autonomic nervous system determination application 120, or may be a database downloaded to the information terminal. A plurality of support information acquired by the support information acquisition unit 117 are stored in the support database DB101 in advance.

As described above, the support information acquisition unit 117 determines, for example, at least one of the ease of sleep onset, sleepiness upon waking up, fatigue recovery level, and biorhythm based on the determination result of the autonomic nervous system determination unit 115. As one example, in the table of the support database DB101, the contents of the determinations executed by the support information acquisition unit 117 are described in the left column. The subjects of the determinations by the support information acquisition unit 117 are described in the center column of the table of the support database DB101. The contents of the support information acquired by the support information acquisition unit 117 are described in the right column of the table of the support database DB101. In step S107, the support information acquisition unit 117 refers to the support database DB101. For example, the support information acquisition unit 117 refers to the support database DB101 by communicating with the server 201. At this time, the support information acquisition unit 117 acquires the support information from the table of the support database DB101.

In step S107, the support information acquisition unit 117 determines, for example, the ease of sleep onset. The support information acquisition unit 117 determines, for example, the ease of sleep onset based on the stress level acquired by the autonomic nervous system acquisition unit 112. As one example, the support information acquisition unit 117 determines the ease of sleep onset of the user based on the ratio of the stress level of the user at sleep onset in the past to a predetermined stress level. For example, the predetermined stress level can be changed as appropriate by a user, and may be set in advance by the user. The stress level at sleep onset in the past may be, for example, the average value of a plurality of stress levels calculated in the past. The "past" refers to a time before the time the state of the autonomic nervous system of the user is determined using the autonomic nervous system determination system 101. The support information acquisition unit 117 calculates, for example, the ratio of the stress level of the user at sleep onset in the past to the predetermined stress level based on the information indicating the balance of the autonomic nervous system at sleep onset acquired in the past by the autonomic nervous system determination unit 115.

For example, when the sympathetic nervous system of the user is dominant over the parasympathetic nervous system at sleep onset in the past, the support information acquisition unit 117 determines that the ratio of the stress level of the user is high and it is difficult for the user to fall asleep. At this time, the support information acquisition unit 117 acquires, as the support information, a comment that encourages exercise having a relaxation effect. As one example, the support information acquisition unit 117 acquires, from the support database DB101, a comment stating that it would be better to perform stretching before going to bed.

In step S107, the support information acquisition unit 117 determines, for example, sleepiness upon waking up. The support information acquisition unit 117 determines sleepiness upon waking up based on the information indicating the balance of the autonomic nervous system upon waking up acquired by the autonomic nervous system determination unit 115. When the parasympathetic nervous system is dominant over the sympathetic nervous system upon waking up, the support information acquisition unit 117 determines that the sleepiness upon waking up is large. At this time, the support information acquisition unit 117 acquires, as the support information, information indicating a breakfast menu according to sleepiness upon waking up. As one example, the support information acquisition unit 117 acquires, from the support database DB101, a comment stating that it would be better to have caffeine at breakfast.

For example, the support information acquisition unit 117 calculates a fatigue recovery level, and determines the calculated fatigue recovery level. The support information acquisition unit 117 determines the fatigue recovery level based on a difference between the total power at sleep onset and the total power upon waking up. The total power refers to the integral value of the power spectrum in a predetermined frequency band (as one example, 0.04 Hz to 0.4 Hz) when HRV frequency analysis is executed on the electrocardiogram of the user acquired by the sensor unit 111.

The total power tends to decrease when the fatigue level is high, and the total power tends to increase when the fatigue level is low. By calculating the difference between the total power at sleep onset and the total power upon waking up, the extent to which the user can be recovered from fatigue through sleep can be calculated as the fatigue recovery level. For example, the support information acquisition unit 117 calculates the calculated fatigue recovery level as a numerical value, and determines whether the calculated fatigue recovery level is equal to or larger than a threshold value. As a result of performing a determination on the fatigue recovery level, the support information acquisition unit 117 acquires, as the support information, for example, information indicating the time period during the day when it is difficult for the user to feel energized and information indicating the presence or absence of the need for a nap during the day.

In step S107, the support information acquisition unit 117 acquires, for example, biorhythm from the breathing information, the body movement information, and the heartbeat information acquired by the sensor unit 111. For example, the biorhythm is represented as the degree of sleepiness according to the time period. The support information acquisition unit 117 determines a time period during which it is easy for the user to demonstrate performance, based on time-series changes in the autonomic nervous system. At this time, the support information acquisition unit 117 acquires, as the support information, information indicating the time period during the day when it is easy for the user to demonstrate performance, based on the calculated biorhythm. As one example, the support information acquisition unit 117 acquires, from the support database DB101, a comment stating that the user can demonstrate higher performance in the morning than in the afternoon.

Next, the display unit 118 displays the support information acquired by the support information acquisition unit 117 (step S108). As described above, the display unit 118 displays, for example, at least one of the comment that encourages exercise having a relaxation effect, the information indicating a breakfast menu according to sleepiness upon waking up, the information indicating the time period during the day when it is difficult for the user to feel energized, the information indicating the presence or absence of the need for a nap during the day, and the information indicating the time period during the day when it is easy for the user to demonstrate performance. Then, the display unit 118 displays the determination result of the autonomic nervous system determination unit 115 (step S108).

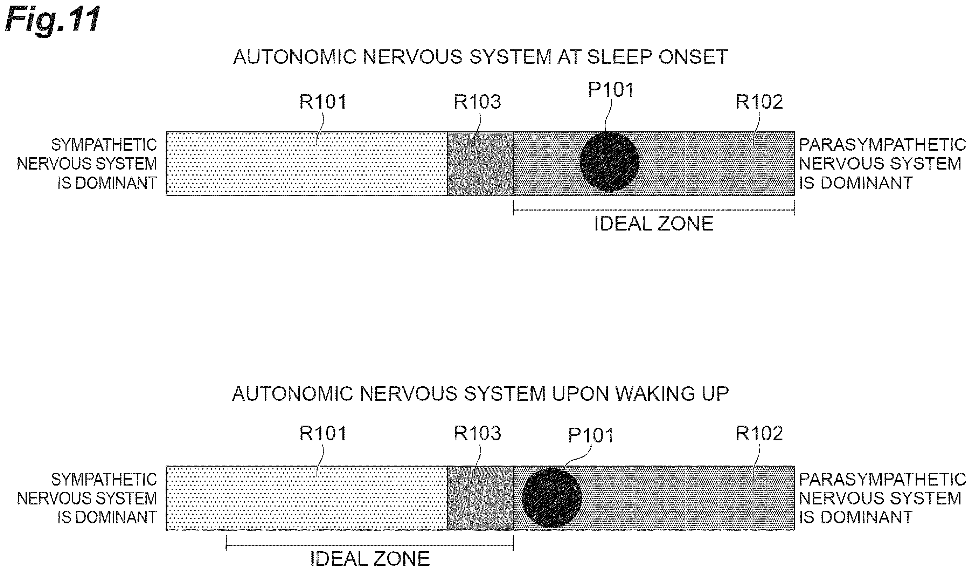

A specific example of the operation in step S108 will be described with reference to FIGS. 11 to 13. FIG. 11 is a view showing one example of a display of the determination result of the autonomic nervous system at sleep onset, and one example of a display of the determination result of the autonomic nervous system upon waking up. In the example of FIG. 11, the display unit 118 displays a sympathetic region R101, a parasympathetic region R102, and an antagonistic region R103.

As one example, the display unit 118 displays the determination result of the autonomic nervous system by displaying a point P101 in a bar-shaped figure in which the sympathetic region R101, the antagonistic region R103, and the parasympathetic region R102 are aligned in order. As a specific example, the figure extends in a horizontal direction, the sympathetic region R101 is located to the left of the antagonistic region R103, and the parasympathetic region R102 is located to the right of the antagonistic region R103. The antagonistic region R103 is located between the sympathetic region R101 and the parasympathetic region R102. In step S108, the display unit 118 displays the point P101 on the sympathetic region R101, the parasympathetic region R102, or the antagonistic region R103, based on the determination results of the autonomic nervous system at sleep onset and upon waking up.

In step S108, the display unit 118 displays, for example, the information indicating the balance of the autonomic nervous system at sleep onset and upon waking up acquired by the autonomic nervous system determination unit 115. The point P101 being displayed on the sympathetic region R101 indicates that the sympathetic nervous system is dominant over the parasympathetic nervous system. The point P101 being displayed on the parasympathetic region R102 indicates that the parasympathetic nervous system is dominant over the sympathetic nervous system. The point P101 being displayed on the antagonistic region R103 indicates that the sympathetic nervous system and the parasympathetic nervous system are antagonistic to each other.

at sleep onset, it is preferable that the parasympathetic nervous system is dominant over the sympathetic nervous system and the user is in a relaxed state. In this case, in the determination result of the autonomic nervous system at sleep onset, the point P101 is located on the parasympathetic region R102 (an ideal zone in FIG. 11). Upon waking up, it is preferable that the sympathetic nervous system is dominant over the parasympathetic nervous system and the user is in an active state. In this case, in the determination result of the autonomic nervous system upon waking up, the point P101 is located on the sympathetic region R101 or the antagonistic region R103 (an ideal zone in FIG. 11). Incidentally, FIG. 11 shows an example in which at sleep onset, the parasympathetic region R102 serves as an ideal zone, and upon waking up, the sympathetic region R101 and the antagonistic region R103 serve as an ideal zone. However, at sleep onset, the parasympathetic region R102 and the antagonistic region R103 may serve as an ideal zone, and upon waking up, the sympathetic region R101 may serve as an ideal zone.

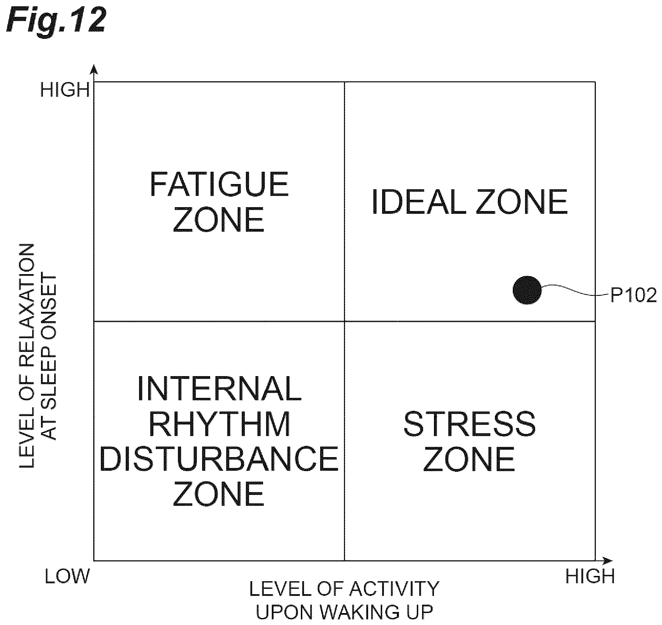

FIG. 12 is a view showing another example of a display of the determination result of the autonomic nervous system. The vertical axis shown in FIG. 12 represents the level of relaxation at sleep onset, and the horizontal axis represents the level of activity upon waking up. In the example of FIG. 12, the autonomic nervous system determination unit 115 classifies the state of the autonomic nervous system of the user into one of an ideal zone, a fatigue zone, a stress zone, and an internal rhythm disturbance zone, based on the level of relaxation at sleep onset and the level of activity upon waking up.

In step S108, the display unit 118 displays, for example, information indicating the evaluation of the state of the autonomic nervous system of the user during sleep. The display unit 118 displays a point P102 on a graph shown in FIG. 12, based on the level of relaxation at sleep onset and the level of activity upon waking up. For example, when the autonomic nervous system determination unit 115 determines that the level of relaxation at sleep onset is high and the level of activity upon waking up is high, the display unit 118 displays the point P102 at a location in the ideal zone of the graph. For example, when the autonomic nervous system determination unit 115 determines that the level of relaxation at sleep onset is low and the level of activity upon waking up is high, the display unit 118 displays the point P102 at a location in the stress zone of the graph.

For example, when the autonomic nervous system determination unit 115 determines that the level of relaxation at sleep onset is high and the level of activity upon waking up is low, the display unit 118 displays the point P102 at a location in the fatigue zone of the graph. For example, when the autonomic nervous system determination unit 115 determines that the level of relaxation at sleep onset is low and the level of activity upon waking up is low, the display unit 118 displays the point P102 in the internal rhythm disturbance zone of the graph.

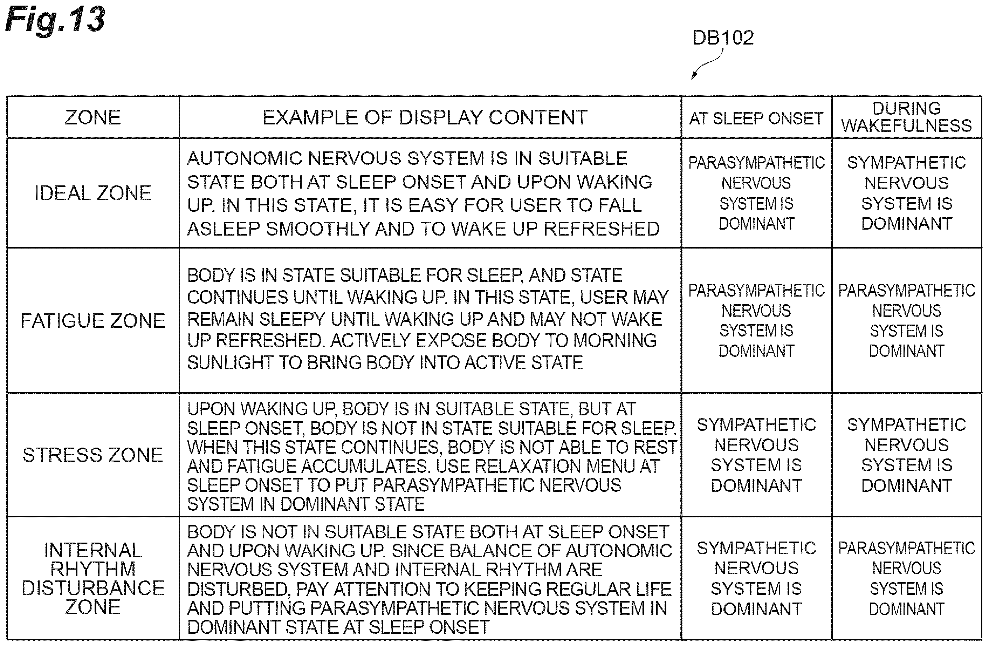

FIG. 13 shows one example of a table of an autonomic nervous system database DB102. The autonomic nervous system database DB102 is stored in, for example, the server 201. However, the autonomic nervous system database DB102 may be part of data held by the autonomic nervous system determination application 120, or may be a database downloaded to the information terminal. In step S108, the display unit 118 refers to the autonomic nervous system database DB102. A plurality of display contents displayed by the display unit 118 are stored in the autonomic nervous system database DB102 in advance.

In step S108, the display unit 118 displays, for example, information presenting advice according to the state of the autonomic nervous system of the user during sleep. The display unit 118 acquires information indicating a zone in which the state of the autonomic nervous system of the user during sleep is located, based on the level of relaxation at sleep onset and the level of activity upon waking up. Then, the display unit 118 refers to the autonomic nervous system database DB102, and displays a content according to the acquired zone.

When the state of the autonomic nervous system of the user during sleep is located in the ideal zone, the display unit 118 displays an indication that at sleep onset, the parasympathetic nervous system is dominant over the sympathetic nervous system, and upon waking up, the sympathetic nervous system is dominant over the parasympathetic nervous system. As a specific example, the display unit 118 displays a message that "the autonomic nervous system is in a suitable state both at sleep onset and upon waking up. In this state, it is easy for the user to fall asleep smoothly and to wake up refreshed".

When the state of the autonomic nervous system of the user during sleep is located in the fatigue zone, the display unit 118 displays an indication that both at sleep onset and upon waking up, the parasympathetic nervous system is dominant over the sympathetic nervous system. As a specific example, the display unit 118 displays a message that "the body is in a state suitable for sleep, and the state continues until waking up. In this state, the user may remain sleepy until waking up and may not wake up refreshed. Actively expose the body to morning sunlight to bring the body into an active state".

When the state of the autonomic nervous system of the user during sleep is located in the stress zone, the display unit 118 displays an indication that both at sleep onset and upon waking up, the sympathetic nervous system is dominant over the parasympathetic nervous system. As a specific example, the display unit 118 displays a message that "upon waking up, the body is in a suitable state, but at sleep onset, the body is not in a state suitable for sleep. When this state continues, the body is not able to rest and fatigue accumulates. Use a relaxation menu at sleep onset to put the parasympathetic nervous system in a dominant state".

When the state of the autonomic nervous system of the user during sleep is located in the internal rhythm disturbance zone, the display unit 118 displays an indication that at sleep onset, the sympathetic nervous system is dominant over the parasympathetic nervous system and upon waking up, the parasympathetic nervous system is dominant over the sympathetic nervous system. As a specific example, the display unit 118 displays a message that "the body is not in a suitable state both at sleep onset and upon waking up. Since the balance of the autonomic nervous system and the internal rhythm are disturbed, pay attention to keeping a regular life and putting the parasympathetic nervous system in a dominant state at sleep onset".

One example of the steps of the autonomic nervous system determination system has been described above. However, the contents and order of the steps of the autonomic nervous system determination system are not limited to the above-described example, and can be changed as appropriate.

Subsequently, as one example, actions and effects of the autonomic nervous system determination system 101 will be described. The autonomic nervous system determination system 101 acquires the sleep state of the user from the breathing information, the body movement information, and the heartbeat information. The autonomic nervous system determination system 101 determines the state of the autonomic nervous system of the user based on both the autonomic nervous system information acquired by the autonomic nervous system acquisition unit 112 and the sleep state acquired by the sleep state acquisition unit 113. For example, it is desirable that at sleep onset, the parasympathetic nervous system is dominant over the sympathetic nervous system, and upon waking up, the sympathetic nervous system is dominant over the parasympathetic nervous system. In the autonomic nervous system determination system 101, the state of the autonomic nervous system can be determined by taking into account the sleep state indicating whether the sleep of the user is deep or shallow or whether the user can sleep soundly.

For example, the autonomic nervous system tends to be disturbed more easily during REM sleep compared to during non-REM sleep. By acquiring the sleep state of the user, it can be determined whether the user is in REM sleep, and therefore, the autonomic nervous system can be evaluated by taking into account whether the user is in the state of REM sleep. Therefore, the state of the autonomic nervous system can be determined according to the sleep state of the user.

For example, the autonomic nervous system of the user upon waking up tends to differ greatly between when the user is under high stress and when the user is not under much stress. By acquiring the sleep state of the user, it can be determined whether the user has woken up, and therefore, the autonomic nervous system can be evaluated by taking into account that the user is in an awakened state. Therefore, the state of the autonomic nervous system can be determined according to the sleep state of the user.

As one example, the autonomic nervous system determination system 101 includes the time period determination unit 114 that determines a detection target time period during which the body movement of the user does not occur between the bedtime and the wake-up time, based on the body movement information. The autonomic nervous system determination unit 115 determines the state of the autonomic nervous system of the user in the detection target time period. For example, unlike during wakefulness, during sleep, since it is normal to turn over during sleep, a state of not moving the body cannot be maintained. When the state of the autonomic nervous system in a time period during which the body movement of the user occurs is determined, the accuracy of the autonomic nervous system information to be acquired may decrease due to the body movement, and therefore, the determination accuracy for the state of the autonomic nervous system may decrease. Therefore, a decrease in determination accuracy can be suppressed by determining the state of the autonomic nervous system in the detection target time period during which the body movement of the user does not occur.

As one example, the autonomic nervous system determination system 101 includes the device control unit 116 that controls the operation of the device 202, which constitutes the environment around the user, using the determination result of the autonomic nervous system determination unit 115. In this case, for example, the operation of a device such as an air conditioner disposed around the user can be controlled according to the state of the autonomic nervous system of the user. Therefore, the sleep environment of the user can be made even more comfortable according to the determined state of the autonomic nervous system.

As one example, the autonomic nervous system determination system 101 includes the support information acquisition unit 117 that acquire the support information, which is information that supports the life of the user, using the determination result of the autonomic nervous system determination unit 115, and the display unit 118 that displays the support information. In this case, the life of the user can be supported by displaying the support information according to the determined state of the autonomic nervous system of the user. For example, the content of a meal according to the determined state of the autonomic nervous system can be proposed. Therefore, the life of the user can be made even more comfortable using the determination result of the autonomic nervous system of the user.

Next, a specific example of the life improvement system will be described. The life improvement system improves the life of a user. For example, the life improvement system may be used for personal or household purposes, or may be used in research institutes or the like for testing and research purposes. The life improvement system may be used in hospitals or the like for treatment. The "user" refers to a person who is the subject of life improvement by the life improvement system. The user is, for example, a person who has a health risk caused by disturbance in life or the like, a person who receives treatment at a hospital or the like, or a person who wishes to improve his or her own life.

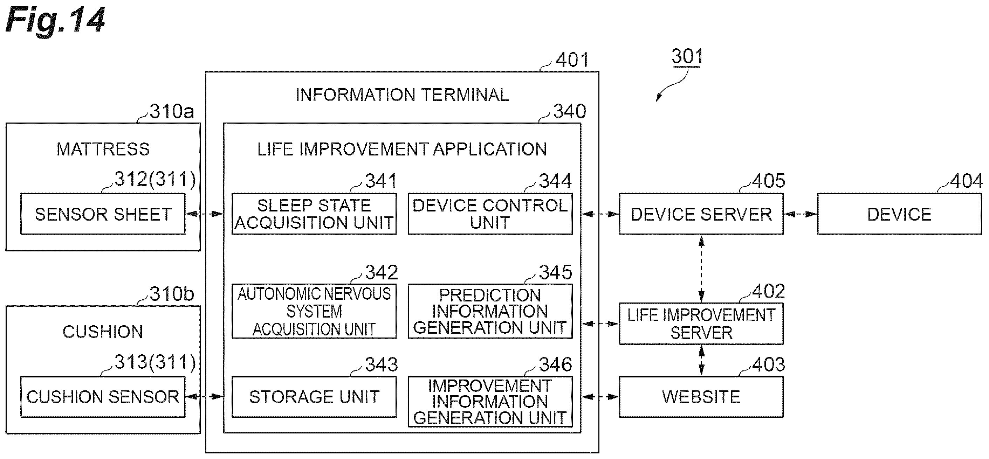

FIG. 14 is a block diagram showing a life improvement system 301 as one example. The life improvement system 301 includes a sensor unit 311, an information terminal 401, and a life improvement server 402.

The information terminal 401 is, for example, a mobile terminal. The "mobile terminal" is, for example, a portable information terminal such as a mobile phone including a smartphone, a tablet, a notebook computer, or a wearable terminal such as a wristwatch. The information terminal 401 may be a terminal other than a mobile terminal, or may be, for example, a desktop computer. The information terminal 401 includes, as one example, a processor (for example, a CPU) that executes an operating system, software (application), and the like; a main storage unit composed of a ROM and a RAM; an auxiliary storage unit composed of a flash memory and the like; a communication control unit composed of a wireless communication module and the like; an input device; and an output device such as a display. However, the configuration of the information terminal 401 is not limited to the above-described configuration, and can be changed as appropriate.

In the information terminal 401, a life improvement application 340 is executed as an application program. The life improvement application 340 may be an application downloaded to the information terminal 401 and executed on the information terminal 401, or may be executed on the life improvement server 402. The life improvement application 340 may be an application downloaded from the life improvement server 402. Hereinafter, an example in which the life improvement application 340 is an application downloaded to the information terminal 401 and the functions of the life improvement application 340 are executed on the information terminal 401 will be described.

Each function of the life improvement application 340 is realized by loading predetermined software into the processor or the main storage unit and executing the software. The data or database required to execute the functions of the life improvement application 340 is stored in the main storage unit or the auxiliary storage unit.

Each functional element of the information terminal 401 is realized by loading predetermined software into the processor or the storage unit (for example, the main storage unit or the auxiliary storage unit described above) and executing the software. The data or database used for the processing of the life improvement server 402 is stored in a storage unit.

The life improvement application 340 may be a distributed processing system composed of a plurality of computers, or may be a client-server system or a cloud system. The life improvement application 340 includes, for example, a main module, a data acquisition module, a determination module, and an output module. The data acquisition module, the determination module, and the output module are executed, thereby causing each functional element of the life improvement application 340 to function. As one example, the life improvement application 340 may be provided in a state where the life improvement application 340 is permanently recorded on a tangible storage medium such as a CD-ROM, a DVD-ROM, or a semiconductor memory. The life improvement application 340 may be provided via a communication network as a data signal superimposed on a carrier wave. A functional configuration of the life improvement application 340 will be described later.

The information terminal 401 is a terminal that allows input of information by accepting the operation of the user. The information terminal 401 can transmit the input information to the life improvement server 402. As one example, the information terminal 401 transmits subjective information, which is information indicating the subjective evaluation of the user, to the life improvement server 402.

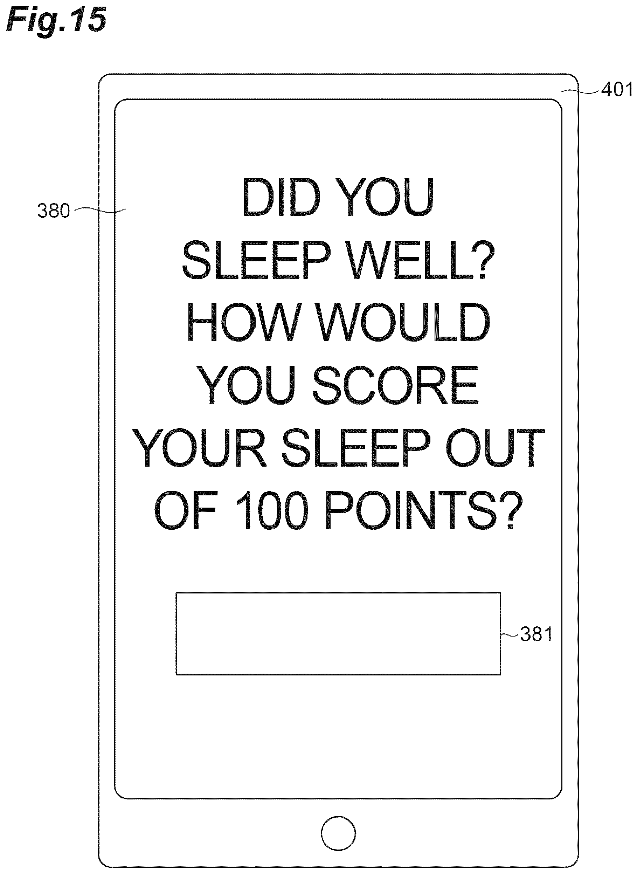

FIG. 15 is a view showing an example of an input screen 380 of the subjective information that is displayed on the information terminal 401. The input screen 380 is a screen to which the subjective information is input. The subjective information includes, for example, a subjective evaluation of sleep. The input screen 380 includes a sleep evaluation input portion 381 that is a portion to which the subjective evaluation of sleep is input. For example, the subjective evaluation of sleep is input into the sleep evaluation input portion 381 as a score, with 100 points being the full score.

The information terminal 401 displays prediction information and improvement information. Details of the prediction information and the improvement information will be described later.

The information terminal 401 can access a predetermined website 403. As one example, the website 403 includes at least one of an online shopping site that handles products including at least one of clothing, food, soap, and cosmetic, and a travel site for advertising travel and accepting reservations for accommodations. The website 403 transmits information to the information terminal 401 in response to a request from the information terminal 401. The information transmitted by the website 403 includes, for example, a Uniform Resource Locator (URL). The information transmitted from the website 403 to the information terminal 401 is displayed on the information terminal 401 and provided to the user. The website 403 may be accessible from the life improvement server 402.

The sensor unit 311 acquires information about the body of the user. The sensor unit 311 acquires at least one of breathing information, body movement information, and heartbeat information. As one example, the sensor unit 311 acquires the breathing information, the body movement information, and the heartbeat information. The contents of the information acquired by the sensor unit 311 can be changed as appropriate. The sensor unit 311 may further acquire at least one of information indicating the body temperature of the user, information indicating blood pressure, and information indicating blood glucose level. The sensor unit 311 includes a sensor sheet 312 attached to a mattress 310a, and a cushion sensor 313 attached to a cushion 310b.

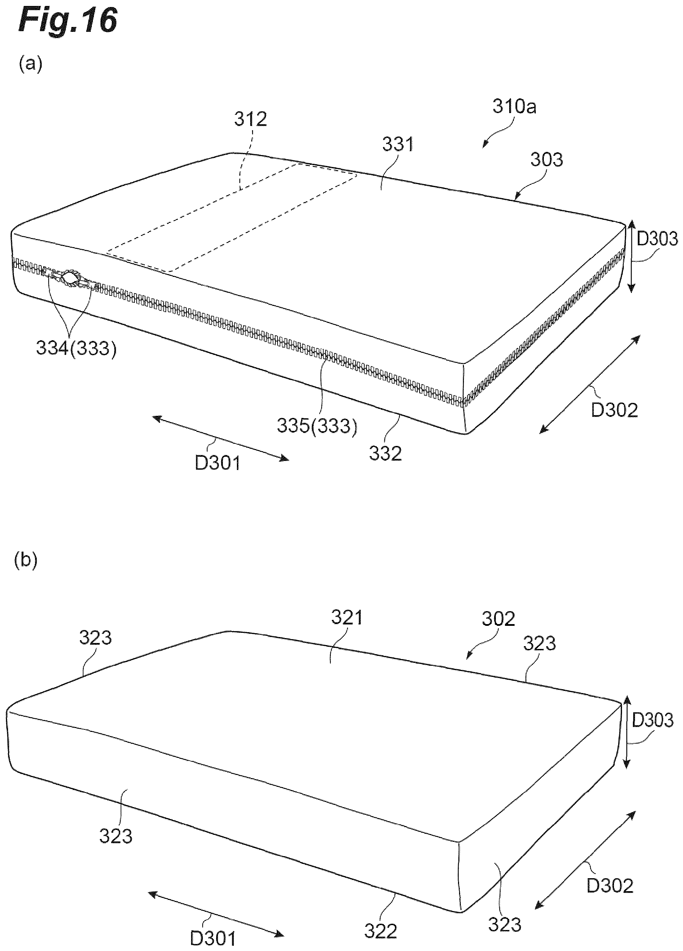

(a) in FIG. 16 is a perspective view showing the mattress 310a to which the sensor sheet 312 is attached. (b) in FIG. 16 is a perspective view showing a core material 302 constituting the mattress 310a shown in (a) of FIG. 16. For example, the sensor sheet 312 is attachable to and detachable from the mattress 310a. The sensor sheet 312 acquires vital data of the user lying on the mattress 310a. As one example, the sensor sheet 312 may acquire an electrocardiogram. The sensor sheet 312 acquires the breathing information, the body movement information, and the heartbeat information of the user during sleep.

For example, the sensor sheet 312 includes a sheet-shaped fabric on which a thread-shaped sensor is embroidered, and the position of the sheet-shaped fabric with respect to the mattress 310a can be changed. The thread-shaped sensor of the sensor sheet 312 is fixed, for example, by being embroidered such that the thread-shaped sensor spreads out two-dimensionally on the sheet-shaped fabric. For example, a piezoelectric sensor of the sensor sheet 312 generates electrical signals corresponding to the applied load of the body of the user based on the load. The sensor sheet 312 acquires the electrical signals as the breathing information, the body movement information, and the heartbeat information described above.

The sensor sheet 312 includes, for example, the thread-shaped sensor (as one example, a piezoelectric sensor), and a communication unit that outputs the breathing information, the body movement information, and the heartbeat information as electrical signals generated by the thread-shaped sensor to the outside of the sensor sheet 312. An example in which the sensor sheet 312 includes a piezoelectric sensor has been described above. However, the type of the sensor of the sensor sheet 312 is not limited to a piezoelectric sensor, and is not particularly limited. For example, the sensor sheet 312 may include an acceleration sensor.

The sensor sheet 312 detects body movement associated with the breathing of the user. The sensor sheet 312 outputs a signal indicating the body movement associated with breathing to the outside of the sensor sheet 312. The sensor sheet 312 detects body movement associated with the heartbeat of the user. The sensor sheet 312 outputs a signal indicating the body movement associated with heartbeat to the outside of the sensor sheet 312. The sensor sheet 312 detects body movement not associated with the breathing and heartbeat of the user. The sensor sheet 312 outputs a signal indicating the body movement not associated with breathing and heartbeat to the outside of the sensor sheet 312.

For example, the sensor sheet 312 is used while being attached to the mattress 310a on which the body of the user is placed. The mattress 310a has a rectangular shape in a plan view. The mattress 310a extends in a longitudinal direction D301 and a lateral direction D302 orthogonal to the longitudinal direction D301. The mattress 310a has a thickness in a thickness direction D303 orthogonal to both the longitudinal direction D301 and the lateral direction D302.

A length of the mattress 310a in the longitudinal direction D301 is, for example, 120 cm or more and 210 cm or less (as one example, 195 cm). A length of the mattress 310a in the lateral direction D302 is, for example, 70 cm or more and 180 cm or less (as one example, 97 cm). A length of the mattress 310a in the thickness direction D303 is, for example, 3 cm or more and 40 cm or less (as one example, 9 cm).

As one example, the user of the mattress 310a places his or her own body on the mattress 310a. At this time, a direction in which the body of the lying user extends (namely, a direction in which the head and legs of the user are connected to each other) coincides with, for example, the longitudinal direction D301 of the mattress 310a.

As shown in (b) of FIG. 16, the mattress 310a includes the core material 302 housed inside a cover fabric 303 to be described later. As one example, the core material 302 has a rectangular shape in a plan view. The core material 302 includes, for example, a content and a bag that houses the content. The content is, for example, urethane foam or polyester.

The core material 302 has a rectangular parallelepiped shape. The core material 302 has an upper surface 321 on which the body of the user is placed, and a lower surface 322 facing opposite the upper surface 321. The upper surface 321 is a surface facing one side (the upper side in (b) of FIG. 16) in the thickness direction D303. The lower surface 322 is a surface facing the other side (the lower side in (b) of FIG. 16) in the thickness direction D303. The core material 302 has a plurality of side surfaces 323 connecting the upper surface 321 and the lower surface 322.

As shown in (a) of FIG. 16, the mattress 310a includes the cover fabric 303. As one example, the cover fabric 303 has a bag shape, and has a rectangular shape in a plan view. The cover fabric 303 includes, for example, a front fabric 331 covering the upper surface 321 of the core material 302; a back fabric 332 covering the lower surface 322 of the core material 302; and an opening and closing member 333 connecting the front fabric 331 and the back fabric 332 to each other. The opening and closing member 333 is provided at a position on the cover fabric 303 which corresponds to the side surfaces 323 of the core material 302. When viewed in the thickness direction D303, the opening and closing member 333 is located outside the side surfaces 323 of the core material 302. The cover fabric 303 is attachable to and detachable from the core material 302.

The opening and closing member 333 is, as one example, a so-called double slider. Namely, the opening and closing member 333 may include two slide members 334 and an opening and closing portion 335 that opens and closes the front fabric 331 and the back fabric 332 when the slide members 334 are slid.

In this case, the front fabric 331 and the back fabric 332 can be opened by sliding one slide member 334 along the opening and closing portion 335 in one direction (a clockwise direction in (a) of FIG. 16) and sliding the other slide member 334 along the opening and closing portion 335 in the other direction (a counterclockwise direction in (a) of FIG. 16).

The front fabric 331 and the back fabric 332 can be closed by sliding the one slide member 334 along the opening and closing portion 335 in the other direction and sliding the other slide member 334 along the opening and closing portion 335 in the one direction. The opening and closing member 333 may be a so-called single slider. In this case, the opening and closing member 333 includes one slide member 334 and the opening and closing portion 335.

The sensor sheet 312 is disposed, for example, inside the cover fabric 303 having a bag shape. More specifically, the sensor sheet 312 is disposed between the core material 302 and the cover fabric 303. When the sensor sheet 312 is attached to the mattress 310a, for example, the sensor sheet 312 is disposed to extend in the lateral direction D302 of the mattress 310a. In this case, the sensor sheet 312 is located on the side opposite the body of the user when viewed from the cover fabric 303 of the mattress 310a. When the body of the user is placed on the mattress 310a, the sensor sheet 312 does not come into contact with the user. The sensor sheet 312 acquires the breathing information, the body movement information, and the heartbeat information of the user in a non-contact manner.

The sensor sheet 312 is attached, for example, at a position corresponding to the heart of the user in the longitudinal direction D301. As one example, the sensor sheet 312 is attached at any position between the position where the head of the user is placed and a position spaced apart in the longitudinal direction D301 of the mattress 310a (a position on the lower right side in (a) of FIG. 16). The attachment position of the sensor sheet 312 in the longitudinal direction D301 can be changed. A distance from the position where the head of the user is placed to the position where the sensor sheet 312 is attached may be, for example, 40 cm or more and 50 cm or less (as one example, 50 cm).

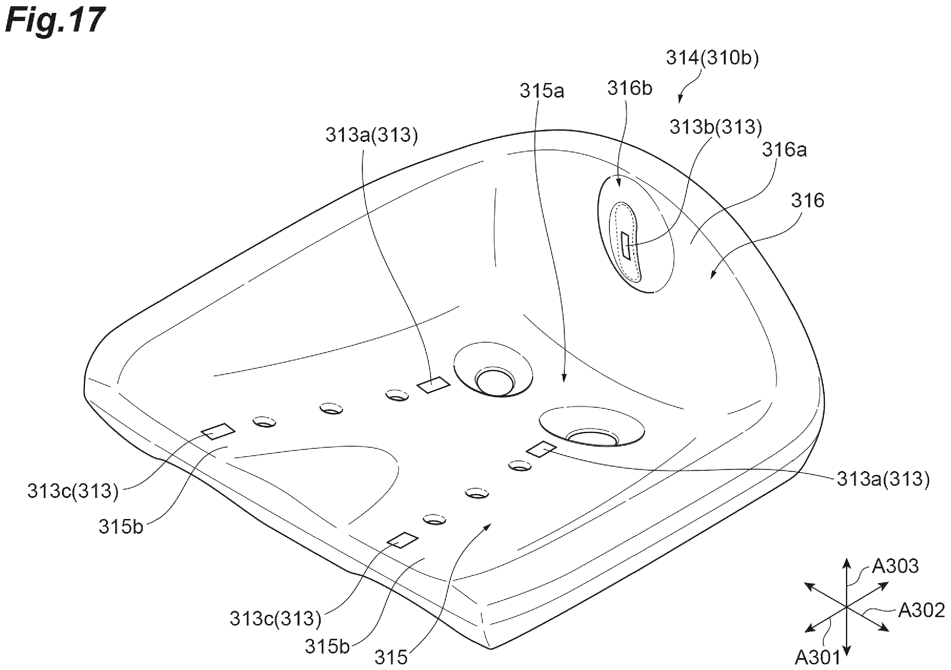

FIG. 17 is a perspective view showing a core material 314 of the cushion 310b to which the cushion sensor 313 is attached. The cushion 310b is configured, for example, by housing the core material 314 in a bag-shaped fabric. The core material 314 includes a seating portion 315 extending in a horizontal direction, and a lumbar support portion 316 extending upward from the seating portion 315. The core material 314 is made of, for example, a flexible material such as urethane foam.

The seating portion 315 extends in a first direction A301 and a second direction A302 intersecting the first direction A301. The first direction A301 is a front-rear direction when viewed from the user seated on the seating portion 315, and the second direction A302 is a left-right direction when viewed from the user seated on the seating portion 315. The seating portion 315 has a thickness in a third direction A303 intersecting both the first direction A301 and the second direction A302. For example, the third direction A303 is a vertical direction. The seating portion 315 includes a buttock support portion 315a and two thigh support portions 315b. The buttock support portion 315a and the thigh support portions 315b are disposed to be aligned along the first direction A301.

Hereinafter, a forward direction when viewed from the user seated on the seating portion 315 may be referred to as "front", "front side", or "forward", and a direction opposite the forward direction may be referred to as "rear", "rear side", or "rearward". However, the directions are provided for the convenience of description, and do not limit the position, orientation, and the like of each part.

The buttock support portion 315a is located on the rear side of the seating portion 315. The buttocks of the user are placed on the buttock support portion 315a. The buttock support portion 315a supports the buttocks of the user. The two thigh support portions 315b are aligned along the second direction A302 on the front side of the seating portion 315. The back sides of the thighs of the user are placed on the thigh support portions 315b. The thigh support portions 315b support the back sides of the thighs of the user.

The lumbar support portion 316 extends upward from an end portion (rear end) of the seating portion 315 in the first direction A301. The lumbar support portion 316 includes, for example, a general portion 316a extending in both the second direction A302 and the third direction A303, and a sacrum support portion 316b located at the center of the general portion 316a in the second direction A302. The general portion 316a is a portion of the lumbar support portion 316 other than the sacrum support portion 316b.

The sacrum support portion 316b has a convex shape protruding forward. The sacrum support portion 316b protrudes forward from the general portion 316a. As one example, the shape of the sacrum support portion 316b when viewed from the front is a shape having a major axis and a minor axis. For example, the shape of the sacrum support portion 316b when viewed from the front is an oval shape (as one example, an elliptical shape).

The cushion sensor 313 acquires vital data of the user seated on the cushion 310b. As one example, the cushion sensor 313 acquires the heartbeat information of the user during the day. The cushion sensor 313 is, for example, a piezoelectric sensor fixed to the core material 314. In this case, the cushion sensor 313 measures pressure that is applied to each portion of the cushion 310b from the body of the user placed on the cushion 310b. The type of the cushion sensor 313 is not particularly limited. The cushion sensor 313 includes, for example, a seating portion sensor 313a attached to the seating portion 315; a sacrum support portion sensor 313b attached to the sacrum support portion 316b; and a thigh support portion sensor 313c attached to each of the two thigh support portions 315b.

The seating portion sensor 313a detects that the user is seated on the seating portion 315. For example, the seating portion sensor 313a detects that the ischium of the user is in contact with the seating portion sensor 313a. The sacrum support portion sensor 313b measures, for example, the load of the body of the user that is in contact with the sacrum support portion sensor 313b. The thigh support portions 315b measure, for example, the load of the thighs of the user seated on the seating portion 315. The seating portion sensor 313a, the sacrum support portion sensor 313b, and the thigh support portion sensors 313c generate an electrical signal corresponding to the load of the body of the user based on the load, and acquires the electrical signal as the heartbeat information described above.

An example of the functional configuration of the life improvement application 340 will be described. As shown in FIG. 14, the life improvement application 340 includes, as the functional configuration, a sleep state acquisition unit 341, an autonomic nervous system acquisition unit 342, a storage unit 343, a device control unit 344, a prediction information generation unit 345, and an improvement information generation unit 346.

The sleep state acquisition unit 341 acquires sleep state information, which is information indicating the sleep state of the user, from at least one of the breathing information, the body movement information, and the heartbeat information acquired by the sensor unit 311. The sleep state refers to the state of sleep of the user. The sleep state may include, for example, the state of sleep such as sleep time and sleep stage, and biological information of the user other than the state of sleep, such as fatigue level and stress level. As one example, the sleep state information includes at least one of the bedtime, wake-up time, ratio of each sleep stage, sleep onset latency (time taken to fall asleep), sleep efficiency, number of times of nocturnal awakening, and nocturnal awakening time of the user.

The sleep stage is an index indicating the depth of sleep of the user. The sleep stages are classified into, for example, wakefulness, REM sleep, and non-REM sleep. The non-REM sleep is classified into, for example, four types of sleep stages. Incidentally, the non-REM sleep may be classified into two types or three types of sleep stages. Hereinafter, the stage of REM sleep or non-REM sleep may be referred to as "sleep". The time the sleep stage of the user changes from wakefulness to sleep between the bedtime and the wake-up time is referred to as the sleep onset time. The time the sleep stage of the user finally becomes the stage of awakening between the bedtime and the wake-up time is referred to as the awakening time.

The ratio of each sleep stage refers to the ratio of the time spent in each sleep stage with respect to the time from the bedtime to the wake-up time. The sleep onset latency (time taken to fall asleep) refers to the length of time from the bedtime to the sleep onset time. The sleep efficiency is an index for evaluating the quality of sleep. The sleep efficiency is, for example, a value obtained by dividing the time, which is obtained by subtracting the nocturnal awakening time from the time from the sleep onset time to the awakening time, by the time from the bedtime to the sleep onset time. The nocturnal awakening refers to a state where the user awakens between the sleep onset time and the awakening time. As used herein, the term "awakening" refers to a state where the sleep stage changes from the stage of REM sleep or non-REM sleep to the stage of awakening.

For example, the sleep state acquisition unit 341 determines the bedtime and the wake-up time based on the body movement information acquired by the sensor unit 311 and the movement of the bedding detected by the sensor unit 311 (for example, an acceleration sensor), and acquires the bedtime and the wake-up time. The sleep state acquisition unit 341 acquires, for example, the sleep stage using the signal indicating body movement associated with breathing, the signal indicating body movement associated with heartbeat, and the signal indicating body movement not associated with breathing and heartbeat that are output from the sensor unit 311.

The sleep state acquisition unit 341 acquires, for example, the ratio of each sleep stage from the sleep stage, the bedtime, and the wake-up time. The sleep state acquisition unit 341 acquires, for example, the time from the bedtime to the sleep onset time as the sleep onset latency based on the bedtime and the sleep stage.

The sleep state acquisition unit 341 acquires, for example, the nocturnal awakening time and the number of times of nocturnal awakening from the bedtime, the wake-up time, and the sleep stage. More specifically, for example, the sleep state acquisition unit 341 acquires the sleep onset time from the bedtime and the sleep stage, and acquires the awakening time from the wake-up time and the sleep stage. The sleep state acquisition unit 341 acquires the nocturnal awakening time and the number of times of nocturnal awakening from the sleep onset time, the awakening time, and the sleep stage.

The sleep state acquisition unit 341 acquires, for example, the sleep efficiency from the bedtime, the wake-up time, and the sleep stage. More specifically, the sleep state acquisition unit 341 acquires, for example, the sleep efficiency from the bedtime, the wake-up time, the sleep onset time, the awakening time, and the nocturnal awakening time.

The autonomic nervous system acquisition unit 342 acquires autonomic nervous system information, which is information indicating the state of the autonomic nervous system of the user, from the heartbeat information acquired by the sensor unit 311. The autonomic nervous system refers to a nervous system that controls the functioning of organs such as the heart or stomach or involuntary functions such as blood circulation. As used herein, the term "involuntary" refers to, for example, a situation in which something does not proceed according to one's own wishes or cannot be controlled by one's own intention. Incidentally, reactions of the autonomic nervous system are reactions that cannot be intentionally controlled by a person's intention, and are used as objective indicators of emotions, feelings, fatigue, stress, and the like.

The autonomic nervous system acquisition unit 342 acquires, for example, the heart rate variability (HRV) of the user by analyzing the heartbeat information acquired by the sensor unit 311. As one example, the autonomic nervous system acquisition unit 342 acquires, from the heart rate variability of the user, information indicating periodic components contained in the heart rate variability. The autonomic nervous system acquisition unit 342 performs frequency analysis on the periodic components of the heart rate variability to acquire information indicating the power spectrum of each frequency.

The autonomic nervous system acquisition unit 342 acquires, as the autonomic nervous system information, information indicating a sympathetic nervous system index and information indicating a parasympathetic nervous system index. The sympathetic nervous system index is an index indicating the dominance of the sympathetic nervous system. The parasympathetic nervous system index is an index indicating the dominance of the parasympathetic nervous system.

The autonomic nervous system acquisition unit 342 acquires the mental state of the user from the autonomic nervous system information. The autonomic nervous system acquisition unit 342 classifies, for example, the mental state of the user into one of a high-performance state, a relaxed state, a stressed state, and a depressed state, based on the autonomic nervous system information.

The high-performance state refers to, for example, a state where the sympathetic nervous system index is equal to or larger than a predetermined first threshold value and the parasympathetic nervous system index is equal to or larger than a predetermined second threshold value. The relaxed state refers to, for example, a state where the sympathetic nervous system index is less than the predetermined first threshold value and the parasympathetic nervous system index is equal to or larger than the predetermined second threshold value. The stressed state refers to, for example, a state where the sympathetic nervous system index is equal to or larger than the predetermined first threshold value and the parasympathetic nervous system index is less than the predetermined second threshold value. The depressed state refers to, for example, a state where the sympathetic nervous system index is less than the predetermined first threshold value and the parasympathetic nervous system index is less than the predetermined second threshold value.

As one example, the life improvement application 340 generates information indicating the exercise performance of the user based on past sleep information stored in the life improvement server 402, the autonomic nervous system information acquired by the autonomic nervous system acquisition unit 342, information indicating body temperature, information indicating blood pressure, and information indicating heart rate that are acquired by the sensor unit 311. The past sleep information refers to the past sleep state information of the user. The past refers to a time before the time the device control unit 344 controls the operation of a device 404. The past sleep information is, for example, accumulated data of sleep information of the user from several days ago to the previous day.

The exercise performance is an index indicating the physical condition of the user. The life improvement application 340 generates information indicating thinking ability, based on the past sleep information, the autonomic nervous system information, and the breathing information. The life improvement application 340 generates, for example, information indicating concentration and information indicating sleepiness, based on the autonomic nervous system information. The life improvement application 340 generates information indicating reaction speed, based on the autonomic nervous system information and the body movement information. The life improvement application 340 may quantify each of the exercise performance, thinking ability, concentration, and reaction speed of the user. The life improvement application 340 generates, for example, information indicating the number of times of turning over during sleep, based on the past sleep information and the body movement information.

The storage unit 343 stores the sleep state information acquired by the sleep state acquisition unit 341. As one example, the storage unit 343 stores the sleep state information in the life improvement server 402. The storage unit 343 may store, for example, the sleep state information in the memory of the information terminal 401.

The device control unit 344 controls the operation of the device 404 based on at least one of the past sleep information stored in advance in the life improvement server 402 and the autonomic nervous system information acquired by the autonomic nervous system acquisition unit 342. The device control unit 344 may determine, for example, whether the ratio of REM sleep is equal to or larger than a predetermined value, based on the past sleep information. The device control unit 344 may determine, for example, whether the sleep efficiency is equal to or larger than a predetermined value, based on the past sleep information. The device control unit 344 may determine, for example, whether the average value of the time from the bedtime to the sleep onset time (sleep onset latency) in the past sleep information is equal to or larger than a predetermined time.

The device control unit 344 may determine, for example, whether the number of times of turning over during sleep is equal to or larger than a predetermined value, based on the information indicating the number of times of turning over during sleep. The device control unit 344 may determine, for example, whether the sympathetic nervous system index is equal to or larger than a predetermined value, based on the autonomic nervous system information. The device control unit 344 may determine, for example, whether the number of times of nocturnal awakening acquired by the sleep state acquisition unit 341 is equal to or larger than a predetermined value. The device control unit 344 may determine, for example, whether the exercise performance of the user is equal to or larger than a predetermined value, based on the information indicating exercise performance. The device control unit 344 may determine, for example, whether the thinking ability of the user is equal to or larger than a predetermined value, based on the information indicating thinking ability. The device control unit 344 may determine, for example, whether the reaction speed of the user is equal to or larger than a predetermined value, based on the information indicating reaction speed. The device control unit 344 may control the operation of the device 404 based on at least one of the determination results described above.

The life improvement system 301 further includes, for example, a device server 405. The device server 405 can communicate with the device control unit 344. The device server 405 receives information for controlling the operation of the device 404 from the device control unit 344. The device server 405 controls the device 404 based on the received information. However, when the device control unit 344 can directly communicate with the device 404, the device server 405 may not be provided. The device server 405 may be able to communicate with the life improvement server 402. In this case, the device server 405 may communicate with the information terminal 401 via the life improvement server 402.

The device 404 constitutes an environment around the user. The device 404 includes, for example, at least one of an air conditioner installed in the bedroom of the user, a lighting fixture, a pillow used by the user during sleep, a heated mattress, a coffee maker, and an automobile.

For example, when the device 404 is an air conditioner, the device control unit 344 may adjust the temperature of a space where the user is present by controlling the operation of the air conditioner. For example, when the device control unit 344 determines that the ratio of REM sleep is equal to or larger than the predetermined value, the device control unit 344 may control the device 404 to increase the temperature of the space to warm the space.

For example, when the device 404 is a lighting fixture, the device control unit 344 may adjust at least one of the brightness and color temperature of a space where the user is present by controlling the operation of the lighting fixture. For example, when the device control unit 344 determines that the sleep efficiency is not equal to or larger than the predetermined value, the device control unit 344 may control the device 404 to brighten a space where the user is present in the morning such as the next morning. For example, when the mental state of the user is classified into the relaxed state or the depressed state by the autonomic nervous system acquisition unit 342, the device control unit 344 may execute the same control as described above. In this case, the space can be brightened, which contributes to improving the mental state of the user and the like.

For example, when the device 404 is a pillow, the device control unit 344 may control the angle of the placement surface of the pillow, on which the head of the user is placed, with respect to a horizontal plane by controlling the operation of the pillow. For example, when the device control unit 344 determines that the average value of the time from the bedtime to the sleep onset time in the past sleep information is equal to or larger than the predetermined time, the device control unit 344 may control the device 404 to reduce the angle of the placement surface with respect to the horizontal plane. For example, when based on the past sleep information and the breathing information, it is determined that securing the airway is necessary, the angle of the head and neck of the user with respect to the horizontal plane can be adjusted, and therefore, the inclination of the pillow can be adjusted such that the head of the user is adapted to sleeping on his or her side. Accordingly, snoring of the user can be suppressed.

For example, when the device 404 is a heated mattress, the device control unit 344 may adjust temperature in the bed of the user by controlling the operation of the heated mattress. For example, when the device control unit 344 determines that the number of times of turning over during sleep is not equal to or larger than the predetermined value and the sleep efficiency is not equal to or larger than the predetermined value, the device control unit 344 may control the device 404 to increase temperature in the bed. For example, when the device control unit 344 determines that the number of times of nocturnal awakening is equal to or larger than the predetermined value, the device control unit 344 may control the device 404 to increase temperature in the bed in real time.

For example, when the device 404 is a coffee maker, the device control unit 344 may adjust the strength of the coffee that the user drinks after awakening by controlling the operation of the coffee maker. For example, when the device control unit 344 determines that the sympathetic nervous system index is not equal to or larger than the predetermined value, the device control unit 344 may control the device 404 to increase the strength of the coffee.

For example, when the device 404 is an automobile, the automobile may execute vehicle control in a plurality of driving modes. The plurality of driving modes include, for example, a steering assist mode in which steering by a driver is assisted (autonomous driving mode) and a normal mode in which steering assistance is not performed. For example, the device control unit 344 may switch the driving mode of the automobile to the steering assist mode or the normal mode or recommend switching to the steering assist mode or the normal mode to the user by controlling the automobile. For example, when the device control unit 344 determines that the exercise performance is not equal to or larger than the predetermined value, the device control unit 344 may switch the operation of the automobile to the steering assist mode, or recommend switching to the steering assist mode to the user. For example, when the device control unit 344 determines that the thinking ability of the user is not equal to or larger than the predetermined value, the device control unit 344 may execute the same control as described above. For example, when the device control unit 344 determines that the reaction speed of the user is not equal to or larger than the predetermined value, the device control unit 344 may execute the same control as described above. For example, the device control unit 344 may switch the operation of the automobile to the normal mode, or recommend switching to the normal mode to the user.

The prediction information generation unit 345 generates the prediction information that is information indicating the predicted state of the user during wakefulness, based on at least one of the past sleep information acquired by the sleep state acquisition unit 341 and the autonomic nervous system information acquired by the autonomic nervous system acquisition unit 342. As one example, the prediction information includes mental information that is information indicating the mental state of the user, physical condition information that is information indicating the physical condition of the user, and brain information that is information indicating the state of the brain of the user.

The prediction information generation unit 345 may determine, for example, whether the sleep efficiency is equal to or larger than the predetermined value, based on the past sleep information. The prediction information generation unit 345 may determine, for example, whether the concentration of the user is equal to or larger than the predetermined value, based on the information indicating concentration. The prediction information generation unit 345 may generate the prediction information based on the determination result of the sleep efficiency and the determination result of the concentration described above.

The prediction information generation unit 345 generates, for example, the mental information based on the autonomic nervous system information. The mental information includes, for example, information about the mood swings of the user. As one example, the mental information includes information indicating whether the mood of the user has a tendency toward becoming depressed. For example, when the mental state of the user is classified into the stressed state or the depressed state by the autonomic nervous system acquisition unit 342, the prediction information generation unit 345 may generate, as the mental information, information indicating that the mood of the user has a tendency toward becoming depressed.

The prediction information generation unit 345 generates, for example, the physical condition information based on the past sleep information. The physical condition information includes, for example, information indicating whether the physical condition of the user is good during wakefulness. For example, when it is determined that the sleep efficiency is not equal to or larger than the predetermined value, the prediction information generation unit 345 may generate, as the physical condition information, information indicating that the physical condition of the user tends to deteriorate. As one example, the physical condition information includes skin information indicating the state of the skin of the user. The skin information includes, for example, information indicating that the skin quality of the user is predicted to be poor. The term "skin quality being poor" refers to, for example, a state where the moisture amount of the skin decreases below a certain value.

The prediction information generation unit 345 generates, for example, the brain information based on the past sleep information and the autonomic nervous system information. The brain information is information indicating whether the brain of the user functions well. The brain information includes, for example, information indicating whether the concentration, memory, and thinking ability of the user are good. For example, when the mental state of the user is classified into the high-performance state by the autonomic nervous system acquisition unit 342, the prediction information generation unit 345 may generate, as the brain information, information indicating that the concentration of the user is predicted to be high. For example, when it is determined that the sleep efficiency is not equal to or larger than the predetermined value, the prediction information generation unit 345 may generate, as the brain information, information indicating that the thinking ability of the user tends to be low. For example, when the mental state of the user is classified into the stressed state, the relaxed state, or the depressed state by the autonomic nervous system acquisition unit 342, or even when it is determined that the concentration of the user is not equal to or larger than the predetermined value, the prediction information generation unit 345 may execute the same control as described above.

The improvement information generation unit 346 generates the improvement information that is information for improving the life of the user, based on the prediction information generated by the prediction information generation unit 345. As one example, the improvement information includes at least one of information indicating the type of clothing recommended to the user, information indicating the type of soap, cosmetic, and food, and information indicating a recommended behavior. The information indicating the type of clothing includes, for example, at least one of information about the color of the clothing, information about the type of fabric of the clothing, and the number of pieces of clothing worn.

As one example, the improvement information generation unit 346 generates, as the improvement information, the information indicating the type of cosmetic, based on the skin information generated by the prediction information generation unit 345. For example, when the prediction information generation unit 345 generates the information indicating that the skin quality is predicted to be poor, the improvement information generation unit 346 generates information indicating the type of cosmetic that enhance the moisturizing power of the skin.

For example, the improvement information generation unit 346 generates, as the improvement information, information indicating the type (for example, color) of apparel recommended to the user, based on the information indicating that the mood of the user is predicted to have a tendency toward becoming depressed. As one example, when the mental state of the user is classified into the depressed state by the autonomic nervous system acquisition unit 342, the improvement information generation unit 346 may generate improvement information indicating that warm-colored (for example, red) apparel is recommended. For example, the improvement information generation unit 346 generates, as the improvement information, at least one of information indicating the type of soap, cosmetic, and food recommended to the user and information indicating that the user has to avoid sunlight during the day, based on the information indicating that the skin quality of the user is predicted to be poor.

For example, the improvement information generation unit 346 generates, as the improvement information, information indicating that it is recommended not to make an important decision, based on the information indicating that the thinking ability of the user is predicted to be low. For example, the improvement information generation unit 346 generates, as the improvement information, information indicating whether the steering assist mode or the normal mode is recommended as the driving mode of an automobile, based on the information indicating exercise performance, the information indicating thinking ability, and the information indicating reaction speed.

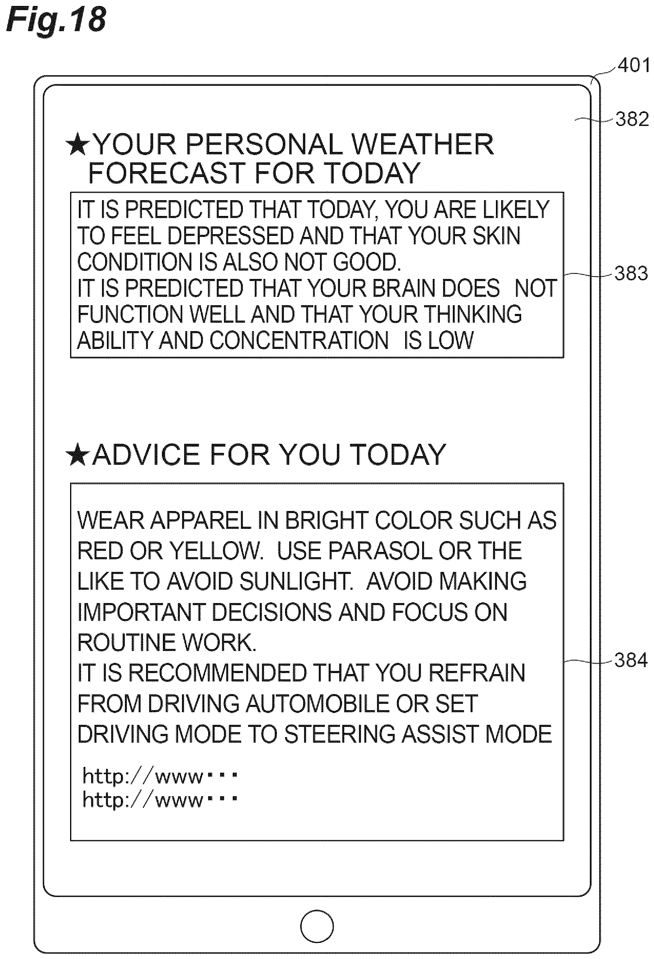

As one example, the life improvement application 340 displays the prediction information generated by the prediction information generation unit 345 and the improvement information generated by the improvement information generation unit 346 on the information terminal 401. FIG. 18 is a view showing an example of an output screen 382 of the prediction information and the improvement information that are displayed on the information terminal 401. The life improvement application 340 displays, for example, the prediction information and the improvement information on the information terminal 401 as the output screen 382. The output screen 382 is, for example, a screen for providing the prediction information and the improvement information to the user. The output screen 382 includes a prediction information display portion 383 that is a portion for displaying the prediction information, and an improvement information display portion 384 that is a portion for displaying the improvement information.

The prediction information display portion 383 displays, for example, at least one of the information indicating that the mood of the user is predicted to have a tendency toward becoming depressed, the information indicating that the skin quality is predicted to be poor, and the information indicating that the concentration and the thinking ability are predicted to be low. The prediction information display portion 383 may display, for example, information indicating that the mood of the user is predicted to have a tendency toward becoming excited, information indicating that the skin quality is predicted to be good, and information indicating that the concentration and the thinking ability are predicted to be high.

The improvement information display portion 384 displays, for example, the information indicating the color of apparel recommended to the user, the information indicating that the user has to avoid sunlight during the day, the information indicating that it is recommended not to make an important decision, and information indicating that the steering assist mode is recommended as the driving mode of an automobile. The improvement information display portion 384 displays, for example, the information indicating soap, cosmetic, and food recommended to the user. The improvement information display portion 384 displays, for example, the URL of the website 403 on which information regarding products recommended to the user is posted.

As one example, the improvement information generation unit 346 generates the improvement information based on the prediction information and the subjective information described above. More specifically, the improvement information generation unit 346 acquires objective information from the past sleep information and the autonomic nervous system information. The objective information refers to objective information of the user obtained from objective data obtained by measurement, such as the sleep state information or the autonomic nervous system information. The objective information includes, for example, an objective evaluation of sleep. The improvement information generation unit 346 acquires the subjective evaluation of sleep included in the subjective information. The improvement information generation unit 346 generates feedback information as the improvement information, based on the subjective evaluation of sleep and the objective evaluation of sleep. The feedback information includes, for example, information indicating a difference between the subjective evaluation of sleep and the objective evaluation of sleep.

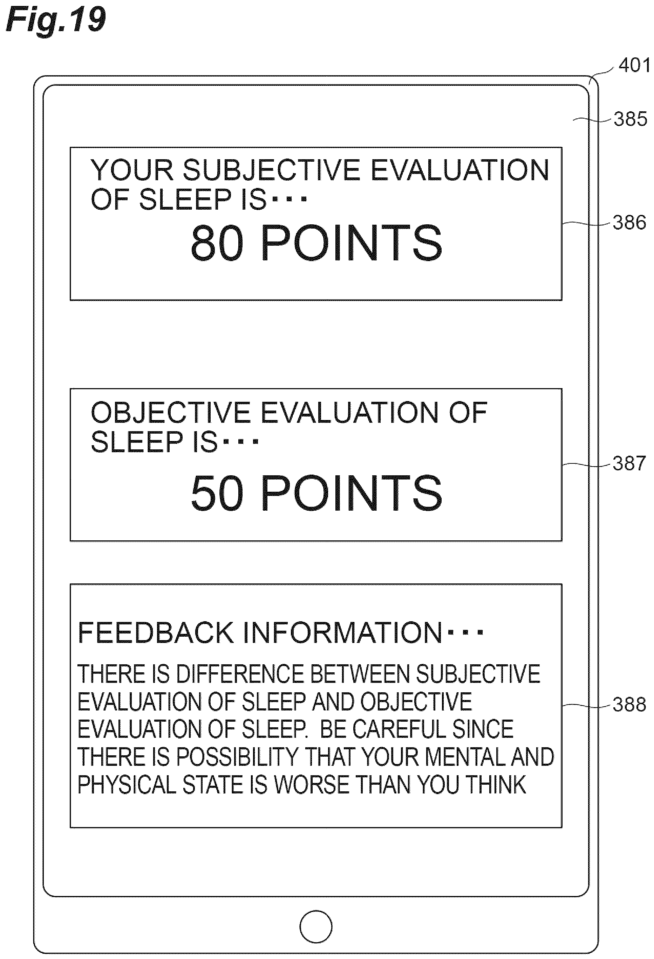

As one example, the life improvement application 340 displays the feedback information generated by the improvement information generation unit 346 on the information terminal 401. FIG. 19 is a view showing an example of an output screen 385 of the feedback information that is displayed on the information terminal 401. The life improvement application 340 displays, for example, the feedback information on the information terminal 401 as the output screen 385. The output screen 385 is, for example, a screen for providing the feedback information to the user. The output screen 385 includes a subjective information display portion 386 that displays the subjective evaluation of sleep; an objective information display portion 387 that displays the objective evaluation of sleep; and a feedback display portion 388 that displays the feedback information.

The subjective information display portion 386 displays the subjective information. The subjective information display portion 386 displays, for example, the subjective evaluation of sleep input by the user into the sleep evaluation input portion 381 (see FIG. 15). The objective information display portion 387 displays the objective information. The objective information display portion 387 displays, for example, the objective evaluation of sleep of the user based on the sleep state information acquired by the sleep state acquisition unit 341 and the autonomic nervous system information acquired by the autonomic nervous system acquisition unit 342. The objective information display portion 387 displays, for example, the objective evaluation of sleep, with 100 points being the full score. The feedback display portion 388 displays, for example, the information indicating the difference between the subjective evaluation of sleep and the objective evaluation of sleep.

The life improvement application 340 performs total feedback. The total feedback includes improving the accuracy of control of the device 404. The total feedback includes improving the accuracy of generation of the prediction information and the improvement information. For example, when the subjective evaluation of sleep is higher than the objective evaluation of sleep by a predetermined value or more, the device control unit 344 may prioritize subjective control of the device 404 over objective control of the device 404. As a result, control of the device 404 that is more in line with the subjective perception of the user.

Communication between the sensor unit 311 and the life improvement application 340, communication between the life improvement application 340 and the life improvement server 402, communication between the life improvement server 402 and the information terminal 401, communication between the life improvement server 402 and the device server 405, communication between the device control unit 344 and the device server 405, and communication between the device server 405 and the device 404 may be realized, for example, by a wireless communication interface such as a wireless Local Area Network (LAN) or Bluetooth (registered trademark). These communications may be realized by wire.

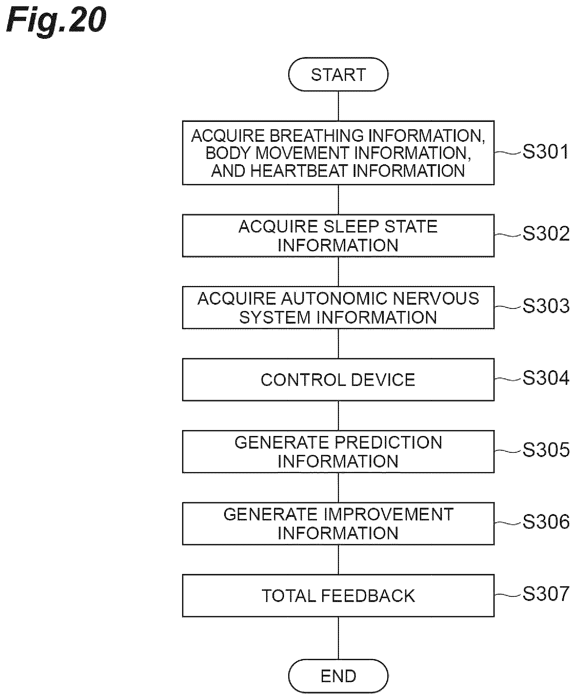

One example of the operation of the life improvement system 301 will be described. FIG. 20 is a flowchart showing one example of the operation of the life improvement system 301. Before the life improvement system 301 is operated, first, the sensor sheet 312 is attached to the mattress 310a, and the cushion sensor 313 is attached to the cushion 310b. Next, the body of the user is placed on the mattress 310a or the cushion 310b. For example, during sleep, the body of the user is placed on the mattress 310a so as to come into contact with the front fabric 331 of the cover fabric 303. For example, during the day, the body of the user is placed on the seating portion 315 of the cushion 310b.

The sensor unit 311 acquires the breathing information, the body movement information, and the heartbeat information from body movement associated with the breathing of the user, body movement associated with heartbeat, and body movement not associated with breathing and heartbeat (step S301). When vital data of the user is acquired by the sensor sheet 312, in step S301, the sensor sheet 312 detects the body movement associated with the breathing of the user, the body movement associated with heartbeat, and the body movement not associated with breathing and heartbeat. The sensor sheet 312 outputs a signal indicating the body movement associated with breathing, a signal indicating the body movement associated with heartbeat, and a signal indicating the body movement not associated with breathing and heartbeat to the outside of the sensor sheet 312. When vital data of the user is acquired by the cushion sensor 313, in step S301, the cushion sensor 313 detects the body movement associated with the heartbeat of the user. The cushion sensor 313 outputs a signal indicating the body movement associated with heartbeat to the outside of the cushion sensor 313. The sensor unit 311 outputs a signal indicating the body movement associated with breathing, a signal indicating the body movement associated with heartbeat, and a signal indicating the body movement not associated with breathing and heartbeat to the outside of the sensor unit 311.

The sleep state acquisition unit 341 acquires, for example, the sleep state information from the signals output from the sensor unit 311. The sleep state acquisition unit 341 acquires the sleep state information of the user from at least one of the breathing information, the body movement information, and the heartbeat information acquired by the sensor unit 311 (step S302). In step S302, the sleep state acquisition unit 341 acquires, for example, the bedtime, wake-up time, ratio of each sleep stage, sleep onset latency, sleep efficiency, number of times of nocturnal awakening, and nocturnal awakening time of the user from the breathing information, the body movement information, and the heartbeat information.

The autonomic nervous system acquisition unit 342 acquires the autonomic nervous system information from the heartbeat information acquired by the sensor unit 311 (step S303). In step S303, for example, the autonomic nervous system acquisition unit 342 acquires the heart rate variability of the user by analyzing the heartbeat information acquired by the sensor unit 311. In step S303, the autonomic nervous system acquisition unit 342 acquires the autonomic nervous system information from the heart rate variability of the user. In step S303, the autonomic nervous system acquisition unit 342 acquires the mental state of the user from the autonomic nervous system information.

The device control unit 344 controls the operation of the device 404 based on at least one of the past sleep information and the autonomic nervous system information (step S304). In step S304, for example, the device control unit 344 controls the operation of a coffee maker. The device control unit 344 may control, for example, the operation of the coffee maker such that the strength of the coffee that the user drinks after awakening is increased, based on the past sleep information and the mental state of the user.

The prediction information generation unit 345 generates the prediction information based on at least one of the past sleep information stored in advance in the life improvement server 402 and the autonomic nervous system information acquired by the autonomic nervous system acquisition unit 342 (step S305). In step S305, the prediction information generation unit 345 generates, for example, at least one of the mental information, the physical condition information, and the brain information as the prediction information, based on the past sleep information and the autonomic nervous system information. The prediction information generation unit 345 generates, for example, the skin information as the physical condition information. For example, the prediction information generation unit 345 generates, as the prediction information, the information indicating that the mood of the user is predicted to have a tendency toward becoming depressed. For example, the prediction information generation unit 345 generates, as the prediction information, the information indicating that the physical condition of the user is good during wakefulness. For example, the prediction information generation unit 345 generates, as the prediction information, the information indicating that the concentration and thinking ability of the user are predicted to be high.

The improvement information generation unit 346 generates the improvement information based on the prediction information generated by the prediction information generation unit 345 (step S306). For example, the improvement information generation unit 346 generates, as the improvement information, the information indicating the type of clothing recommended to the user. For example, the improvement information generation unit 346 generates, as the improvement information, the information indicating the type of soap, cosmetic, and food. For example, the improvement information generation unit 346 generates, as the improvement information, the information indicating a recommended behavior.

In step S306, the improvement information generation unit 346 generates the improvement information based on the prediction information and the subjective information. The improvement information generation unit 346 acquires, for example, the objective evaluation of sleep included in the objective information obtained from the sleep state information and the autonomic nervous system information. The improvement information generation unit 346 acquires, for example, the subjective evaluation of sleep included in the subjective information input by the user. The improvement information generation unit 346 generates, for example, the feedback information based on the subjective evaluation of sleep and the objective evaluation of sleep. The improvement information generation unit 346 generates, for example, the information indicating the difference between the subjective evaluation of sleep and the objective evaluation of sleep.

The life improvement application 340 displays the prediction information generated by the prediction information generation unit 345 and the improvement information generated by the improvement information generation unit 346. The life improvement application 340 displays, for example, the prediction information and the improvement information on the information terminal 401 as the output screen 382 (see FIG. 18). The life improvement application 340 displays the feedback information generated by the improvement information generation unit 346. The life improvement application 340 displays, for example, the feedback information on the information terminal 401 as the output screen 385 (see FIG. 19).

The life improvement application 340 performs total feedback (step S307). In step S307, for example, when the subjective evaluation of sleep is higher than the objective evaluation of sleep by the predetermined value or more, the device control unit 344 of the life improvement application 340 prioritizes subjective control of the device 404 over objective control of the device 404.

One example of the steps of the operation of the life improvement system 301 has been described above. However, the contents and order of the steps of the operation of the life improvement system 301 are not limited to the above-described example, and can be changed as appropriate.

Subsequently, actions and effects of the life improvement system 301 will be described. As one example, the life improvement system 301 generates the prediction information of the user from at least one of the past sleep information and the autonomic nervous system information. For example, the life improvement system 301 can provide, as the prediction information, information indicating the mental and physical state of the user to the user. The life improvement system 301 generates the improvement information from the prediction information. For example, when the mental and physical state of the user is predicted to have a tendency toward becoming poor, the life improvement system 301 can provide, to the user, the information indicating that it is recommended not to make an important decision as the improvement information. The life improvement system 301 not only controls the device 404 while the user sleeps, but can also predict the state of the user during wakefulness based on the sleep of the user and provide information for improving the life of the user based on the sleep state.

In the life improvement system 301, the prediction information and the improvement information can be generated from the past sleep state of the user. The prediction information indicating the predicted state of the user during wakefulness and the improvement information for improving the life can be generated by taking into account not only the sleep state of that day but also the past sleep state of the user. Compared to when the prediction information and the improvement information are generated solely based on the sleep state from the time the user goes to bed until the user wakes up, the prediction information and the improvement information can be generated based on longer-term records. As a result, the prediction information and the improvement information can be more accurately generated.

As one example, the sensor unit 311 includes the cushion sensor 313 attached to the cushion 310b on which the user is seated. Accordingly, for example, vital data of the user can be continuously acquired not only during sleep but also during the day. Since the prediction information and the improvement information can be generated by taking into account the vital data of the user that is continuously measured during the day, the prediction information and the improvement information can be more accurately generated using both states during sleep and during wakefulness.

As one example, the prediction information includes the mental information that is information indicating the mental state of the user, the physical condition information that is information indicating the physical condition of the user, and the brain information that is information indicating the state of the brain of the user. Accordingly, the mental, physical condition, and state of the brain of the user during wakefulness can be predicted. The physical condition information includes the skin information indicating the state of the skin of the user. Accordingly, the information indicating the state of the skin of the user can be provided as the prediction information.

As one example, the improvement information generation unit 346 generates the improvement information based on the subjective information, which is information indicating the subjective evaluation of the user, and the prediction information. Since the improvement information can be generated by taking into account the subjective evaluation of the user, the improvement information can be more accurately generated.

As one example, the improvement information includes information indicating the type of clothing, food, and cosmetic recommended to the user. Accordingly, the information indicating the type of clothing, food, and cosmetic recommended to the user can be provided as the improvement information.

As one example, the device 404 may include a pillow used by the user during sleep. The device control unit 344 may control the angle of the placement surface of the pillow, on which the head of the user is placed, with respect to a horizontal plane. In this case, since the angle of the placement surface of the pillow can be adjusted while the user sleep, the sleep state of the user can be further improved.

As one example, the life improvement system 301 includes the sensor unit 311 that acquires the heartbeat information that is information indicating the heartbeat of the user; the autonomic nervous system acquisition unit 342 that acquires the autonomic nervous system information that is information indicating the state of the autonomic nervous system of the user, based on the heartbeat information; the prediction information generation unit 345 that generates the prediction information that is information indicating the predicted state of the user during wakefulness, based on the autonomic nervous system information; and the improvement information generation unit 346 that generates the improvement information that is information for improving the life of the user, based on the prediction information.

For example, the life improvement system 301 generates the prediction information of the user from the autonomic nervous system information, and generates the improvement information from the prediction information. Therefore, the state of the user during wakefulness can be predicted, and the information for improving the life of the user can be provided.

Next, specific examples of the sleeping posture determination system and the sleeping posture determination program will be described. As one example, the sleeping posture determination system and the sleeping posture determination program determine the sleeping posture of a user, and provides advice for improving the sleeping posture to the user. The sleeping posture determination system and the sleeping posture determination program may be used for personal or household purposes, or may be used for testing and research purposes. The sleeping posture determination system and the sleeping posture determination program may be used in facilities such as hospitals or welfare facilities.

The "user" refers to a person who uses the sleeping posture determination system or the sleeping posture determination program. The user may be a user of bedding. The user may be, for example, a person who wishes to have his or her sleeping posture determined, a person who has trouble sleeping, or a person who is unable to sleep well and suffers from physical and mental disorders. The number of the "users" may be one or may be plural. The sleeping posture determination system and the sleeping posture determination program may be used for a plurality of users.

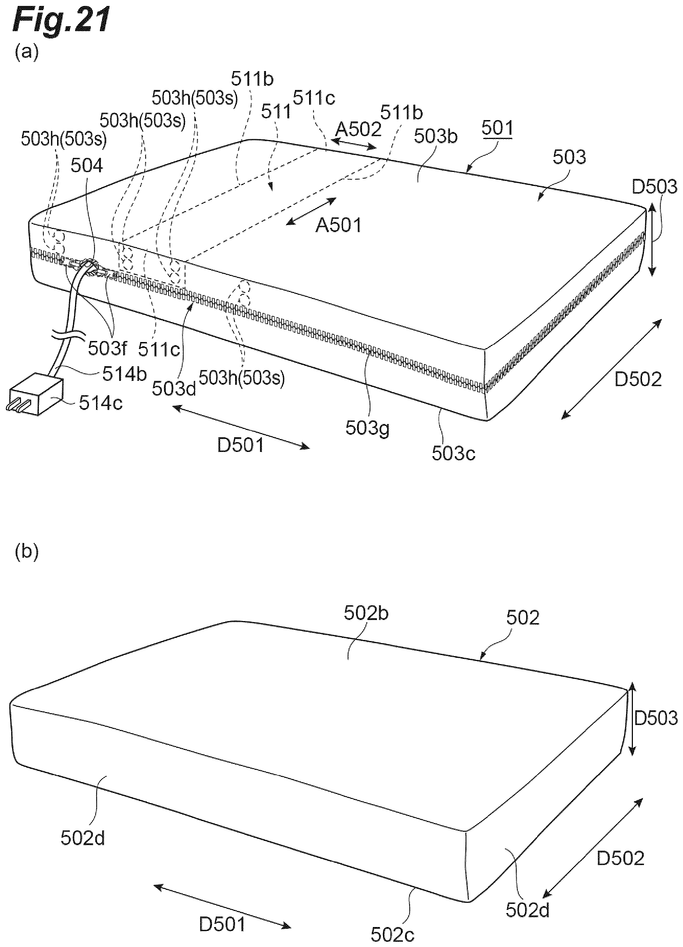

As one example, the sleeping posture determination system includes a sensor unit mounted on the bedding. First, an example of the sensor unit will be described with reference to (a) and (b) in FIG. 21. (a) in FIG. 21 is a perspective view showing bedding 501 on which a sensor unit 511 as one example is mounted. (b) in FIG. 21 is a perspective view showing a core material 502 of the bedding 501.

As shown in (a) and (b) of FIG. 21, the bedding 501 is a cushion body having a rectangular shape in a plan view. The bedding 501 extends in a longitudinal direction D501 and a lateral direction D502 orthogonal to the longitudinal direction D501. The bedding 501 has a thickness in a thickness direction D503 orthogonal to both the longitudinal direction D501 and the lateral direction D502.

For example, the bedding 501 is a mattress. The bedding 501 includes the core material 502 and a cover fabric 503 that houses the core material 502. The core material 502 includes, for example, a content and a bag that houses the content. The core material 502 has an upper surface 502b on which the body of the user is placed; a lower surface 502c facing opposite the upper surface 502b; and a plurality of side surfaces 502d connecting the upper surface 502b and the lower surface 502c to each other.

The cover fabric 503 includes, for example, an upper fabric 503b covering the upper surface 502b of the core material 502; a lower fabric 503c covering the lower surface 502c of the core material 502; and an opening and closing member 503d connecting the upper fabric 503b and the lower fabric 503c to each other. The opening and closing member 503d is disposed at a position facing the side surfaces 502d of the core material 502. When viewed in the thickness direction D503, the opening and closing member 503d is located outside the side surfaces 502d of the core material 502.

The cover fabric 503 is attachable to and detachable from the core material 502. The term "being attachable to and detachable from the core material" includes a case in which the cover fabric is turned over with respect to the core material, and a case in which the cover fabric is turned over to expose at least a part of the core material. The opening and closing member 503d is, as one example, a double slider. For example, the opening and closing member 503d includes two sliders 503f and an element 503g that serves as the movement path of the sliders 503f and that attaches and detaches the upper fabric 503b to and from the lower fabric 503c upon the movement of the sliders 503f.

In a state where the upper fabric 503b is attached to the lower fabric 503c, an opening 504 through which a cable 514b to be described later passes is formed between the two sliders 503f. The opening 504 is widened by moving one of the two sliders 503f along the element 503g in this state, and the core material 502 can be removed from the widened opening 504. By moving one of the two sliders 503f along the element 503g such that the opening 504 is closed in a state where the core material 502 is housed in the lower fabric 503c, the upper fabric 503b can be attached to the lower fabric 503c.

The cover fabric 503 includes a fixed portion 503h to which the sensor unit 511 is fixed. The fixed portion 503h is provided a back surface of the cover fabric 503. The cover fabric 503 includes a plurality of the fixed portions 503h. The fixed portions 503h are provided, for example, at positions on the upper fabric 503b which face the side surfaces 502d of the core material 502. As one example, the plurality of fixed portions 503h are arranged along the longitudinal direction D501. In the example of (a) of FIG. 21, two fixed portions 503h are arranged along the thickness direction D503. For example, a plurality of (as one example, four) columns, each being composed of two fixed portions 503h aligned along the thickness direction D503, are aligned along the longitudinal direction D501. For example, each of the fixed portions 503h is composed of a snap button 503s.

For example, the sensor unit 511 is a sensor sheet disposed between the core material 502 and the cover fabric 503. The sensor unit 511 acquires vital data of the user of the bedding 501. The vital data includes, for example, information regarding the heartbeat, breathing, and body movement of the user. The contents of the vital data can be changed as appropriate. The vital data may include, for example, information regarding blood pressure.

The sensor unit 511 is disposed, for example, at a position corresponding to the heart of the user in the longitudinal direction D501. As one example, the sensor unit 511 is disposed at any position between the position where the head of the user is placed and a position spaced apart in the longitudinal direction D501 of the bedding 501 (a position on the lower right side in (a) of FIG. 21).

For example, the sensor unit 511 has long sides 511b extending along a first direction A501 that is a longitudinal direction of the sensor unit 511, and short sides 511c extending along a second direction A502 that is a lateral direction of the sensor unit 511. For example, the sensor unit 511 is disposed such that the long sides 511b extend along the lateral direction D502 of the bedding 501 and the short sides 511c extend along the longitudinal direction D501 of the bedding 501.

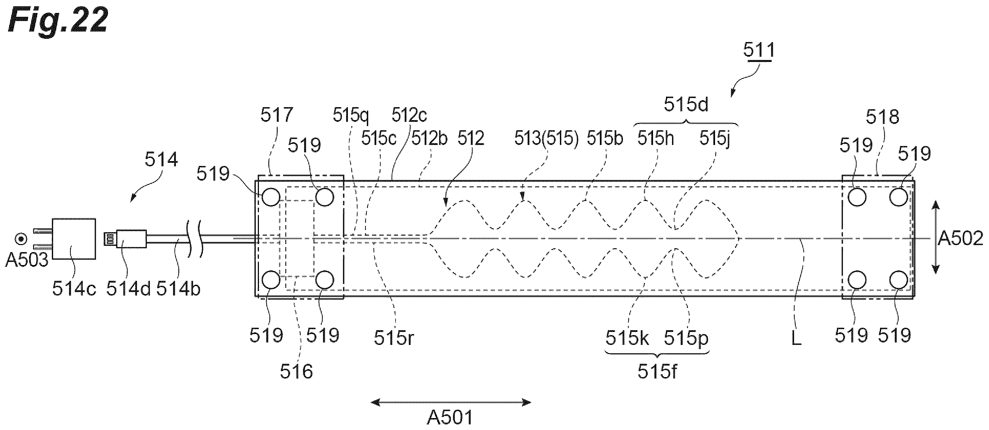

FIG. 22 is a plan view showing the sensor unit 511. As shown in (a) of FIG. 21 and FIG. 22, the sensor unit 511 includes, for example, a sheet-shaped fabric 512, a sensor 513, and a power supply unit 514. In a state where the sensor unit 511 is disposed in the bedding 501, a central portion of the sheet-shaped fabric 512 is placed on the upper surface 502b of the core material 502, and both end portions of the sheet-shaped fabric 512 in the first direction A501 face the side surfaces 502d of the core material 502. For example, the sheet-shaped fabric 512 has stretchability.

For example, the sheet-shaped fabric 512 has a triple-layer structure in which three pieces of fabric are layered along a third direction A503 intersecting both the first direction A501 and the second direction A502. The sheet-shaped fabric 512 includes an inner fabric 512b and an outer fabric 512c that houses the inner fabric 512b. The inner fabric 512b is sewn to the outer fabric 512c inside the outer fabric 512c. As one example, a peripheral edge portion of the inner fabric 512b is sewn to the outer fabric 512c.

For example, the outer fabric 512c is made of a waterproof material. As one example, the outer fabric 512c may be a fabric, the back surface of which (an inner surface of the outer fabric 512c) is laminated or resin-treated. The outer fabric 512c may be a membrane fabric. The outer fabric 512c may be a water-repellent fabric.

The sensor 513 includes, for example, a thread-shaped sensor 515 that is embroidered on the sheet-shaped fabric 512 and that detects vital data from the body of the user placed on the sensor unit 511, and a data acquisition unit 516 that acquires the vital data detected by the thread-shaped sensor 515. For example, the thread-shaped sensor 515 is embroidered on the inner fabric 512b. The thread-shaped sensor 515 is fixed, for example, by being embroidered such that the thread-shaped sensor 515 spreads out two-dimensionally on the sheet-shaped fabric 512. The data acquisition unit 516 is fixed to an end portion of the inner fabric 512b in the first direction A501. The data acquisition unit 516 outputs the acquired vital data to the outside of the sensor unit 511. The function of the data acquisition unit 516 will be described in detail later.

The thread-shaped sensor 515 acquires vital data of the user of the bedding 501. The type of the thread-shaped sensor 515 is not particularly limited. For example, the thread-shaped sensor 515 is a single sensor, and one thread-shaped sensor 515 is fixed by being embroidered such that the one thread-shaped sensor 515 spreads out two-dimensionally on the sheet-shaped fabric 512. In the example of FIG. 22, the thread-shaped sensor 515 has a shape symmetrical with respect to a center line L passing through the center of the sheet-shaped fabric 512 in the second direction A502 and extending along the first direction A501.

For example, the thread-shaped sensor 515 includes a curved portion 515b having a curved shape when viewed along the third direction A503, and a linear portion 515c having a linear shape when viewed along the third direction A503. The curved portion 515b includes a first wave-shaped portion 515d located on one side in the lateral direction D502 when viewed from the center line L, and a second wave-shaped portion 515f located on the other side in the lateral direction D502 when viewed from the center line L.

The first wave-shaped portion 515d includes first peak portions 515h and first valley portions 515j that are alternately aligned along the first direction A501, and the second wave-shaped portion 515f includes second peak portions 515k and second valley portions 515p that are alternately aligned along the first direction A501. The first wave-shaped portion 515d and the second wave-shaped portion 515f are connected to each other at the end portion of the sheet-shaped fabric 512 on the side opposite the data acquisition unit 516.

The linear portion 515c includes a first linear portion 515q and a second linear portion 515r disposed to be aligned along the second direction A502. One end of the first linear portion 515q in the first direction A501 is connected to an end portion of the first wave-shaped portion 515d in the first direction A501. One end of the second linear portion 515r in the first direction A501 is connected to an end portion of the second wave-shaped portion 515f in the first direction A501.

Each of an end portion of the first linear portion 515q on the side opposite the first wave-shaped portion 515d and an end portion of the second linear portion 515r on the side opposite the second wave-shaped portion 515f is connected to the data acquisition unit 516. By connecting the first linear portion 515q, the first wave-shaped portion 515d, the second wave-shaped portion 515f, and the second linear portion 515r, the thread-shaped sensor 515 is formed as a single sensor.

The sensor 513 is activated when the power supply unit 514 supplies electric power from the data acquisition unit 516. The power supply unit 514 includes, for example, the cable 514b and a plug 514c. One end of the cable 514b is connected to the data acquisition unit 516, and the other end of the cable 514b is connected to a connector 514d. The cable 514b is connected to the plug 514c via the connector 514d. For example, the plug 514c is inserted into an outlet. In the power supply unit 514, electric power is supplied to the data acquisition unit 516 from the plug 514c via the connector 514d and the cable 514b.

The sensor unit 511 includes fixing portions 517 and 518. The fixing portions 517 and 518 are portions for fixing the sheet-shaped fabric 512 to the cover fabric 503. The fixing portions 517 and 518 are attachable to and detachable from the cover fabric 503. The fixing portion 517 is provided on one end side of the sheet-shaped fabric 512 in the first direction A501, and the fixing portion 518 is provided on the other end side of the sheet-shaped fabric 512 in the first direction A501.

Each of the fixing portions 517 and 518 includes a snap button 519. For example, the snap button 519 locks to the snap button 503s of the cover fabric 503. Each of the fixing portions 517 and 518 includes a plurality of (as one example, four) the snap buttons 519. In the fixing portion 517, two snap buttons 519 aligned along the first direction A501 are provided on the one end side of the sheet-shaped fabric 512 in the second direction A502. In the fixing portion 517, two snap buttons 519 aligned along the first direction A501 are provided on the other end side of the sheet-shaped fabric 512 in the second direction A502. In the fixing portion 517, for example, a plurality of the snap buttons 519 are disposed to form a polygonal shape (as one example, a quadrilateral shape). Since the disposition of the snap buttons 519 in the fixing portion 518 are similar to the disposition of the snap buttons 519 in the fixing portion 517, the description will be omitted.

A portion of the sheet-shaped fabric 512 in which the fixing portions 517 and 518 are not provided is placed on the upper surface 502b of the core material 502. Each of the fixing portions 517 and 518 is fixed to the fixed portions 503h of the cover fabric 503 at a position facing the side surface 502d of the core material 502. At this time, each of the snap buttons 519 is locked to the corresponding snap button 503s constituting the fixed portion 503h.

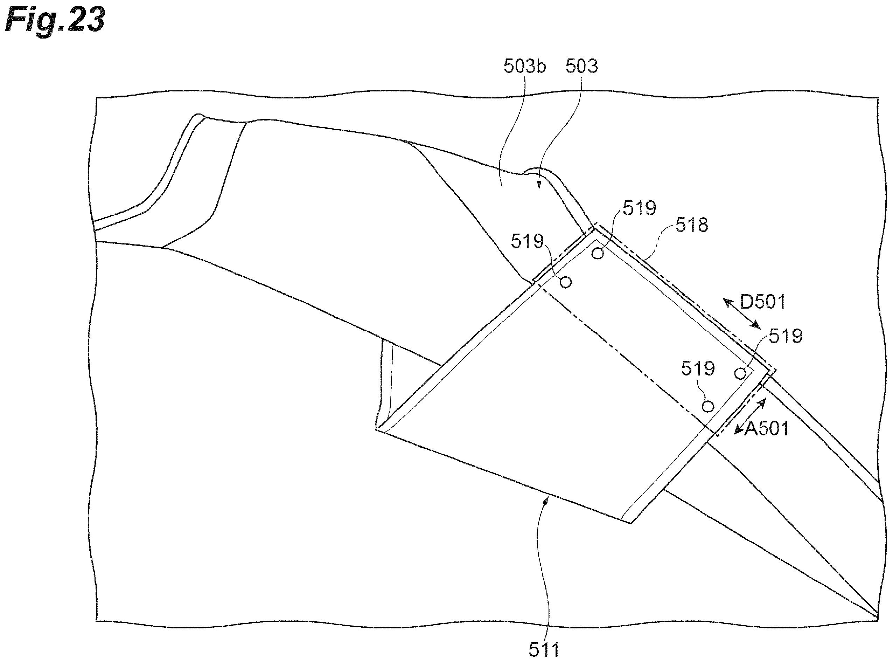

FIG. 23 is a perspective view showing a fixing portion of the sensor unit 511 to the cover fabric 503. FIG. 23 illustrates a state where the upper fabric 503b of the cover fabric 503 is turned over in a state where the sensor unit 511 is fixed to the cover fabric 503. The fixing portions 517 and 518 fix the sheet-shaped fabric 512 to the cover fabric 503 at positions between the side surfaces 502d of the core material 502 and the cover fabric 503.

Each of two snap buttons 519 aligned along the first direction A501 in the fixing portion 518 is fixed to the fixed portion 503h of the cover fabric 503 (see FIG. 21). As described above, the fixed portion 503h includes two snap buttons 503s arranged along the thickness direction D503 on the cover fabric 503. Two snap buttons 519 arranged in the first direction A501 in the fixing portion 518 are locked to the two respective snap buttons 503s arranged in the thickness direction D503 on the cover fabric 503. Similarly to the fixing portion 518, the snap buttons 519 in the fixing portion 517 are locked to the snap buttons 503s of the fixed portions 503h in the cover fabric 503.

By fixing the sensor unit 511 to the cover fabric 503 using the plurality of snap buttons 519, misalignment of the sensor unit 511 when the body of the user is placed on the bedding 501 can be suppressed. Since the fixing portions 517 and 518 fix the sensor unit 511 to the cover fabric 503 via the snap buttons 519, the sensor unit 511 can be easily removed from the cover fabric 503.

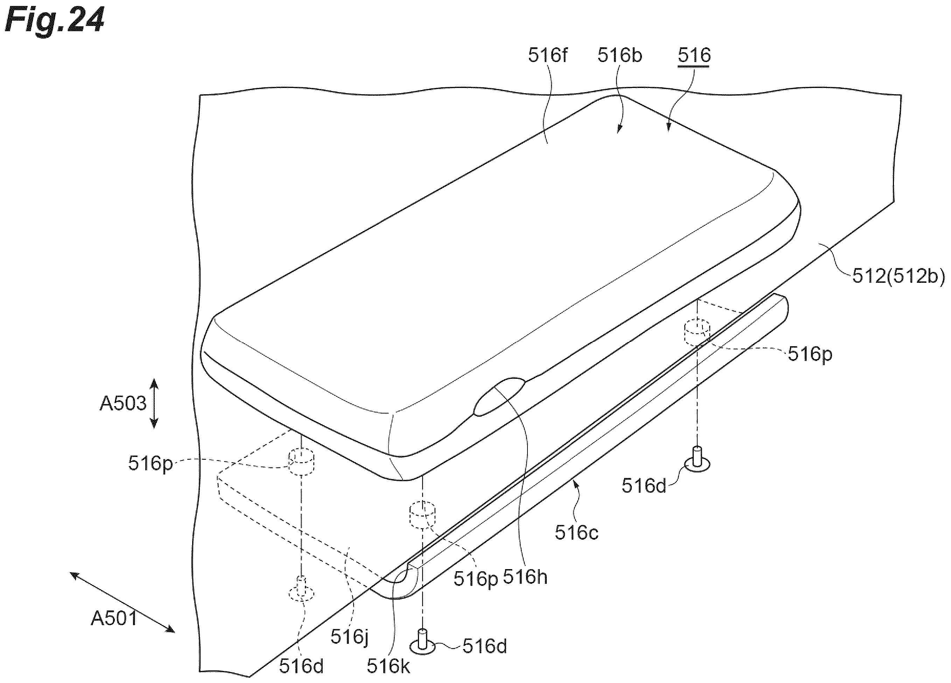

FIG. 24 is a perspective view showing the data acquisition unit 516 as one example. The data acquisition unit 516 includes an electrical function portion 516b, an attachment member 516c, and screws 516d. The electrical function portion 516b includes an electronic component and a housing 516f that houses the electronic component. The electronic component is electrically connected to the thread-shaped sensor 515.

The housing 516f includes a socket 516h into which the one end of the cable 514b is inserted, and insertion holes for inserting the screws 516d. A screw groove into which the screw 516d is screwed is provided on an inner surface of each insertion hole. The attachment member 516c is a member for attaching the electrical function portion 516b to the sheet-shaped fabric 512. The attachment member 516c includes a plate-shaped portion 516j and a protruding portion 516k protruding in the third direction A503 from an end portion of the plate-shaped portion 516j in the first direction A501 (an end portion at the lower right in FIG. 24). The plate-shaped portion 516j has through-holes 516p penetrating through the plate-shaped portion 516j in the third direction A503.

A method for fixing the data acquisition unit 516 to the sheet-shaped fabric 512 will be described. The electrical function portion 516b is disposed on the sheet-shaped fabric 512 (the inner fabric 512b or the outer fabric 512c). As one example, the electrical function portion 516b is disposed on a front surface of the inner fabric 512b, and the attachment member 516c is disposed on a back surface (lower surface in FIG. 24) of the inner fabric 512b. The plate-shaped portion 516j comes into contact with the back surface of the inner fabric 512b. The plate-shaped portion 516j is disposed such that the protruding portion 516k protrudes in the first direction A501 from the end portion of the sheet-shaped fabric 512 in the first direction A501 and protrudes in the third direction A503 (upward in FIG. 24).

The screws 516d are inserted into the through-holes 516p along the third direction A503. For example, hole into which the screws 516d are inserted are formed in the sheet-shaped fabric 512, and the screws 516d inserted into the through-holes 516p penetrate through the holes. The screws 516d penetrating through the holes are screwed into the screw grooves of the insertion holes of the housing 516f. The inner fabric 512b is sandwiched between the electrical function portion 516b and the attachment member 516c, so that the data acquisition unit 516 is fixed to the sheet-shaped fabric 512.

Incidentally, the configuration of the data acquisition unit 516 and the mode of fixing the data acquisition unit 516 to the sheet-shaped fabric 512 are not limited to the above-described example, and can be changed as appropriate. The data acquisition unit 516 may not be fixed to the sheet-shaped fabric 512.

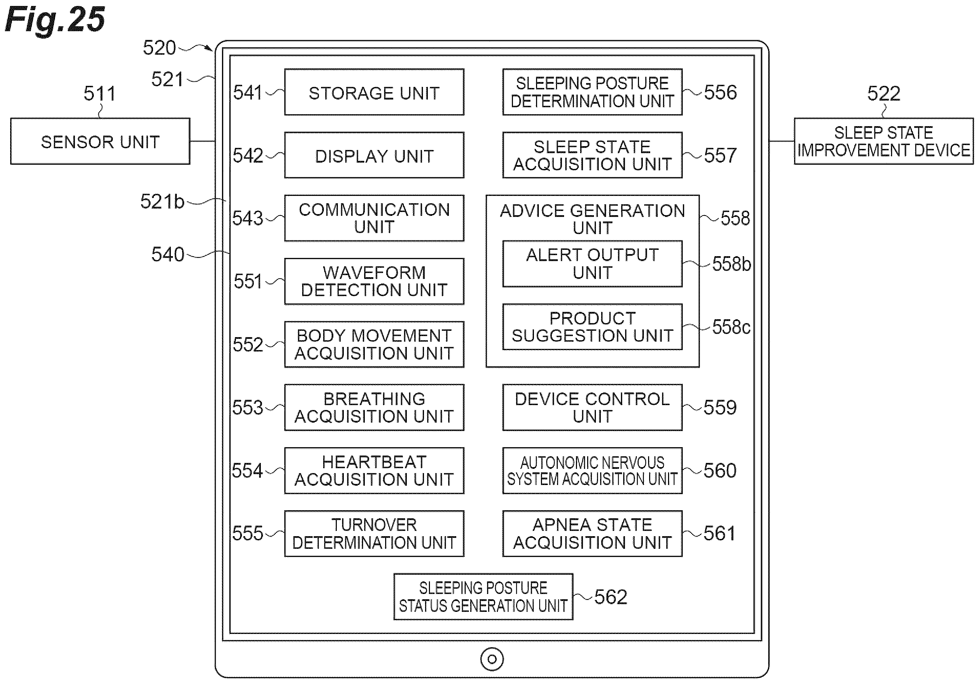

As one example, the sleeping posture determination system and the sleeping posture determination program will be described. FIG. 25 is a block diagram showing a functional configuration of a sleeping posture determination system 520 and a sleeping posture determination program 540 as one example. As shown in FIG. 25, the sleeping posture determination system 520 includes, for example, the sensor unit 511, an information terminal 521, and a sleep state improvement device 522.

The information terminal 521 is a computer that executes each step of the sleeping posture determination program 540. The information terminal 521 is, for example, a mobile terminal. The "mobile terminal" refers to, for example, a portable information terminal such as a mobile phone including a smartphone, a tablet, a notebook computer, or a wearable terminal such as a wristwatch. The information terminal 521 may be a terminal other than a mobile terminal, or may be, for example, a desktop computer.

The information terminal 521 includes, as one example, a processor (for example, a CPU) that executes an operating system, software (application), and the like; a main storage unit composed of a ROM and a RAM; an auxiliary storage unit composed of a flash memory and the like; a communication control unit composed of a wireless communication module and the like; an input device; and an output device such as a display. However, the configuration of the information terminal 521 is not limited to the above-described configuration, and can be changed as appropriate. Hereinafter, the ROM and the RAM may be collectively referred to as memory.

In the information terminal 521, the sleeping posture determination program 540 is executed as an application program. The sleeping posture determination program 540 is, for example, an application downloaded to the information terminal 521 and executed on the information terminal 521. However, the sleeping posture determination program 540 may be executed in a server. The sleeping posture determination program 540 may be a program downloaded from the server. Hereinafter, an example in which the sleeping posture determination program 540 is an application downloaded to the information terminal 521 and the functions of the sleeping posture determination program 540 are executed on the information terminal 521 will be described.

Each function of the sleeping posture determination program 540 is realized by loading predetermined software into the processor or the main storage unit and executing the software. The data or database required to execute the functions of the sleeping posture determination program 540 is stored in the main storage unit or the auxiliary storage unit.

Each functional element of the information terminal 521 is realized by loading predetermined software into the processor or the storage unit (for example, the main storage unit or the auxiliary storage unit described above) and executing the software. The data or database used for the processing of the information terminal 521 is stored in the storage unit.

The sleeping posture determination program 540 may be a distributed processing system composed of a plurality of computers, or may be a client-server system or a cloud system. The sleeping posture determination program 540 includes, for example, a main module, a data acquisition module, a determination module, and an output module. The data acquisition module, the determination module, and the output module are executed, thereby causing each functional element of the sleeping posture determination program 540 to function. As one example, the sleeping posture determination program 540 may be provided in a state where the sleeping posture determination program 540 is permanently recorded on a tangible storage medium such as a CD-ROM, a DVD-ROM, or a semiconductor memory. The sleeping posture determination program 540 may be provided via a communication network as a data signal superimposed on a carrier wave.

As described above, the sensor unit 511 acquires information about the body of the user as vital data. The sensor unit 511 acquires at least one of breathing information, body movement information, and heartbeat information as the vital data. As one example, the sensor unit 511 acquires the breathing information, the body movement information, and the heartbeat information. The contents of the information acquired by the sensor unit 511 can be changed as appropriate. The sensor unit 511 may acquire, for example, at least one of information indicating blood pressure (blood pressure information) and pulse rate.

The sensor unit 511 generates an electrical signal corresponding to the applied load of the body of the user based on the load. The sensor unit 511 generates the electrical signal as a waveform, and the waveform includes the breathing information, the body movement information, and the heartbeat information described above. Hereinafter, the waveform may also be referred to as signal. The sensor unit 511 includes a communication unit that outputs the waveform to the outside of the sensor unit 511. Incidentally, an example in which the sensor unit 511 includes the thread-shaped sensor 515 has been described above. However, the sensor unit 511 may include a sensor other than the thread-shaped sensor 515, and may include, for example, an acceleration sensor.

The breathing information includes a signal (waveform) indicating body movement associated with the breathing of the user. The sensor unit 511 detects body movement associated with the breathing of the user. The sensor unit 511 outputs the signal indicating the body movement associated with breathing to the outside of the sensor unit 511.

The sensor unit 511 detects body movement associated with the heartbeat of the user. The sensor unit 511 outputs a signal indicating the body movement associated with heartbeat to the outside of the sensor unit 511. The sensor unit 511 detects body movement not associated with the breathing and heartbeat of the user. The sensor unit 511 outputs a signal indicating the body movement not associated with breathing and heartbeat to the outside of the sensor unit 511.

An example of the functional configuration of the sleeping posture determination program 540 will be described. The sleeping posture determination program 540 includes a storage unit 541, a display unit 542, and a communication unit 543. The storage unit 541 stores data used to execute the functions of the sleeping posture determination program 540 and data obtained as a result of executing the functions of the sleeping posture determination program 540. The storage unit 541 stores, for example, the data in the memory of the information terminal 521. In this case, the function of the storage unit 541 is realized by the CPU and the memory of the information terminal 521.

For example, the display unit 542 displays the result of executing the functions of the sleeping posture determination program 540 on a display 521b of the information terminal 521. In this case, the function of the display unit 542 is realized by the CPU and the output device of the information terminal 521. However, the display unit 542 may display the result of executing the functions of the sleeping posture determination program 540 on the display of a terminal other than the information terminal 521.

The communication unit 543 transmits and receives the data stored in the storage unit 541 to and from a terminal other than the information terminal 521. For example, the communication unit 543 transmits the data, which is obtained as a result of executing the functions of the sleeping posture determination program 540, to an information terminal external to the information terminal 521. The communication unit 543 may allow the information terminal 521 to receive data from the information terminal external to the information terminal 521. The function of the communication unit 543 is realized by the communication control unit of the information terminal 521.

The sleeping posture determination program 540 includes, as the functional configuration, a waveform detection unit 551, a body movement acquisition unit 552, a breathing acquisition unit 553, a heartbeat acquisition unit 554, a turnover determination unit 555, a sleeping posture determination unit 556, a sleep state acquisition unit 557, an advice generation unit 558, a device control unit 559, an autonomic nervous system acquisition unit 560, an apnea state acquisition unit 561, and a sleeping posture status generation unit 562. The functions of the waveform detection unit 551, the body movement acquisition unit 552, the breathing acquisition unit 553, the heartbeat acquisition unit 554, the turnover determination unit 555, the sleeping posture determination unit 556, the sleep state acquisition unit 557, the advice generation unit 558, the device control unit 559, the autonomic nervous system acquisition unit 560, the apnea state acquisition unit 561, and the sleeping posture status generation unit 562 are realized by causing the CPU of the information terminal 521 to operate according to instructions of the sleeping posture determination program 540 installed in the information terminal 521. The autonomic nervous system acquisition unit 560 acquires autonomic nervous system information, which is information indicating the state of the autonomic nervous system of the user, from the heartbeat information acquired by the sensor unit 511. The apnea state acquisition unit 561 detects the state of apnea of the user from the breathing information acquired by the sensor unit 511.

The waveform detection unit 551 detects, via the communication unit 543, the waveform generated by the sensor unit 511. For example, the waveform detected by the waveform detection unit 551 includes waveforms indicating the breathing information, the body movement information, and the heartbeat information. The waveform detected by the waveform detection unit 551 may include at least one of the blood pressure information and the pulse rate.

The body movement acquisition unit 552 acquires the body movement information of the user from the waveform obtained via the waveform detection unit 551. The breathing acquisition unit 553 acquires the breathing information of the user from the waveform obtained via the waveform detection unit 551. The heartbeat acquisition unit 554 acquires the heartbeat information of the user from the waveform obtained via the waveform detection unit 551.

The turnover determination unit 555 determines, for example, the presence or absence of the turning over of the user during sleep, based on a voltage value shown as a waveform obtained from the thread-shaped sensor 515 described above. The turnover determination unit 555 may determine the presence or absence of the turning over of the user during sleep, based on the waveform, and when it is determined that the user has turned over during sleep, based on the waveform, the turnover determination unit 555 may store information about the turning over during sleep in the storage unit 541. The information about turning over during sleep indicates information about a change in turning over during sleep such as from a supine posture to a lateral posture or from a lateral posture to a prone posture.

The sleeping posture determination unit 556 determines the sleeping posture of the user based on the waveform output by the sensor unit 511. The sleeping posture determination unit 556 has a function different from that of the turnover determination unit 555 in that the turnover determination unit 555 determines whether the user has turned over during sleep, whereas the sleeping posture determination unit 556 determines that the user turns over at that time during sleep. The sleeping posture determination unit 556 executes, in the information terminal 521, a step of determining the sleeping posture of the user based on the waveform indicating the load of the user of the bedding 501 output by the sensor unit 511.

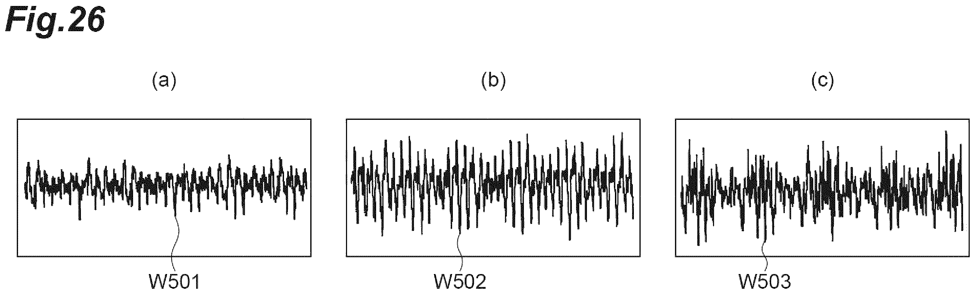

The sleeping posture determination unit 556 determines whether the sleeping posture of the user is a supine posture, a lateral posture, or a prone posture, based on the waveform output by the sensor unit 511. (a), (b), and (c) in FIG. 26 show an example of a waveform obtained when the user is in a supine posture, an example of a waveform obtained when the user is in a lateral posture, and an example of a waveform obtained when the user is in a prone posture, respectively. In each of (a), (b), and (c) of FIG. 26, the horizontal axis represents time, and the vertical axis represents the amplitude of the voltage value.

As shown in (a) of FIG. 26, when the user is in a supine posture, relatively flat portions of the user such as the back and the buttocks come into contact with the sensor unit 511, and the pressure is less likely to be concentrated thereon, and therefore, a waveform W501 having a relatively small amplitude is obtained by the sensor unit 511. As shown in (b) of FIG. 26, when the user is in a lateral posture, a relatively protruding portion of the user such as the shoulder, a protruding portion of the ilium, the upper arm, the elbow, the forearm, the side, or the ribs comes into contact with the sensor unit 511, and the pressure is likely to be concentrated at a single point, and therefore, a waveform W502 having a relatively large amplitude is obtained by the sensor unit 511. As shown in (c) of FIG. 26, when the user is in a prone posture, the distance from the sensor unit 511 to the heart is short or the hands are caught between the front of the body and the cover fabric 503, and therefore, a waveform W503 having more noise compared to other cases is obtained.

For example, the storage unit 541 stores a first simulated waveform indicating a supine posture, a second simulated waveform indicating a lateral posture, and a third simulated waveform indicating a prone posture in advance. The first simulated waveform is a waveform similar to the waveform W501, the second simulated waveform is a waveform similar to the waveform W502, and the third simulated waveform is a waveform similar to the waveform W503.

For example, the sleeping posture determination unit 556 compares the waveform output by the sensor unit 511 with the first simulated waveform, the second simulated waveform, and the third simulated waveform, and determines which of the first simulated waveform, the second simulated waveform, and the third simulated waveform is closest to the waveform output by the sensor unit 511. In this case, when the waveform output by the sensor unit 511 is closest to the first simulated waveform, the sleeping posture determination unit 556 determines that the sleeping posture of the user is a supine posture, when the waveform output by the sensor unit 511 is closest to the second simulated waveform, the sleeping posture determination unit 556 determines that the sleeping posture of the user is a lateral posture, and when the waveform output by the sensor unit 511 is closest to the third simulated waveform, the sleeping posture determination unit 556 determines that the sleeping posture of the user is a prone posture.

For example, the sleeping posture determination unit 556 may measure time-series data of each of the supine posture, the lateral posture, and the prone posture. As one example, the sleeping posture determination unit 556 determines the sleeping posture of the user based on the waveform of the voltage value obtained from the thread-shaped sensor 515. The sleeping posture determination unit 556 may measure the time and the number of times the user is in a supine posture, the time and the number of times the user is in a lateral posture, and the time and the number of times the user is in a prone posture.

For example, the sleep state acquisition unit 557 acquires a sleep stage from the breathing information, the body movement information, and the heartbeat information. The sleep state acquisition unit 557 acquires a sleep onset state (ease of falling asleep) from the time from a bedtime to a sleep onset time. The sleep state acquisition unit 557 acquires an awake state (ease of waking) from the time from an awakening time to a wake-up time and a sleep stage a certain time before the awakening time. For example, the certain time is set in advance, and can be changed as appropriate. The certain time before the awakening time means, for example, immediately before the awakening time. When the awake state is acquired from the sleep stage the certain time before the awakening time, the ease of waking can be effectively identified.

The sleep onset time refers to the time the sleep stage first becomes the stage of sleep (a stage other than wakefulness) between the bedtime and the wake-up time. The awakening time refers to the time the sleep stage finally becomes the stage of awakening between the bedtime and the wake-up time.

As one example, the sleep state acquisition unit 557 acquires the bedtime and the wake-up time of the user from the body movement information acquired by the sensor unit 511 and the movement of the bedding detected by the acceleration sensor of the sensor unit 511. The sleep state acquisition unit 557 acquires the sleep stage using the signal indicating body movement associated with breathing, the signal indicating body movement associated with heartbeat, and the signal indicating body movement not associated with breathing and heartbeat that are output from the sensor unit 511.

For example, the sleep state acquisition unit 557 acquires the sleep onset time from the bedtime and the sleep stage. More specifically, the sleep state acquisition unit 557 acquires, as the sleep onset time, the time the sleep stage first becomes the stage of sleep between the bedtime and the wake-up time. The sleep state acquisition unit 557 acquires the awakening time from the wake-up time and the sleep stage. More specifically, the sleep state acquisition unit 557 acquires, as the awakening time, the time the sleep stage finally becomes the stage of awakening between the bedtime and the wake-up time. The sleep state acquisition unit 557 acquires the sleep stage the certain time before the awakening time.

The sleep state acquisition unit 557 determines, for example, whether the time from the bedtime to the sleep onset time is equal to or longer than a predetermined time, using the bedtime and the sleep onset time. The sleep state acquisition unit 557 determines whether the sleep onset state of the user is good, based on the determination result regarding the time from the bedtime to the sleep onset time. For example, the sleep state acquisition unit 557 determines, as the ease of falling asleep, whether the sleep onset state is good.

The sleep state acquisition unit 557 determines, for example, whether the time from the awakening time to the wake-up time is equal to or longer than a predetermined time, using the awakening time and the wake-up time. The sleep state acquisition unit 557 determines, for example, whether the sleep stage of the user the certain time before the awakening time is non-REM sleep, using the sleep stage the certain time before the awakening time. The sleep state acquisition unit 557 determines whether the awake state of the user is good, based on at least one of the above-described determination results. For example, the sleep state acquisition unit 557 determines, as the ease of waking, whether the awake state is good.

For example, when it is determined that the time from the bedtime to the sleep onset time is not equal to or longer than the predetermined time, the sleep state acquisition unit 557 determines that the sleep onset state of the user is good (falling asleep easily). For example, when it is determined that the time from the bedtime to the sleep onset time is equal to or longer than the predetermined time, the sleep state acquisition unit 557 determines that the sleep onset state of the user is poor (falling asleep poorly).

For example, when it is determined that the time from the awakening time to the wake-up time is not equal to or longer than the predetermined time, the sleep state acquisition unit 557 determines that the awake state of the user is good (waking easily). For example, when it is determined that the sleep stage of the user the certain time before the awakening time is not non-REM sleep, the sleep state acquisition unit 557 determines that the awake state of the user is good (waking easily). For example, when it is determined that the time from the awakening time to the wake-up time is equal to or longer than the predetermined time, or when it is determined that the sleep stage of the user the certain time before the awakening time is non-REM sleep, the sleep state acquisition unit 557 may determine that the awake state of the user is poor.

The advice generation unit 558 generates advice for improving the sleeping posture of the user based on the sleeping posture determined by the sleeping posture determination unit 556. The advice generation unit 558 executes, in the information terminal 521, a step of generating advice for improving the sleeping posture of the user based on the sleeping posture determined in the determination step.

For example, the advice generation unit 558 includes an alert output unit 558b and a product suggestion unit 558c. The alert output unit 558b outputs an alert regarding the sleeping posture to the user as advice. For example, the alert output unit 558b generates, as advice, an alert indicating that the user is in a supine posture more often and experiences apnea more frequently than in other sleeping postures. The product suggestion unit 558c outputs advice regarding product suggestions. For example, the product suggestion unit 558c outputs, as advice, a suggestion of a body pillow product that encourages a user, who experiences apnea frequently, to sleep on his or her side.

The advice is, for example, advice for improving the physical condition of the user. For example, the advice is either advice regarding which sleeping posture leads to a good sleep state or advice regarding which sleeping posture can improve the sleep state when the sleep state is not good.

The advice may be either advice regarding which sleeping posture the user should spend more time in to feel energized during the day or advice regarding which sleeping posture the user should spend more time in to enhance immunity. The advice generated by the advice generation unit 558 should be useful to the user whose sleeping posture is determined, and the type of advice is not particularly limited.

The storage unit 541 stores a plurality types of advice to be provided to the user. The advice generation unit 558 generates advice to be provided to the user by extracting advice corresponding to the determination result of the sleeping posture determination unit 556 from the advice stored in the storage unit 541. For example, the advice extracted from the storage unit 541 by the advice generation unit 558 is displayed on the display 521b of the information terminal 521 by the display unit 542.

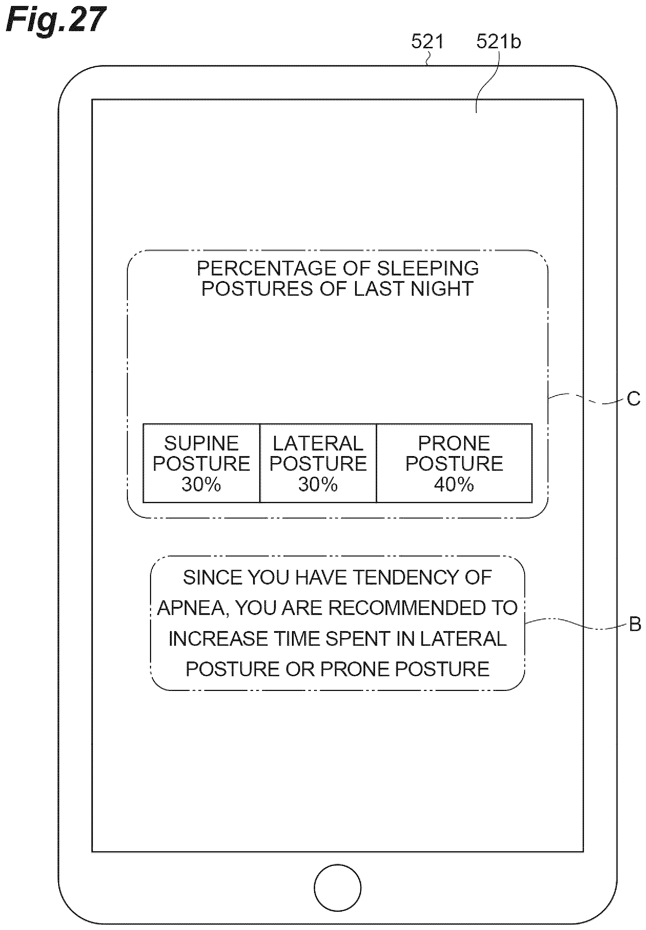

FIG. 27 is a view showing an example of advice. For example, the display unit 42 displays a sleep state C together with advice B. The sleep state C indicates the percentage of the sleeping postures during the previous sleep (last night). The sleep state C indicates the percentage of a supine posture state, a lateral posture state, and a prone posture state during the previous sleep. The advice B is advice for improving sleep according to the content of the sleep state C. As one example, the advice B indicates that since the measured user has a tendency of apnea, the user is recommended to assume a lateral posture or a prone posture.

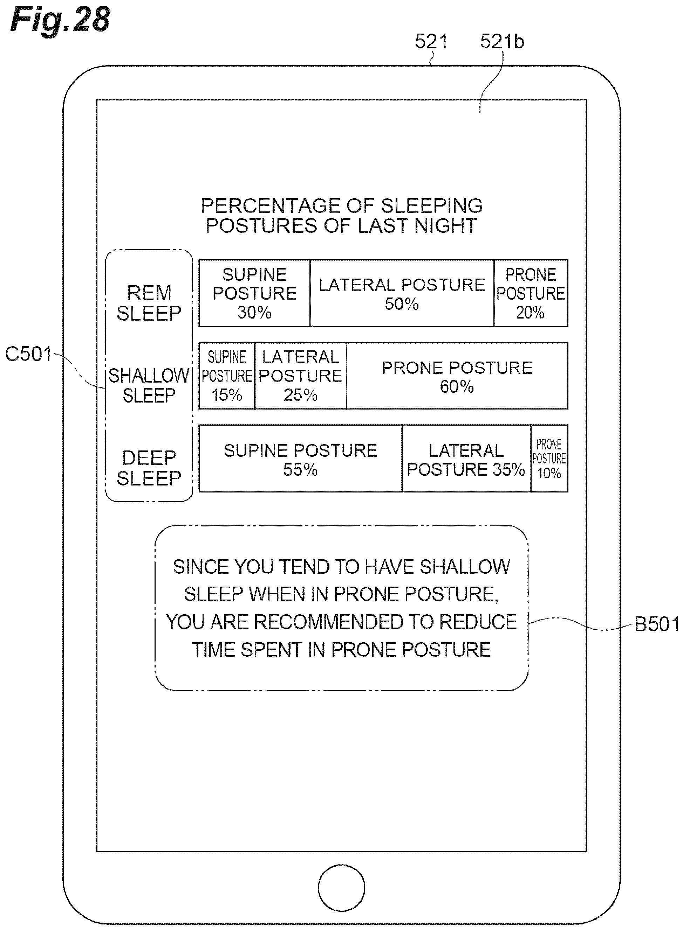

FIG. 28 is a view showing another example of advice. For example, the display unit 542 displays advice B501 extracted by the advice generation unit 558 on the display 521b of the information terminal 521, together with a sleep state C501 acquired by the sleep state acquisition unit 557. The display unit 542 displays the percentage of the sleeping postures during each of REM sleep and non-REM sleep based on the sleeping posture determined by the sleeping posture determination unit 556 and the sleep state acquired by the sleep state acquisition unit 557.

For example, the display unit 542 displays the percentage of the sleeping postures during each of REM sleep, non-REM sleep and shallow sleep, and non-REM sleep and deep sleep. FIG. 28 shows an example in which during REM sleep, the percentage of the supine posture is 30%, the percentage of the lateral posture is 50%, and the percentage of the prone posture is 20%, during shallow sleep, the percentage of the supine posture is 15%, the percentage of the lateral posture is 25%, and the percentage of the prone posture is 60%, and during deep sleep, the percentage of the supine posture is 55%, the percentage of the lateral posture is 35%, and the percentage of the prone posture is 10%.

By displaying the percentage of the sleeping postures when the user is in each sleep state (sleep stage), the user is enabled to identify in which sleep state (sleep stage) the user is more likely to assume a supine posture, a lateral posture, or a prone posture. In the case of the example of FIG. 28, it can be identified that during REM sleep, the user is more often in a lateral posture, during shallow sleep, the user is more often in a prone posture, and during deep sleep, the user is more often in a supine posture.

The display unit 542 displays the advice B501 together with the percentage of the sleeping postures in each sleep state. The advice B501 is, for example, advice indicating the current condition of the sleep state with respect to the sleeping posture. As a specific example, the advice B501 is advice stating "since you tend to have shallow sleep when in a prone posture, you are recommended to reduce the time spent in a prone posture". The type of the advice B501 displayed on the display 521b differs for each user depending on the sleeping posture and the sleep state during the previous sleep. Therefore, optimal advice can be provided to the user based on the sleeping posture and the sleep state. In this manner, the sleeping posture for improving the sleep state can be suggested for each user.

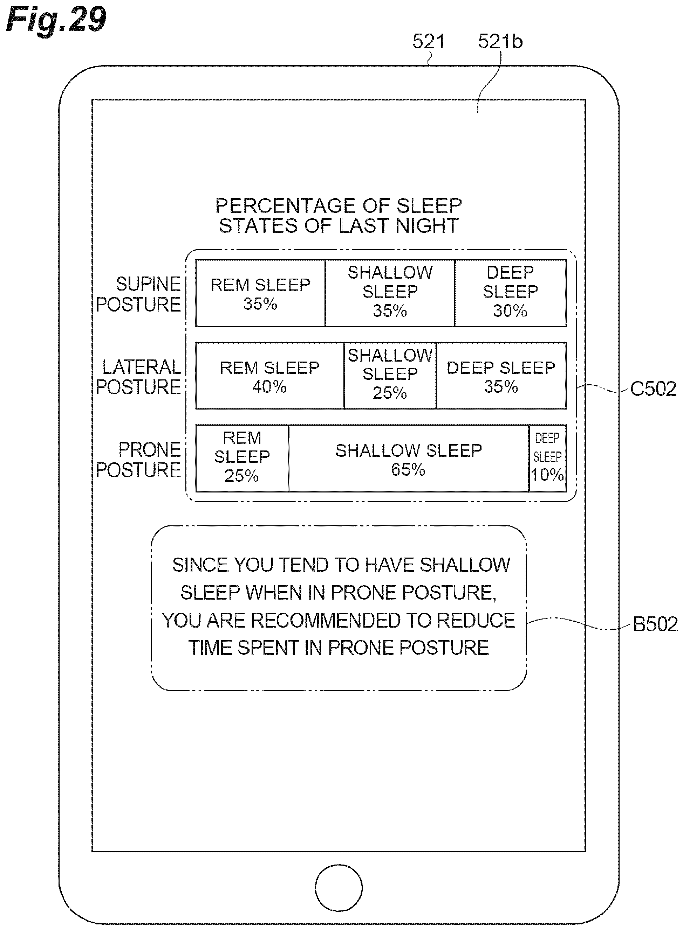

FIG. 29 is a view showing a display screen, advice B502, and a sleep state C502 different from those of FIG. 28. The display unit 542 displays the percentage of the sleep states (sleep stages) when the user is in each of a supine posture, a lateral posture, and a prone posture, based on the sleeping posture determined by the sleeping posture determination unit 556 and the sleep state acquired by the sleep state acquisition unit 557. For example, the display unit 542 displays the percentage of REM sleep and non-REM sleep (shallow sleep and deep sleep) when the user is in each of a supine posture, a lateral posture, and a prone posture.

FIG. 29 shows an example in which when the user is in a supine posture, the percentage of REM sleep is 35%, the percentage of shallow sleep is 35%, and the percentage of deep sleep is 30%, when the user is in a lateral posture, the percentage of REM sleep is 40%, the percentage of shallow sleep is 25%, and the percentage of deep sleep is 35%, and when the user is in a prone posture, the percentage of REM sleep is 25%, the percentage of shallow sleep is 65%, and the percentage of deep sleep is 10%. By displaying the percentage of the sleep states (sleep stages) when the user is in each sleeping posture, the user is enabled to identify in which sleeping posture the user is more likely to have REM sleep, shallow sleep, or deep sleep. In the case of the example of FIG. 29, it can be identified that the user is more likely to have either REM sleep or deep sleep when in a lateral posture, and the user is more likely to have shallow sleep when in a prone posture.

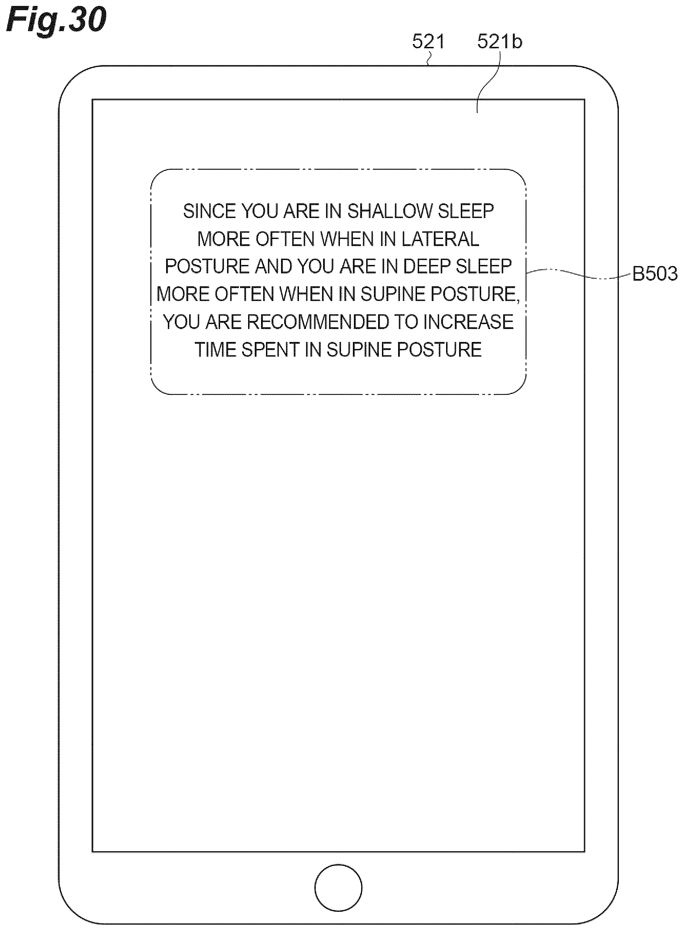

FIG. 30 is a view showing an example of advice B503 different from those of FIGS. 28 and 29. The advice generation unit 558 generates the advice B503 for improving the sleeping posture of the user based on the sleeping posture determined by the sleeping posture determination unit 556, and for example, the advice B503 is displayed on the display 521b of the information terminal 521 by the display unit 542. In FIG. 30, since the sleeping posture determination unit 556 determines that the user is in shallow sleep more often when in a lateral posture and the user is in deep sleep more often when in a supine posture, the advice generation unit 558 generates the advice B503 indicating a recommendation of a supine posture, and the advice B503 is displayed on the display 521b.

As shown in FIG. 25, the sleeping posture determination program 540 includes the device control unit 559. The device control unit 559 controls, for example, the operation of the sleep state improvement device 522 based on at least one of the sleep state stored in advance in the storage unit 541, the autonomic nervous system information acquired by the autonomic nervous system acquisition unit 560, and the apnea state acquired by the apnea state acquisition unit 561.

The sleep state improvement device 522 includes, for example, at least one of the bedding 501, an air conditioner installed in the bedroom of the user, a lighting fixture, a pillow used by the user during sleep, and a heated mattress. For example, when the sleep state improvement device 522 is a pillow, the device control unit 559 controls the angle of the placement surface of the pillow, on which the head of the user is placed, with respect to a horizontal plane by controlling the operation of the pillow. For example, when the apnea state acquisition unit 561 acquires an apnea state for a certain period of time or longer, the device control unit 559 adjusts the inclination of the pillow such that the head of the user is adapted to sleeping on his or her side. Accordingly, apnea of the user can be reduced.

The autonomic nervous system acquisition unit 560 acquires the autonomic nervous system information, which is information indicating the state of the autonomic nervous system of the user, from the heartbeat information acquired by the sensor unit 511. The autonomic nervous system acquisition unit 560 acquires, for example, the heart rate variability (HRV) of the user by analyzing the heartbeat information acquired by the sensor unit 511. As one example, the autonomic nervous system acquisition unit 560 acquires, from the heart rate variability of the user, information indicating periodic components contained in the heart rate variability. The autonomic nervous system acquisition unit 560 performs frequency analysis on the periodic components of the heart rate variability to acquire information indicating the power spectrum of each frequency. The autonomic nervous system acquisition unit 560 acquires, as the autonomic nervous system information, information indicating a sympathetic nervous system index and information indicating a parasympathetic nervous system index.

The autonomic nervous system acquisition unit 560 acquires the mental state of the user from the autonomic nervous system information. The autonomic nervous system acquisition unit 560 classifies, for example, the mental state of the user into one of a high-performance state, a relaxed state, a stressed state, and a depressed state, based on the autonomic nervous system information.

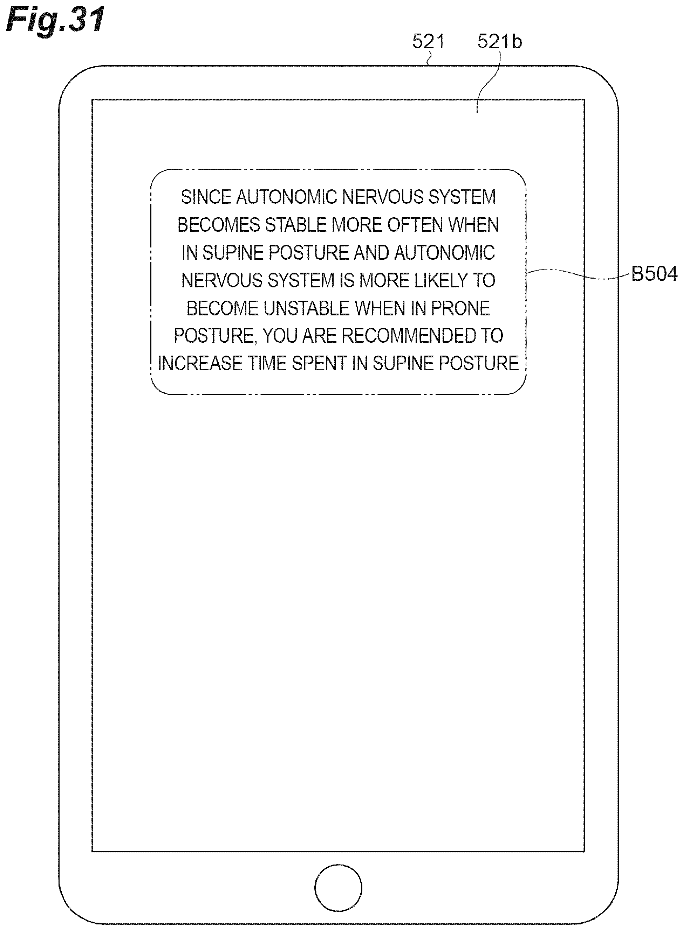

The autonomic nervous system information acquired by the autonomic nervous system acquisition unit 560 is stored in the storage unit 541. As shown in FIG. 31, the advice generation unit 558 may generate advice B504 including the autonomic nervous system information acquired by the autonomic nervous system acquisition unit 560, and the display unit 542 may display the advice B504 on the display 521b of the information terminal 521.

For example, the advice generation unit 558 generates the advice B504 regarding which sleeping posture is likely to cause the autonomic nervous system to become stable when more time is spent therein and which sleeping posture is likely to cause the autonomic nervous system to become unstable when more time is spent therein. In the example of FIG. 31, since the autonomic nervous system is likely to become stable when the user is in a supine posture, and the autonomic nervous system is likely to become unstable when the user is in a prone posture, the advice B504 indicating a recommendation of a supine posture is displayed on the display 521b.

The apnea state acquisition unit 561 detects the state of apnea (apnea state) of the user from the breathing information. The apnea state acquisition unit 561 detects, for example, the abnormal breathing state of the user from the signal indicating body movement associated with breathing, the signal indicating body movement associated with heartbeat, and the signal indicating body movement not associated with breathing and heartbeat that are output from the sensor unit 511.

By the way, when the breathing state changes from the abnormal breathing state to the normal breathing state that is not the abnormal breathing state, the body movement of the user tends to occur. As one example, the apnea state acquisition unit 561 detects the abnormal breathing state from the body movement information acquired by the sensor unit 511. The apnea state acquisition unit 561 detects, for example, the abnormal breathing state of the user from the signal indicating body movement associated with breathing, the signal indicating body movement associated with heartbeat, and the signal indicating body movement not associated with breathing and heartbeat that are output from the sensor unit 511.

When the breathing state changes from the abnormal breathing state to the normal breathing state, the heart rate of the user may suddenly increase. The apnea state acquisition unit 561 may detect the abnormal breathing state from the heartbeat information acquired by the sensor unit 511. In this case, the apnea state acquisition unit 561 detects the abnormal breathing state of the user from the signal indicating body movement associated with heartbeat that is output from the sensor unit 511.

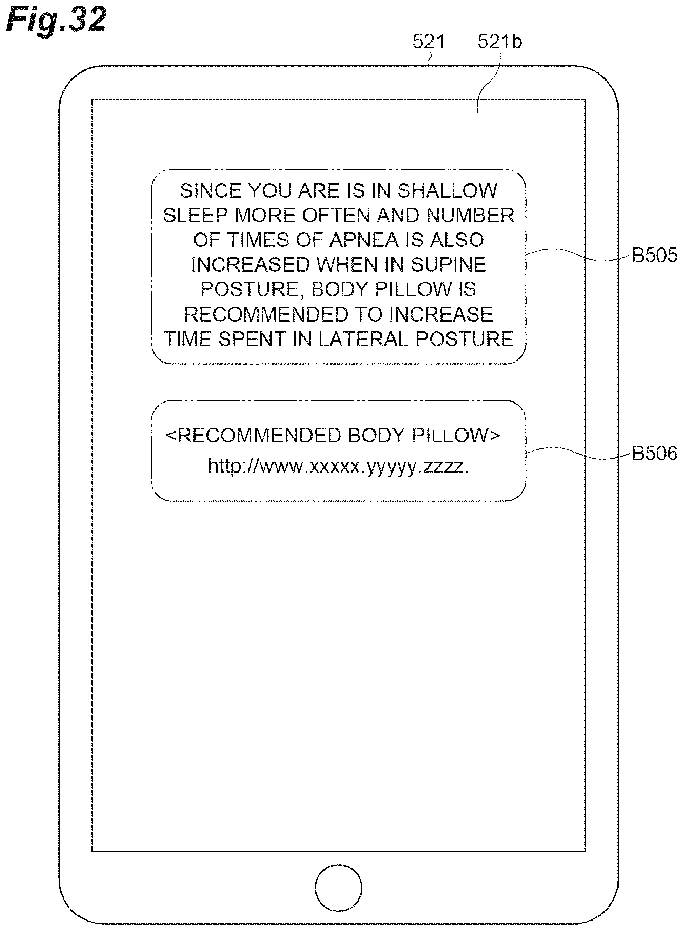

The abnormal breathing state (apnea state) detected by the apnea state acquisition unit 561 is stored in the storage unit 541. As shown in FIG. 32, the advice generation unit 558 may generate advice B505 including information about the abnormal breathing state acquired by the apnea state acquisition unit 561, and the display unit 542 may display the advice B505 on the display 521b of the information terminal 521.

For example, the advice generation unit 558 generates the advice B505 regarding which sleeping posture causes the abnormal breathing state to occur more frequently when more time is spent therein. In the example of FIG. 32, since the user is in shallow sleep more often and the number of times of apnea is also increased when in a supine posture, the advice B505 indicating that the user should assume a lateral posture is displayed on the display 521b.

The product suggestion unit 558c of the advice generation unit 558 may generate advice B506 for suggesting a product that improves the sleep state of the user. In the example of FIG. 32, the advice B506 that encourages the user to purchase a body pillow so as to recommend a lateral posture is generated by the advice generation unit 558, and is display on the display 521b. The display unit 542 may display, as a URL, the advice B506 for the suggested product on the display 521b.

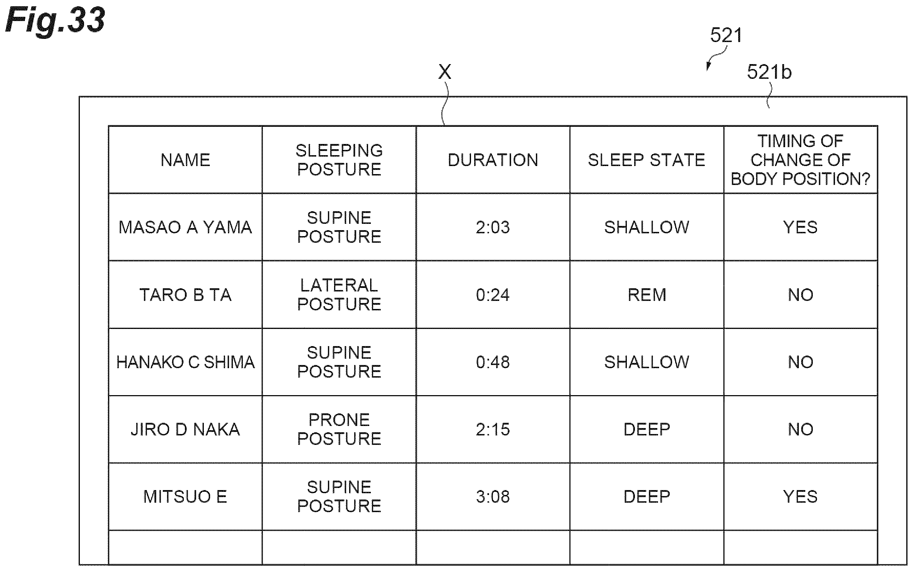

The sleeping posture status generation unit 562 generates the status of the sleeping posture of the user. As shown in FIG. 33, the status of the sleeping posture of the user generated by the sleeping posture status generation unit 562 is displayed on the display 521b of the information terminal 521 by the display unit 542. For example, when the sleeping posture determination system 520 and the sleeping posture determination program 540 are used for a plurality of users in a facility, the sleeping posture determination unit 556 determines the sleeping posture of each user, the sleep state acquisition unit 557 acquires the sleep state of each user, and the advice generation unit 558 generates advice for each user. The sleeping posture status generation unit 562 generates the sleeping posture of each user, the sleep state of each user, and advice for each user, and for example, the display unit 542 displays the advice as Table X on the display 521b of the information terminal 521.

In Table X, the sleeping posture, the duration of the sleeping posture, the sleep state, and whether it is the timing of change of body position are displayed for the name of each user. In the example of FIG. 33, whether it is the timing of change of body position is displayed as advice extracted by the advice generation unit 558. For example, in a facility such as a hospital, there are many users (patients) who are unable to change the body position by himself or herself. Nurses or the like may need to change the body positions of the users at regular time intervals to prevent bedsores.

By displaying Table X on the display 521b, it is possible to identify which user should undergo a change in body position. Since the state of the sleeping posture, the duration of the sleeping posture, and the sleep state can be identified for each user, the status of the user can be identified with higher accuracy.

The alert output unit 558b may output an alert to a user whose specific sleeping posture has continued for a certain period of time or longer. The alert is output, for example, by at least one of a display on the display 521b and a voice. For example, the alert output unit 558b may highlight the row of a user whose duration is two hours (or three hours) or more in Table X. As one example, the alert output unit 558b may output an alert to a user who has continued to assume one of a supine posture, a lateral posture, and a prone posture for two hours (three hours) or more. In this case, since a user who needs to change the body position can be determined, bedsores can be more reliably suppressed. Incidentally, even when a specific sleeping posture has continued for a certain period of time or longer, for example, if the sleep state is deep sleep, the advice generation unit 558 may give advice indicating that the body position may not be changed (for example, display "NO" in Table X).

As one example, actions and effects obtained by the sleeping posture determination system 520 and the sleeping posture determination program 540 will be described in more detail. As shown in FIGS. 25 to 27, the sleeping posture determination system 520 includes the sensor unit 511 mounted on the bedding 501, and the sensor unit 511 receives the load of the body of the user placed on the bedding 501, and outputs a waveform corresponding to the load. In the sleeping posture determination system 520 and the sleeping posture determination program 540, the sleeping posture determination unit 556 determines a sleeping posture based on the waveform output by the sensor unit 511. In the sleeping posture determination system 520 and the sleeping posture determination program 540, the advice generation unit 558 generates the advice B for improving the sleeping posture of the user based on the sleeping posture determined by the sleeping posture determination unit 556. The user can receive the advice B for improving his or her own sleeping posture. The life of the user can be improved by improving the sleeping posture.

The sensor unit 511 may acquire, as a waveform, at least one of the breathing information that is information indicating the breathing of the user, the body movement information that is information indicating the body movement of the user, and the heartbeat information that is information indicating the heartbeat of the user. The sleeping posture determination system 520 and the sleeping posture determination program 540 may include the sleep state acquisition unit 557 that acquires a sleep state, which is information indicating the sleep state of the user, from the waveform indicating at least one of the breathing information, the body movement information, the heartbeat information, and the display unit 542 that displays the advice B generated by the advice generation unit 558 and the sleep state C acquired by the sleep state acquisition unit 557. The sensor unit 511 may acquire at least one of the breathing information, the body movement information, and the heartbeat information as a waveform, and the sleep state of the user may be acquired by the sleep state acquisition unit 557 from the waveform. The sleep state C acquired by the sleep state acquisition unit 557 may displayed together with the advice B by the display unit 542. In this case, since the user can receive the advice B together with the tendency of his or her own sleeping posture and the sleep state, more useful information can be provided to the user.

The sensor unit 511 may acquire, as a waveform, the heartbeat information that is information indicating the heartbeat of the user. As shown in FIGS. 25 and 31, the sleeping posture determination system 520 and the sleeping posture determination program 540 may include the autonomic nervous system acquisition unit 560 that acquires the autonomic nervous system information, which is information indicating the autonomic nervous system of the user, from the heartbeat information. The advice generation unit 558 may generate the advice B504 including the autonomic nervous system information acquired by the autonomic nervous system acquisition unit 560. In this case, since the user can identify the state of the autonomic nervous system together with the tendency of his or her own sleeping posture, even more useful information can be provided to the user.

The sensor unit 511 may acquire, as a waveform, the breathing information that is information indicating the state of the breathing of the user. As shown in FIGS. 25 and 32, the sleeping posture determination system 520 and the sleeping posture determination program 540 may include the apnea state acquisition unit 561 that detects the state of apnea of the user from the breathing information. The advice generation unit 558 generates the advice B505 including the state of apnea acquired by the apnea state acquisition unit 561. In this case, by detecting the state of apnea of the user using the apnea state acquisition unit 561, the user is enabled to identify whether he or she experiences apnea and the frequency of apnea. The advice generation unit 558 may generate the advice B505 by taking into account the state of apnea. In this case, the user can obtain information for improving the state of his or her apnea.

The sensor unit 511 may be a single sensor sheet disposed on the bedding 501. In this case, various information (data) can be acquired without coming into contact with the body of the user. More specifically, since the cover fabric 503 and the clothing of the user are interposed between the body of the user and the sensor unit 511, the sensor unit 511 does not come into direct contact with the body. Various information such as not only the sleeping posture but also the sleep state, the autonomic nervous system, and apnea can be easily and accurately acquired using the sensor unit 511 having a sheet shape.

Next, specific examples of the bruxism detection system and the bruxism detection program will be described. As one example, the bruxism detection system detects bruxism of a user. The "bruxism" refers to the dynamic or static grinding of the teeth or the clenching of the teeth. Engaging in bruxism causes the teeth to wear down and crack and worsens tooth sensitivity or periodontal disease, which is a concern. Engaging in bruxism may be a factor in causing temporomandibular joint disorders, headaches, or shoulder stiffness. Since sound is generated by bruxism, the quality of sleep of a human sleeping in the same room may also be worsened.

The bruxism detection system detects bruxism of the user from a sleep onset time to an awakening time. For example, the bruxism detection system may be used for personal or household purposes, or may be used in research institutes or the like for testing and research purposes. The bruxism detection system may be used in hospitals or the like for treatment. The "user" refers to a person who is the subject of bruxism detection by the bruxism detection system. The user is, for example, a person who has a health risk caused by bruxism, a person who receives sleep treatment including treatment for bruxism at a hospital or the like, or a person who wishes to check for the presence or absence of bruxism during his or her sleep.

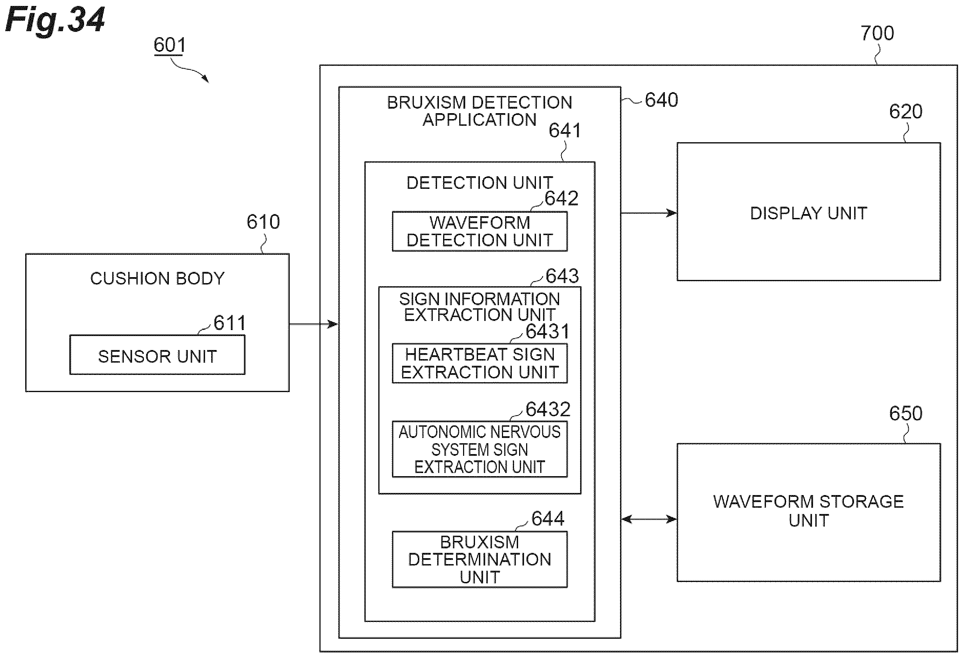

FIG. 34 is a block diagram showing a bruxism detection system 601 as one example. The bruxism detection system 601 can be executed, for example, in an information terminal 700 such as a computer, a tablet terminal, a smartphone, a wristwatch, or a wearable terminal. The bruxism detection system 601 includes, for example, a sensor unit 611 and the information terminal 700. The information terminal 700 includes a bruxism detection application 640 (bruxism detection program) and a display unit 620.

The bruxism detection application 640 is, for example, an application program executed on the information terminal 700. The bruxism detection application 640 may be an application downloaded to the information terminal 700 and executed on the information terminal 700. Hereinafter, an example in which the bruxism detection application 640 is an application downloaded to the information terminal 700 and the functions of the bruxism detection application 640 are executed on the information terminal 700 will be described.

Each function of the bruxism detection application 640 is realized by loading predetermined software into the processor or the main storage unit and executing the software. The data or database required to execute the functions of the bruxism detection application 640 is stored in the main storage unit or the auxiliary storage unit.

The bruxism detection system 601 may be a distributed processing system composed of a plurality of computers, or may be a client-server system or a cloud system. The bruxism detection application 640 includes, for example, a main module, a data acquisition module, a detection module, and an output module. The data acquisition module, the detection module, and the output module are executed, thereby causing each functional element of the bruxism detection application 640 to function. As one example, the bruxism detection application 640 may be provided in a state where the bruxism detection application 640 is permanently recorded on a tangible storage medium such as a CD-ROM, a DVD-ROM, or a semiconductor memory. The bruxism detection application 640 may be provided via a communication network as a data signal superimposed on a carrier wave.

The display unit 620 displays, for example, information generated by the bruxism detection application 640 on the display of the information terminal 700. The display unit 620 is a functional unit realized by the processor and the display included in the information terminal 700. The sensor unit 611 acquires information about the body of the user. As one example, the sensor unit 611 acquires vibration information. The vibration information is information indicating vibration of the user.

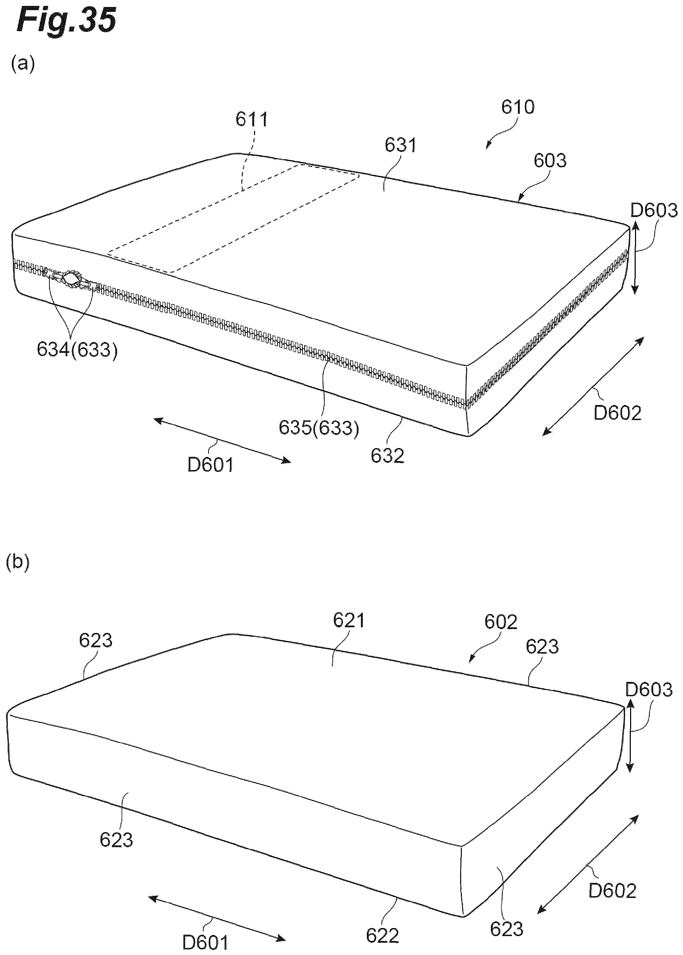

(a) in FIG. 35 is a perspective view showing a cushion body 610 to which the sensor unit 611 is attached. (b) in FIG. 35 is a perspective view showing a core material 602 constituting the cushion body 610 shown in (a) of FIG. 35. As shown in FIGS. 34 and 35, the sensor unit 611 is, for example, a sensor sheet that is attachable to and detachable from the cushion body 610. The sensor unit 611 acquires vital data of the user lying on the cushion body 610.

For example, the sensor unit 611 includes a sheet-shaped fabric on which a thread-shaped sensor is embroidered, and the position of the sheet-shaped fabric with respect to the cushion body 610 can be changed. The thread-shaped sensor of the sensor unit 611 is fixed, for example, by being embroidered such that the thread-shaped sensor spreads out twodimensionally on the sheet-shaped fabric. For example, a piezoelectric sensor of the sensor unit 611 generates an electrical signal corresponding to the applied load of the body of the user based on the load, and the sensor unit 611 acquires the electrical signal as the vibration information described above. The electrical signal acquired as the vibration information may be referred to as a "signal" below.

The sensor unit 611 includes, for example, the thread-shaped sensor (as one example, a piezoelectric sensor), and a communication unit that outputs the vibration information as an electrical signal generated by the thread-shaped sensor to the outside of the sensor unit 611. An example in which the sensor unit 611 includes a piezoelectric sensor has been described above. However, the type of the sensor of the sensor unit 611 is not limited to a piezoelectric sensor, and is not particularly limited. For example, the sensor unit 611 may include an acceleration sensor.

For example, the sensor unit 611 is used while being attached to the cushion body 610 on which the body of the user is placed. The cushion body 610 has a rectangular shape in a plan view. The cushion body 610 extends in a longitudinal direction D601 and a lateral direction D602 orthogonal to the longitudinal direction D601. The cushion body 610 has a thickness in a thickness direction D603 orthogonal to both the longitudinal direction D601 and the lateral direction D602.

A length of the cushion body 610 in the longitudinal direction D601 is, for example, 120 cm or more and 210 cm or less (as one example, 195 cm). A length of the cushion body 610 in the lateral direction D602 is, for example, 70 cm or more and 180 cm or less (as one example, 97 cm). A length of the cushion body 610 in the thickness direction D603 is, for example, 3 cm or more and 40 cm or less (as one example, 9 cm).

As one example, the cushion body 610 is a mattress. The user of the cushion body 610 places his or her own body on the cushion body 610. At this time, a direction in which the body of the lying user extends (namely, a direction in which the head and legs of the user are connected to each other) coincides with, for example, the longitudinal direction D601 of the cushion body 610.

As shown in (b) of FIG. 35, the cushion body 610 includes the core material 602 housed inside a cover fabric 603 to be described later. As one example, the core material 602 has a rectangular shape in a plan view. The core material 602 includes, for example, a content and a bag that houses the content. The content is, for example, urethane foam or polyester.

The core material 602 has a rectangular parallelepiped shape. The core material 602 has an upper surface 621 on which the body of the user is placed, and a lower surface 622 facing opposite the upper surface 621. The upper surface 621 is a surface facing one side (the upper side in (b) of FIG. 35) of the core material 602 in the thickness direction D603. The lower surface 622 is a surface facing the other side (the lower side in (b) of FIG. 35) of the core material 602 in the thickness direction D603. The core material 602 has a plurality of side surfaces 623 connecting the upper surface 621 and the lower surface 622.

As shown in (a) of FIG. 35, the cushion body 610 includes the cover fabric 603. As one example, the cover fabric 603 has a bag shape, and has a rectangular shape in a plan view. The cover fabric 603 includes, for example, a front fabric 631 covering the upper surface 621 of the core material 602; a back fabric 632 covering the lower surface 622 of the core material 602; and an opening and closing member 633 connecting the front fabric 631 and the back fabric 632 to each other. The opening and closing member 633 is provided at a position on the cover fabric 603 which faces the side surfaces 623 of the core material 602. When viewed in the thickness direction D603, the opening and closing member 633 is located outside the side surfaces 623 of the core material 602. The cover fabric 603 is attachable to and detachable from the core material 602.

The opening and closing member 633 is, as one example, a so-called double slider. The opening and closing member 633 includes two sliders 634 and an element 635 that opens and closes the front fabric 631 and the back fabric 632 when the sliders 634 are slid.

The front fabric 631 and the back fabric 632 can be opened by sliding one slider 634 along the element 635 in one direction (a clockwise direction in (a) of FIG. 35) and sliding the other slider 634 along the element 635 in the other direction (a counterclockwise direction in (a) of FIG. 35).

The front fabric 631 and the back fabric 632 can be closed by sliding the one slider 634 along the element 635 in the other direction and sliding the other slider 634 along the element 635 in the one direction. The opening and closing member 633 may be a so-called single slider. In this case, the opening and closing member 633 includes one slider 634 and the element 635.

The sensor unit 611 is disposed, for example, inside the cover fabric 603 having a bag shape. More specifically, the sensor unit 611 is disposed between the core material 602 and the cover fabric 603. When the sensor unit 611 is attached to the cushion body 610, for example, the sensor unit 611 is disposed to extend in the lateral direction D602 of the cushion body 610. In this case, the sensor unit 611 is located on the side opposite the body of the user when viewed from the cover fabric 603 of the cushion body 610. When the body of the user is placed on the cushion body 610, the sensor unit 611 does not come into contact with the user.

The term "the sensor unit 611 not coming into contact with the user" refers to the sensor unit 611 not coming into direct contact with the body of the user. A case in which the sensor unit 611 and the user are indirectly connected to each other due to the cushion body 610 being located between the sensor unit 611 and the user is included in the state where "the sensor unit 611 is not in contact with the user". For example, the clothing worn by the user and the cover fabric 603 is interposed between the body of the user and the sensor unit 611. Accordingly, the sensor unit 611 is not in contact with the body of the user. The sensor unit 611 acquires the vibration information without coming into contact with the user.

The sensor unit 611 is attached, for example, at a position corresponding to the heart of the user in the longitudinal direction D601. As one example, the sensor unit 611 is attached at any position between the position where the head of the user is placed and a position spaced apart in the longitudinal direction D601 of the cushion body 610 (a position on the lower right side in (a) of FIG. 35). The attachment position of the sensor unit 611 in the longitudinal direction D601 can be changed. A distance from the position where the head of the user is placed to the position where the sensor unit 611 is attached may be, for example, 40 cm or more and 50 cm or less (as one example, 50 cm).

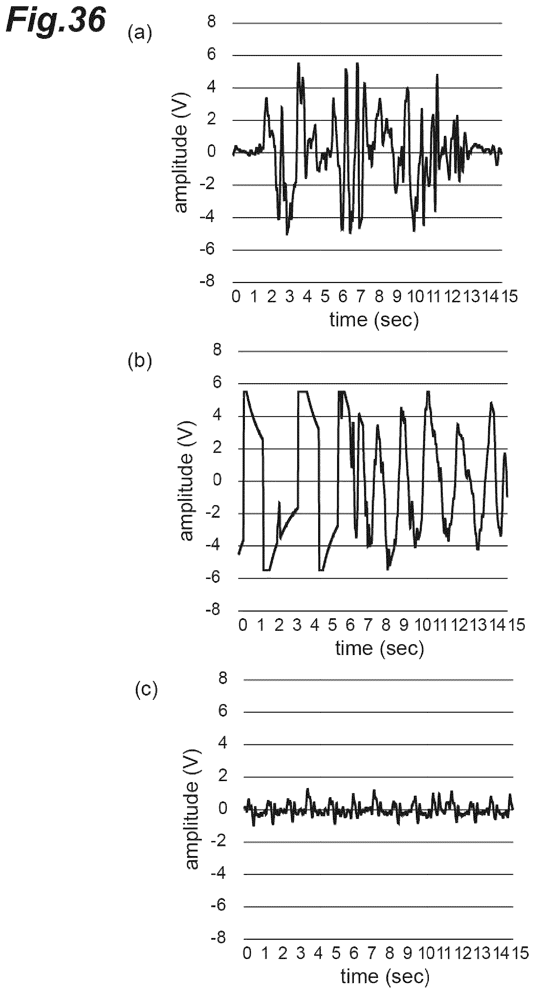

The bruxism detection system 601 includes a waveform storage unit 650. The waveform storage unit 650 is a functional unit realized by the processor and the storage unit included in the information terminal 700. As shown in each of (a), (b), and (c) of FIG. 36, the waveform storage unit 650 stores a schematic waveform obtained by waveform conversion of vibration caused by bruxism (hereinafter, referred to as the "schematic waveform of bruxism"), a schematic waveform obtained by waveform conversion of vibration caused by turning over during sleep (hereinafter, referred to as the "schematic waveform of turning over during sleep"), and a schematic waveform obtained by waveform conversion of vibration occurring at rest (hereinafter, referred to as the "schematic waveform at rest"). (a) in FIG. 36 is a schematic waveform diagram of bruxism. (b) in FIG. 36 is a schematic waveform diagram of turning over during sleep. (c) in FIG. 36 is a schematic waveform diagram at rest.

The schematic waveform of bruxism is, for example, a waveform that reflects theoretical values of the amplitude, frequency, and duration characteristics of vibration caused by bruxism. The schematic waveform of turning over during sleep is, for example, a waveform that reflects theoretical values of the amplitude, frequency, and duration characteristics of vibration caused by turning over during sleep. The schematic waveform at rest is, for example, a waveform that reflects theoretical values of the amplitude, frequency, and duration characteristics of vibration at rest. Each theoretical value may be, for example, a value calculated based on the age, gender, or body type of the user.

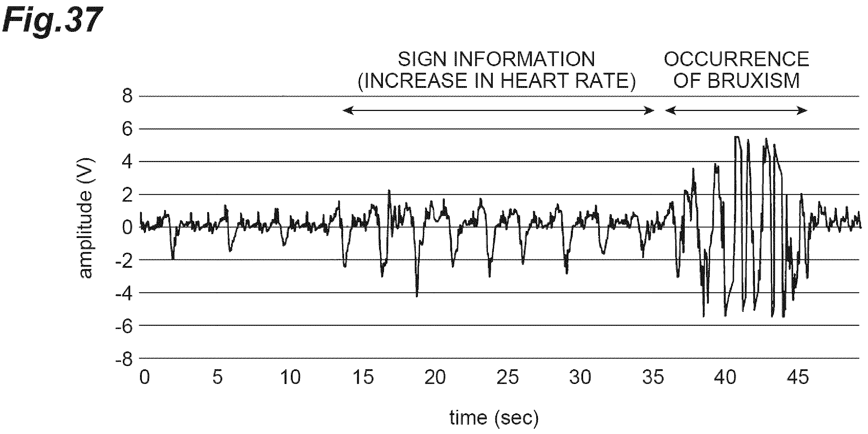

The waveform storage unit 650 stores a typical example of sign information indicating a sign occurring before the onset of bruxism. Examples of the sign information include an increase in the heart rate of the user a predetermined time before the onset of bruxism and an increase in the sympathetic nervous system index of the user before the onset of bruxism. The "heartbeat" refers to the beating of the heart. The "predetermined time" is, for example, a few seconds or immediately before the onset of bruxism. The waveform storage unit 650 stores a schematic waveform of a waveform indicating sign information caused by an increase in heart rate before the onset of bruxism. FIG. 37 is a schematic waveform diagram of the waveform indicating the sign information caused by an increase in heart rate before the onset of bruxism. As shown in FIG. 37, a waveform caused by an increase in heart rate can be confirmed immediately before a waveform caused by bruxism.

The waveform storage unit 650 stores a bruxism waveform in advance. The waveform storage unit 650 stores, for example, the bruxism waveform output by a bruxism determination unit 644 to be described later. The "schematic waveform of bruxism" is a waveform that reflects the theoretical values of the characteristics of vibration caused by bruxism, whereas the "bruxism waveform" is a waveform of past bruxism of the user that is actually detected. The waveform storage unit 650 has, for example, data on the same number of bruxism waveforms as the number of waveforms of past bruxism of the user that is detected. The bruxism waveform may include waveforms of past bruxism of humans other than the user. In this case, the waveform storage unit 650 has data on a larger number of bruxism waveforms than the number of waveforms of past bruxism of the user.

The bruxism detection application 640 acquires, for example, the sleep onset time and the awakening time of the user from the vibration information. For example, the bruxism detection application 640 may determine the sleep onset time based on body movement information acquired by the sensor unit 611 and the movement of the bedding detected by the acceleration sensor of the sensor unit 611, and acquire the sleep onset time. The same applies to the acquisition of the awakening time. Unlike the above-described example, the user may operate the bruxism detection application 640 on the information terminal 700, and the sleep onset time and the awakening time may be acquired through the operation of the user.

The bruxism detection application 640 includes a detection unit 641 that detects bruxism of the user from the vibration information and the sign information. The detection unit 641 includes a waveform detection unit 642 that detects the vibration information as a waveform; a sign information extraction unit 643 that extracts the sign information; and the bruxism determination unit 644 that determines whether the detected waveform is a waveform caused by bruxism.

The detection unit 641 extracts autonomic nervous system information, which is information indicating the state of the autonomic nervous system of the user, from the vibration information. For example, the detection unit 641 extracts heartbeat information from the vibration information. The detection unit 641 acquires the heart rate variability (HRV) of the user by analyzing the extracted heartbeat information. The detection unit 641 acquires the autonomic nervous system information from the acquired heart rate variability of the user. The detection unit 641 performs frequency analysis on the periodic components of the heart rate variability to acquire information indicating the power spectrum of each frequency. The detection unit 641 acquires, as the autonomic nervous system information, information indicating a sympathetic nervous system index and information indicating a parasympathetic nervous system index.

The waveform detection unit 642 detects the vibration information as a waveform by performing waveform conversion on a signal indicating a change in the load applied to the piezoelectric sensor of the sensor unit 611 by the user. When the difference between the amplitude of the detected waveform and the amplitude of the schematic waveform of bruxism is less than or equal to a predetermined threshold value, the waveform detection unit 642 extracts the detected waveform as a candidate waveform. For example, the amplitude of the waveform of bruxism measured by the sheet-type sensor is larger than the amplitude of a waveform caused by breathing and heartbeat at rest measured by the sheet-type sensor, and is smaller than the amplitude of a waveform caused by turning over during sleep and body movement measured by the sheet-type sensor. When the amplitude of the waveform measured by the sheet-type sensor is larger than the amplitude of the waveform at rest and is smaller than the amplitude of the waveform of turning over during sleep, the waveform detection unit 642 may extract, as a candidate waveform, the waveform measured by the sheet-type sensor.

The waveform detection unit 642 may extract a candidate waveform using a method other than comparing the amplitude of the detected waveform with the amplitude of the schematic waveform of bruxism. The waveform detection unit 642 may extract, for example, a candidate waveform based on the amplitude of the detected waveform as well as the frequency of the waveform and the duration of the waveform. For example, bruxism measured by the sheet-type sensor is a phenomenon in which a periodic vibration of 2 Hz or more and 4 Hz or less continues for two seconds or more. The waveform detection unit 642 detects a waveform that continues for two seconds or more. The waveform detection unit 642 detects the frequency of the waveform and the magnitude of the amplitude. The waveform detection unit 642 may extract the waveform as a candidate waveform using the fact that the difference between the amplitude of the waveform and the amplitude of the schematic waveform of bruxism is less than or equal to a threshold value, the fact that the difference between the frequency of the waveform and the frequency of the schematic waveform of bruxism is less than or equal to a predetermined value, and the fact that the duration of the waveform is two seconds or more.

The waveform detection unit 642 may further detect that the detected waveform does not have the characteristics of the schematic waveform of turning over during sleep and the characteristics of the schematic waveform at rest that are stored in the waveform storage unit 650. In this case, the possibility of confusing the waveform of turning over during sleep and the waveform at rest with the waveform of bruxism can be suppressed.

The sign information extraction unit 643 includes a heartbeat sign extraction unit 6431 that extracts the sign information from the heartbeat information, and an autonomic nervous system sign extraction unit 6432 that extracts the sign information from the autonomic nervous system information. The heart rate of the user may increase before the user engages in bruxism. The heartbeat sign extraction unit 6431 extracts, as the sign information, an increase in heart rate, which occurs before the onset of bruxism, from the heartbeat information. More specifically, the heartbeat sign extraction unit 6431 extracts, as the sign information, an increase in the heart rate of the user a predetermined time before the candidate waveform from the heartbeat information.

The sympathetic nervous system of the user may become hyperactive before the user engages in bruxism. The autonomic nervous system sign extraction unit 6432 extracts, as the sign information, an increase in the exchange nervous system index, which occurs before the onset of bruxism, from the autonomic nervous system information. More specifically, the autonomic nervous system sign extraction unit 432 extracts, as the sign information, an increase in the sympathetic nervous system index of the user a predetermined time before the candidate waveform from the autonomic nervous system information.

The bruxism determination unit 644 specifies the candidate waveform as a waveform caused by bruxism, based on the extraction of the sign information. When the sign information is not extracted, the bruxism determination unit 644 may refer to the waveform storage unit 650, and specify the candidate waveform as a waveform caused by bruxism by comparing the bruxism waveform and the candidate waveform stored in advance with each other. When the candidate waveform is specified as a waveform caused by bruxism, the bruxism determination unit 644 outputs the candidate waveform to the waveform storage unit 650 as a new bruxism waveform. The waveform storage unit 650 stores the output new bruxism waveform as a past bruxism waveform.

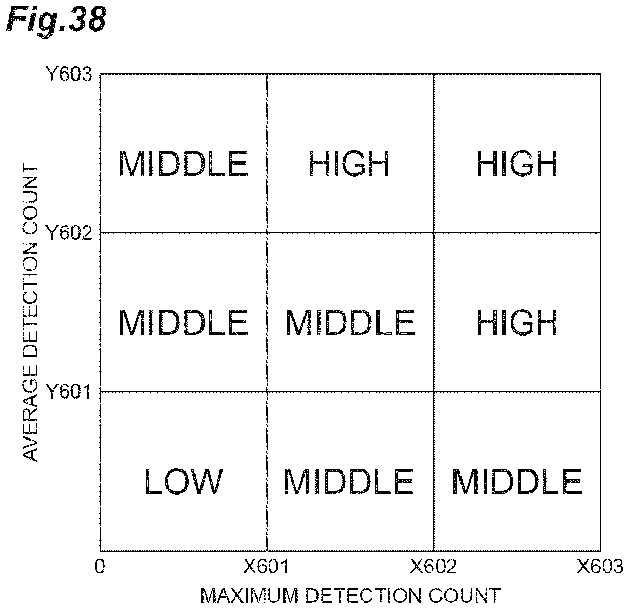

The bruxism determination unit 644 determines, for example, the level of occurrence frequency of bruxism. FIG. 38 is a schematic view showing one example of criteria for the level of occurrence frequency of bruxism. The terms "high", "medium", and "low" shown in FIG. 38 indicate the level of occurrence frequency of bruxism. In the two-dimensional graph shown in FIG. 38, the horizontal axis represents the maximum detection count. When sections are generated by dividing the time period during which the user sleeps at predetermined intervals, the maximum detection count is the maximum number of times of bruxism detected per section.

The "one section" described above is a unit time obtained by dividing the sleep time at regular time intervals, and is one hour as one example. In this case, for example, when the sleep time of the user is from 10:00 p.m. to 6:00 a.m. the next morning, the "one section" is 10:00 p.m. to 11:00 p.m., 11:00 p.m. to 12:00 a.m., 12:00 a.m. to 1:00 a.m., and so on up to 5:00 a.m. to 6:00 a.m. For example, when the duration of the waveform specified as bruxism is equal to or longer than two seconds, a single occurrence of bruxism is detected. In the graph shown in FIG. 38, the vertical axis represents the average detection count. The average detection count is the average number of times of bruxism detected per section.

"X601", "X602", "X603", "Y601", "Y602" and "Y603" are predetermined natural numbers. As one example, X601 is 5, X602 is 10, and X603 is 15. As one example, Y601 is 2, Y602 is 4, and Y603 is 6. The values of X601 to X603 and Y601 to Y603 may be changeable values. The bruxism determination unit 644 outputs the level of occurrence frequency of bruxism to the display unit 620 included in the bruxism detection system 601.

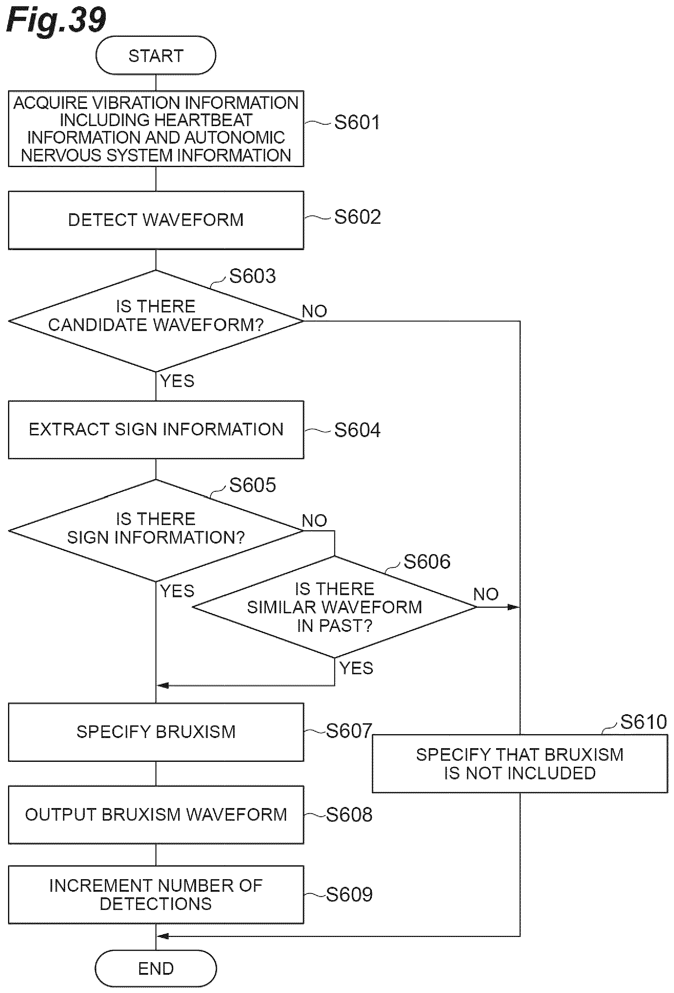

One example of the operation of the bruxism detection system 601 will be described. FIG. 39 is a flowchart showing one example of the operation of the bruxism detection system 601. Before the bruxism detection system 601 is operated, the sensor unit 611 is attached to the cushion body 610. The sensor unit 611 is attached to the cushion body 610 such that the sensor unit 611 does not come into contact with the user when the body of the user is placed on the cushion body 610. For example, the sensor unit 611 is disposed between the core material 602 and the cover fabric 603. The body of the user is laid on the cushion body 610 to which the sensor unit 611 is attached. The body of the user is placed, for example, on the cushion body 610 so as to come into contact with the front fabric 631 of the cover fabric 603.

The sensor unit 611 acquires vibration information including the heartbeat information and the autonomic nervous system information from vibration caused by the body movement of the user (step S601). In step S601, the sensor unit 611 acquires the vibration information indicating vibration of the user. The sensor unit 611 outputs, for example, a signal indicating a change in the load applied to the piezoelectric sensor by the user to the outside of the sensor unit 611. The detection unit 641 receives the signal output by the sensor unit 611. The detection unit 641 extracts the heartbeat information and the autonomic nervous system information from the vibration information received as a signal.

The waveform detection unit 642 detects the vibration information as a waveform by performing waveform conversion on the signal output from the sensor unit 611 (step S602).

The waveform detection unit 642 determines whether the detected waveform includes a candidate waveform (step S603). In step S603, the waveform detection unit 642 extracts the candidate waveform by confirming that the detected waveform includes the characteristics of the schematic waveform of bruxism and that the detected waveform does not include the characteristics of the schematic waveform of turning over during sleep and the characteristics of the schematic waveform at rest.

When the waveform detection unit 642 determines that the detected waveform includes the candidate waveform (S603: YES), the sign information extraction unit 643 extracts sign information from the vibration information (step S604). In step S604, the heartbeat sign extraction unit 6431 extracts sign information from the heartbeat information immediately before the time the candidate waveform is detected. The autonomic nervous system sign extraction unit 6432 extracts sign information from the autonomic nervous system information immediately before the time the candidate waveform is detected.

The bruxism determination unit 44 determines whether the sign information is included in the vibration information (step S605). In step S605, the bruxism determination unit 644 determines both whether the heartbeat sign extraction unit 6431 extracts the sign information from the heartbeat information and whether the autonomic nervous system sign extraction unit 6432 extracts the sign information from the autonomic nervous system information.

When the bruxism determination unit 644 determines that the sign information is not included (S605: NO), the bruxism determination unit 644 compares the candidate waveform with a past bruxism waveform stored in advance (step S606). In step S606, the bruxism determination unit 644 determines whether the candidate waveform has the characteristics of the past bruxism waveforms stored in advance.

When the bruxism determination unit 644 determines that the sign information is included (S605: YES), the bruxism determination unit 644 specifies the candidate waveform as a waveform caused by bruxism (step S607). As described above, the detection unit 641 detects bruxism from the vibration information and the sign information.

When the bruxism determination unit 644 determines that the candidate waveform has the characteristics of the past bruxism waveforms stored in advance (S606: YES), the bruxism determination unit 644 specifies the candidate waveform as a waveform caused by bruxism (step S607).

The bruxism determination unit 644 outputs the specified bruxism waveform (step S608). In step S608, the bruxism determination unit 644 outputs the candidate waveform to the waveform storage unit 650 as a new bruxism waveform.

The bruxism determination unit 644 increments the number of detections of bruxism by one (step S609). In step S609, the bruxism determination unit 644 increments the number of detections by one in association with the time the candidate waveform is detected. For example, when a candidate waveform is detected between 3:00 a.m. and 4:00 a.m. (one section) and the candidate waveform is specified as a waveform caused by bruxism, the number of times of bruxism is incremented by one in one section from 3:00 a.m. and 4:00 a.m.

While the user sleeps, the bruxism determination unit 644 counts the number of detections. When the user awakens, the bruxism determination unit 644 calculates the average detection count and the maximum detection count of the number of times of bruxism based on the incremented number of times of bruxism for each section. The bruxism determination unit 644 outputs the calculated average detection count and maximum detection count to the display unit 620. The display unit 620 displays the two-dimensional graph shown in FIG. 38, based on the average detection count and the maximum detection count output from the bruxism determination unit 644. The display unit 620 displays the occurrence frequency of bruxism of the user by plotting points corresponding to the maximum detection count and the average detection count of the user on the two-dimensional graph shown in FIG. 38.

When the waveform detection unit 642 determines that the candidate waveform is not included in the vibration information (S603: NO), or when the bruxism determination unit 644 determines that the candidate waveform does not have the characteristics of the past bruxism waveforms stored in advance (S606: NO), the bruxism determination unit 644 specifies that the detected waveform does not include bruxism (step S610). Incidentally, the contents and order of each step of the operation of the bruxism detection system 601 are not limited to the above-described example, and can be changed as appropriate.

As one example, actions and effects of the bruxism detection system 601 will be described. In the bruxism detection system 601, the sensor unit 611 acquires the vibration information indicating vibration of the user without coming into contact with the user. Since bruxism is detected in a state where the user is not in contact with the sensor unit 611, the burden on the user when bruxism is detected can be reduced. The detection unit 641 detects bruxism from the vibration information indicating vibration of the user and the sign information indicating a sign of bruxism occurring before the onset of bruxism. Since the vibration information includes vibration of bruxism itself, bruxism can be detected from the vibration information. Since bruxism is detected by taking into account the vibration information as well as the sign information that is a sign occurring before the onset of bruxism, the certainty that vibration in the vibration information is caused by bruxism can be improved by using the sign information. Therefore, bruxism can be detected with high accuracy while reducing the burden on the user.

Generally, there are few means for measuring bruxism that can be used on a daily basis. The bruxism detection system 601 can measure the presence or absence and frequency of bruxism on a daily basis by detecting vibration specific to bruxism in a non-contact manner.

A computer that executes the bruxism detection application 640 detects bruxism from the vibration information indicating vibration of the user and the sign information indicating a sign of bruxism occurring before the onset of bruxism. Since the vibration information includes vibration of bruxism itself, bruxism can be detected from the vibration information. Since bruxism is detected by taking into account the vibration information as well as the sign information, the certainty that vibration in the vibration information is caused by bruxism can be improved by using the sign information. Therefore, bruxism can be detected with high accuracy.

The detection unit 641 extracts the heartbeat information indicating the heartbeat of the user from the vibration information, and the heartbeat sign extraction unit 6431 included in the detection unit 641 extracts, as the sign information, an increase in heart rate, which occurs before the onset of bruxism, from the heartbeat information. The heart rate of the user may increase before the user engages in bruxism. Since the heartbeat sign extraction unit 6431 extracts, as the sign information, an increase in heart rate occurring before the onset of bruxism, the certainty that vibration in the vibration information is caused by bruxism can be improved by using the increase in heart rate.

The detection unit 641 extracts the autonomic nervous system information indicating the state of the autonomic nervous system of the user from the vibration information, and the autonomic nervous system sign extraction unit 6432 included in the detection unit 641 extracts, as the sign information, an increase in the sympathetic nervous system index, which occurs before the onset of bruxism, from the autonomic nervous system information. The sympathetic nervous system of the user may become hyperactive before the user engages in bruxism. Since the autonomic nervous system sign extraction unit 6432 extracts, as the sign information, an increase in the sympathetic nervous system index occurring before the onset of bruxism, the certainty that the detected vibration is caused by bruxism can be improved.

The waveform detection unit 642 included in the detection unit 641 detects the vibration information as a waveform, and the bruxism determination unit 644 specifies bruxism by comparing the detected waveform with the bruxism waveforms stored in advance. In this case, by using the bruxism waveform that is detected in advance and that is detected from the vibration information and the sign information with high accuracy, it is specified whether the detected waveform is caused by bruxism. Therefore, the certainty that the detected waveform is caused by bruxism can be improved.

The specific examples of the health risk determination system, the autonomic nervous system determination system, the life improvement system, the sleeping posture determination system, the sleeping posture determination program, the bruxism detection system, and the bruxism detection program according to the present disclosure have been described above. However, the configuration and functions of each of the health risk determination system, the autonomic nervous system determination system, the life improvement system, the sleeping posture determination system, the sleeping posture determination program, the bruxism detection system, and the bruxism detection program according to the present disclosure are not limited to the above-described specific examples, and may be further modified without departing from the scope of the concepts described in the claims.

The present invention may be a combination of a part of the health risk determination system, the autonomic nervous system determination system, the life improvement system, the sleeping posture determination system, the sleeping posture determination program, the bruxism detection system, and the bruxism detection program in the above-described specific examples and the remainder of the health risk determination system, the autonomic nervous system determination system, the life improvement system, the sleeping posture determination system, the sleeping posture determination program, the bruxism detection system, and the bruxism detection program in the above-described specific examples. The health risk determination system, the autonomic nervous system determination system, the life improvement system, the sleeping posture determination system, the sleeping posture determination program, the bruxism detection system, and the bruxism detection program in the above-described specific examples may be capable of being combined with each other. Some or all of the above-described embodiments are represented by (Supplementary Note 1) to (Supplementary Note 28) to be described below, but are not limited to the following descriptions.

### (Supplementary Note 1)

A health risk determination system that determines a health risk caused by an abnormal breathing state of a user from a sleep onset time to an awakening time, comprising:
a sensor unit that acquires breathing information, which is information indicating a breathing of the user, without coming into contact with the user;
an abnormality detection unit that detects the abnormal breathing state of the user from the breathing information;
a unit count acquisition unit that acquires the number of detections of the abnormal breathing state for each unit time period having a predetermined time span between the sleep onset time and the awakening time;
an average count acquisition unit that acquires an average detection count that is a value obtained by dividing a total value of the number of detections of the abnormal breathing state between the sleep onset time and the awakening time by a time from the sleep onset time to the awakening time;
a maximum count acquisition unit that acquires a maximum detection count that is a maximum value among a plurality of numbers of detections acquired for each unit time period; and
a health risk determination unit that determines the health risk of the user based on both the average detection count and the maximum detection count.

### (Supplementary Note 2)

The health risk determination system according to Supplementary Note 1,
wherein the sensor unit acquires body movement information that is information indicating a body movement of the user, and heartbeat information that is information indicating a heartbeat of the user, and
the abnormality detection unit detects the abnormal breathing state from the body movement information and the heartbeat information.

### (Supplementary Note 3)

The health risk determination system according to Supplementary Note 2, further comprising:
a sleep stage acquisition unit that determines a sleep stage of the user based on the breathing information, the body movement information, and the heartbeat information,
wherein the health risk determination unit determines the health risk of the user based on a detection result of the abnormality detection unit and the sleep stage.

### (Supplementary Note 4)

The health risk determination system according to Supplementary Note 3,
wherein the breathing information includes a signal indicating a body movement associated with the breathing of the user,
a first amplifier and a second amplifier that amplify the signal and output the amplified signal to the sleep stage acquisition unit are further provided, and
a gain of the first amplifier is larger than a gain of the second amplifier.

### (Supplementary Note 5)

An autonomic nervous system determination system that determines a state of an autonomic nervous system of a user from a bedtime to a wake-up time, comprising:
a sensor unit that acquires at least one of breathing information that is information indicating a breathing of the user, body movement information that is information indicating a body movement of the user, and brainwave information that is information indicating a brainwave of the user, and heartbeat information that is information indicating a heartbeat of the user;
an autonomic nervous system acquisition unit that acquires autonomic nervous system information, which is information indicating the state of the autonomic nervous system of the user, from the heartbeat information;
a sleep state acquisition unit that acquires a sleep state of the user from at least one of the breathing information, the body movement information, the heartbeat information, and the brainwave information; and
an autonomic nervous system determination unit that determines the state of the autonomic nervous system of the user based on both the autonomic nervous system information and the sleep state.

### (Supplementary Note 6)

The autonomic nervous system determination system according to Supplementary Note 5, further comprising:
a time period determination unit that determines a detection target time period during which the body movement of the user does not occur between the bedtime and the wake-up time, based on the body movement information,
wherein the autonomic nervous system determination unit determines the state of the autonomic nervous system of the user in the detection target time period.

### (Supplementary Note 7)

The autonomic nervous system determination system according to Supplementary Note 5 or 6, further comprising:
a device control unit that controls an operation of a device, which constitutes an environment around the user, using a determination result of the autonomic nervous system determination unit.

### (Supplementary Note 8)

The autonomic nervous system determination system according to any one of Supplementary Notes 5 to 7, further comprising:
a support information acquisition unit that acquires support information, which is information that supports a life of the user, using a determination result of the autonomic nervous system determination unit; and
a display unit that displays the support information.

### (Supplementary Note 9)

A life improvement system for improving a life of a user, comprising:
a sensor unit that acquires at least one of breathing information that is information indicating a breathing of the user, body movement information that is information indicating a body movement of the user, and heartbeat information that is information indicating a heartbeat of the user;
a sleep state acquisition unit that acquires sleep state information, which is information indicating a sleep state of the user, from at least one of the breathing information, the body movement information, and the heartbeat information;
an autonomic nervous system acquisition unit that acquires autonomic nervous system information, which is information indicating a state of an autonomic nervous system of the user, from the heartbeat information;
a device control unit that controls an operation of a device constituting an environment around the user, based on at least one of past sleep information, which is the sleep state information of the user in the past stored in advance, and the autonomic nervous system information;
a prediction information generation unit that generates prediction information that is information indicating a predicted state of the user during wakefulness, based on at least one of the past sleep information and the autonomic nervous system information; and
an improvement information generation unit that generates improvement information that is information that improves the life of the user, based on the prediction information.

### (Supplementary Note 10)

The life improvement system according to Supplementary Note 9,
wherein the sensor unit includes a cushion sensor attached to a cushion on which the user is seated.

### (Supplementary Note 11)

The life improvement system according to Supplementary Note 9 or 10,
wherein the prediction information includes mental information that is information indicating a mental state of the user, physical condition information that is information indicating a physical condition of the user, and brain information that is information indicating a state of a brain of the user.

### (Supplementary Note 12)

The life improvement system according to Supplementary Note 11,
wherein the physical condition information includes skin information indicating a state of a skin of the user.

### (Supplementary Note 13)

The life improvement system according to any one of Supplementary Notes 9 or 12,
wherein the improvement information generation unit generates the improvement information based on subjective information that is information indicating a subjective evaluation of the user.

### (Supplementary Note 14)

The life improvement system according to any one of Supplementary Notes 9 or 13,
wherein the improvement information includes information indicating a type of clothing recommended to the user.

### (Supplementary Note 15)

The life improvement system according to any one of Supplementary Notes 9 or 14,
wherein the improvement information includes information indicating a type of food recommended to the user.

### (Supplementary Note 16)

The life improvement system according to any one of Supplementary Notes 9 or 15,
wherein the improvement information includes information indicating a type of cosmetic recommended to the user.

### (Supplementary Note 17)

The life improvement system according to any one of Supplementary Notes 9 or 16,
wherein the device include a pillow used by the user during sleep, and
the device control unit controls an angle of a placement surface of the pillow, on which a head of the user is placed, with respect to a horizontal plane.

### (Supplementary Note 18)

A life improvement system for improving a life of a user, comprising:
a sensor unit that acquires heartbeat information that is information indicating a heartbeat of the user;
an autonomic nervous system acquisition unit that acquires autonomic nervous system information, which is information indicating a state of an autonomic nervous system of the user, from the heartbeat information;
a prediction information generation unit that generates prediction information that is information indicating a predicted state of the user during wakefulness, based on the autonomic nervous system information; and
an improvement information generation unit that generates improvement information that is information that improves the life of the user, based on the prediction information.

### (Supplementary Note 19)

A sleeping posture determination system, comprising:
a sensor unit that is mounted on bedding and that receives a load of a body of a user of the bedding placed on the bedding and outputs a waveform corresponding to the load;
a sleeping posture determination unit that determines a sleeping posture of the user based on the waveform output by the sensor unit; and
an advice generation unit that generates advice for improving the sleeping posture of the user based on the sleeping posture determined by the sleeping posture determination unit.

### (Supplementary Note 20)

The sleeping posture determination system according to Supplementary Note 19,
wherein the sensor unit acquires, as the waveform, at least one of breathing information that is information indicating a breathing of the user, body movement information that is information indicating a body movement of the user, and heartbeat information that is information indicating a heartbeat of the user, and
a sleep state acquisition unit that acquires a sleep state, which is information indicating a sleep state of the user, from the waveform indicating at least one of the breathing information, the body movement information, and the heartbeat information, and a display unit that displays the advice generated by the advice generation unit and the sleep state acquired by the sleep state acquisition unit are provided.

### (Supplementary Note 21)

The sleeping posture determination system according to Supplementary Note 19 or 20,
wherein the sensor unit acquires, as a waveform, heartbeat information that is information indicating a heartbeat of the user,
an autonomic nervous system acquisition unit that acquires autonomic nervous system information, which is information indicating a state of an autonomic nervous system of the user, from the heartbeat information is provided, and
the advice generation unit generates the advice including the autonomic nervous system information acquired by the autonomic nervous system acquisition unit.

### (Supplementary Note 22)

The sleeping posture determination system according to any one of Supplementary Notes 19 to 21,
wherein the sensor unit acquires, as the waveform, breathing information that is information indicating a state of a breathing of the user,
an apnea state acquisition unit that detects a state of apnea of the user from the breathing information is provided, and
the advice generation unit generates the advice including the state of apnea acquired by the apnea state acquisition unit.

### (Supplementary Note 23)

A non-transitory computer-readable storage medium storing a sleeping posture determination program, the program causing a computer to execute:
a step of determining a sleeping posture of a user of bedding based on a waveform indicating a load of the user output by a sensor unit mounted on the bedding; and
a step of generating advice for improving the sleeping posture of the user based on the sleeping posture determined by the step of determining.

### (Supplementary Note 24)

A bruxism detection system that detects bruxism of a user from a sleep onset time to an awakening time, comprising:
a sensor unit that acquires vibration information, which is information indicating vibration of the user, without coming into contact with the user; and
a detection unit that extracts sign information indicating a sign occurring before on an onset of the bruxism from the vibration information, and that detects the bruxism from the vibration information and the sign information.

### (Supplementary Note 25)

The bruxism detection system according to Supplementary Note 24,
wherein the detection unit extracts heartbeat information indicating a heartbeat of the user from the vibration information, and
the detection unit extracts, as the sign information, an increase in heart rate occurring before the onset of the bruxism from the heartbeat information.

### (Supplementary Note 26)

The bruxism detection system according to Supplementary Note 24 or 25,
wherein the detection unit extracts autonomic nervous system information indicating a state of an autonomic nervous system of the user from the vibration information, and
the detection unit extracts, as the sign information, an increase in a sympathetic nervous system index occurring before the onset of the bruxism from the autonomic nervous system information.

### (Supplementary Note 27)

The bruxism detection system according to any one of Supplementary Notes 24 to 26,
wherein the detection unit detects the vibration information as a waveform, and specifies the bruxism by comparing the detected waveform with a bruxism waveform stored in advance.

### (Supplementary Note 28)

A non-transitory computer-readable storage medium storing a bruxism detection program, the program causing a computer to execute:
a step of extracting sign information indicating a sign occurring before an onset of bruxism from vibration information indicating vibration of a user acquired by a sensor unit; and
a step of detecting the bruxism from the vibration information and the sign information.

At least one of a sleep state improvement system, a sleep state improvement device, and a sleeping posture determination program may be further combined with the health risk determination system, the autonomic nervous system determination system, the life improvement system, the sleeping posture determination system, the sleeping posture determination program, the bruxism detection system, and the bruxism detection program described above. Examples of the sleep state improvement system, the sleep state improvement device, and sleep state improvement program include (Supplementary Note 29) to (Supplementary Note 36) below.

### (Supplementary Note 29)

A sleep state improvement system, comprising:
a sensor unit that acquires at least one of breathing information that is information indicating a breathing of the user, body movement information that is information indicating a body movement of the user, and heartbeat information that is information indicating a heartbeat of the user;
a sleep state acquisition unit that acquires a sleep state of the user from at least one of the breathing information, the body movement information, and the heartbeat information;
a sleep state improvement device constituting an environment around the user; and
a device control unit that controls brightness and fragrance of the environment by controlling an operation of the sleep state improvement device based on a past sleep state that is the sleep state of the user in the past acquired in advance by the sleep state acquisition unit.

### (Supplementary Note 30)

The sleep state improvement system according to Supplementary Note 29,
wherein the sleep state acquisition unit acquires a sleep onset state of the user from a time from a bedtime to a sleep onset time, and acquires an awake state of the user from a time from an awakening time to a wake-up time, and
the device control unit controls the operation of the sleep state improvement device based on the sleep onset state and the awake state acquired by the sleep state acquisition unit when the user has previously slept.

### (Supplementary Note 31)

The sleep state improvement system according to Supplementary Note 29 or 30,
wherein the sleep state acquisition unit acquires a nocturnal awakening time from the sleep state between a sleep onset time and an awakening time, and
the device control unit controls the operation of the sleep state improvement device based on the nocturnal awakening time acquired by the sleep state acquisition unit when the user has previously slept.

### (Supplementary Note 32)

The sleep state improvement system according to Supplementary Note 30,
wherein the sleep state acquisition unit acquires the awake state from the sleep state a certain time before the awakening time.

### (Supplementary Note 33)

The sleep state improvement system according to any one of Supplementary Notes 29 or 32,
wherein the device control unit controls the operation of the sleep state improvement device according to a control pattern of brightness and fragrance set in advance.

### (Supplementary Note 34)

The sleep state improvement system according to Supplementary Note 33,
wherein the device control unit controls the operation of the sleep state improvement device according to a control pattern of at least one of airflow and sound set in advance.

### (Supplementary Note 35)

A sleep state improvement device that operates according to a sleep state acquired from at least one of breathing information that is information indicating a breathing of the user, body movement information that is information indicating a body movement of the user, and heartbeat information that is information indicating a heartbeat of the user, and that constitutes an environment around the user, comprising:
a lighting unit that controls brightness of the environment;
a fragrance generation unit that controls fragrance of the environment; and
a control unit that controls the lighting unit and the fragrance generation unit,
wherein the control unit controls the lighting unit and the fragrance generation unit based on a past sleep state that is the sleep state of the user in the past.

### (Supplementary Note 36)

A non-transitory computer-readable storage medium storing a sleep state improvement program, the program causing a computer to execute:
a step of acquiring a sleep state of a user from at least one of breathing information that is information indicating a breathing of the user, body movement information that is information indicating a body movement of the user, and heartbeat information that is information indicating a heartbeat of the user; and
a step of controlling brightness and fragrance of an environment around the user by controlling an operation of a sleep state improvement device constituting the environment, based on a past sleep state that is the sleep state of the user in the past acquired in advance in the step of acquiring.

### Reference Signs List

1: health risk determination system, 2: core material, 3: cover fabric, 10: cushion body, 11: sensor unit, 12: first amplifier, 13: second amplifier, 14: sleep stage acquisition unit, 15: abnormality detection unit, 16: unit count acquisition unit, 17: average count acquisition unit, 18: maximum count acquisition unit, 19: health risk determination unit, 20: display unit, 21: upper surface, 22: lower surface, 23: side surface, 31: front fabric, 32: back fabric, 33: opening and closing member, 34: slide member, 35: opening and closing portion, 40: health risk determination application, 42: display unit, 44: determination unit, 50: waveform storage unit, 82: output screen, 101: autonomic nervous system determination system, 102: core material, 103: cover fabric, 110: cushion body, 111: sensor unit, 112: autonomic nervous system acquisition unit, 113: sleep state acquisition unit, 114: time period determination unit, 115: autonomic nervous system determination unit, 116: device control unit, 117: support information acquisition unit, 118: display unit, 120: autonomic nervous system determination application, 121: upper surface, 122: lower surface, 123: side surface, 131: front fabric, 132: back fabric, 133: opening and closing member, 134: slide member, 135: opening and closing portion, 201: server, 202: device, 301: life improvement system, 302: core material, 303: cover fabric, 310a: mattress, 310b: cushion, 311: sensor unit, 312: sensor sheet, 313: cushion sensor, 313a: seating portion sensor, 313b: sacrum support portion sensor, 313c: thigh support portion sensor, 314: core material, 315: seating portion, 315a: buttock support portion, 315b: thigh support portion, 316: lumbar support portion, 316a: general portion, 316b: sacrum support portion, 321: upper surface, 322: lower surface, 323: side surface, 331: front fabric, 332: back fabric, 333: opening and closing member, 334: slide member, 335: opening and closing portion, 340: life improvement application, 341: sleep state acquisition unit, 342: autonomic nervous system acquisition unit, 343: storage unit, 344: device control unit, 345: prediction information generation unit, 346: improvement information generation unit, 380: input screen, 381: sleep evaluation input portion, 382: output screen, 383: prediction information display portion, 384: improvement information display portion, 385: output screen, 386: subjective information display portion, 387: objective information display portion, 388: feedback display portion, 401: information terminal, 402: life improvement server, 403: website, 404: device, 405: device server, 432: autonomic nervous system sign extraction unit, 501: bedding, 502: core material, 502b: upper surface, 502c: lower surface, 502d: side surface, 503: cover fabric, 503b: upper fabric, 503c: lower fabric, 503d: opening and closing member, 503f: slider, 503g: element, 503h: fixed portion, 503s: snap button, 504: opening, 511: sensor unit, 511b: long side, 511c: short side, 512: sheet-shaped fabric, 512b: inner fabric, 512c: outer fabric, 513: sensor, 514: power supply unit, 514b: cable, 514c: plug, 514d: connector, 515: thread-shaped sensor, 515b: curved portion, 515c: linear portion, 515d: first wave-shaped portion, 515f: second wave-shaped portion, 515h: first peak portion, 515j: first valley portion, 515k: second peak portion, 515p: second valley portion, 515q: first linear portion, 515r: second linear portion, 516: data acquisition unit, 516b: electrical function portion, 516c: attachment member, 516d: screw, 516f: housing, 516h: socket, 516j: plate-shaped portion, 516k: protruding portion, 516p: through-hole, 517: fixing portion, 518: fixing portion, 519: snap button, 520: sleeping posture determination system, 521: information terminal, 521b: display, 522: sleep state improvement device, 540: sleeping posture determination program, 541: storage unit, 542: display unit, 543: communication unit, 551: waveform detection unit, 552: body movement acquisition unit, 553: breathing acquisition unit, 554: heartbeat acquisition unit, 555: turnover determination unit, 556: sleeping posture determination unit, 557: sleep state acquisition unit, 558: advice generation unit, 558b: alert output unit, 558c: product suggestion unit, 559: device control unit, 560: autonomic nervous system acquisition unit, 561: apnea state acquisition unit, 562: sleeping posture status generation unit, 601: detection system, 602: core material, 603: cover fabric, 610: cushion body, 611: sensor unit, 620: display unit, 621: upper surface, 622: lower surface, 623: side surface, 631: front fabric, 632: back fabric, 633: opening and closing member, 634: slider, 635: element, 640: detection application, 641: detection unit, 642: waveform detection unit, 643: sign information extraction unit, 644: determination unit, 650: waveform storage unit, 700: information terminal, 6431: heartbeat sign extraction unit, 6432: autonomic nervous system sign extraction unit, B, B501, B502, B503, B504, B505, B506: advice, C: sleep state, C501, C502: sleep state, DB101: support database, DB102: autonomic nervous system database, L: center line, P, P101, P102: point, R101: sympathetic region, R102: parasympathetic region, R103: antagonistic region, W501, W502, W503: waveform.

## Claims

1. A health risk determination system that determines a health risk caused by an abnormal breathing state of a user from a sleep onset time to an awakening time, comprising:
a sensor unit that acquires breathing information, which is information indicating a breathing of the user, without coming into contact with the user;
an abnormality detection unit that detects the abnormal breathing state of the user from the breathing information;
a unit count acquisition unit that acquires the number of detections of the abnormal breathing state for each unit time period having a predetermined time span between the sleep onset time and the awakening time;
an average count acquisition unit that acquires an average detection count that is a value obtained by dividing a total value of the number of detections of the abnormal breathing state between the sleep onset time and the awakening time by a time from the sleep onset time to the awakening time;
a maximum count acquisition unit that acquires a maximum detection count that is a maximum value among a plurality of numbers of detections acquired for each unit time period; and
a health risk determination unit that determines the health risk of the user based on both the average detection count and the maximum detection count.

2. An autonomic nervous system determination system that determines a state of an autonomic nervous system of a user from a bedtime to a wake-up time, comprising:
a sensor unit that acquires at least one of breathing information that is information indicating a breathing of the user, body movement information that is information indicating a body movement of the user, and brainwave information that is information indicating a brainwave of the user, and heartbeat information that is information indicating a heartbeat of the user;
an autonomic nervous system acquisition unit that acquires autonomic nervous system information, which is information indicating the state of the autonomic nervous system of the user, from the heartbeat information;
a sleep state acquisition unit that acquires a sleep state of the user from at least one of the breathing information, the body movement information, the heartbeat information, and the brainwave information; and
an autonomic nervous system determination unit that determines the state of the autonomic nervous system of the user based on both the autonomic nervous system information and the sleep state.

3. A life improvement system for improving a life of a user, comprising:
a sensor unit that acquires at least one of breathing information that is information indicating a breathing of the user, body movement information that is information indicating a body movement of the user, and heartbeat information that is information indicating a heartbeat of the user;
a sleep state acquisition unit that acquires sleep state information, which is information indicating a sleep state of the user, from at least one of the breathing information, the body movement information, and the heartbeat information;
an autonomic nervous system acquisition unit that acquires autonomic nervous system information, which is information indicating a state of an autonomic nervous system of the user, from the heartbeat information;
a device control unit that controls an operation of a device constituting an environment around the user, based on at least one of past sleep information, which is the sleep state information of the user in the past stored in advance, and the autonomic nervous system information;
a prediction information generation unit that generates prediction information that is information indicating a predicted state of the user during wakefulness, based on at least one of the past sleep information and the autonomic nervous system information; and
an improvement information generation unit that generates improvement information that is information that improves the life of the user, based on the prediction information.

4. A life improvement system for improving a life of a user, comprising:
a sensor unit that acquires heartbeat information that is information indicating a heartbeat of the user;
an autonomic nervous system acquisition unit that acquires autonomic nervous system information, which is information indicating a state of an autonomic nervous system of the user, from the heartbeat information;
a prediction information generation unit that generates prediction information that is information indicating a predicted state of the user during wakefulness, based on the autonomic nervous system information; and
an improvement information generation unit that generates improvement information that is information that improves the life of the user, based on the prediction information.

5. A sleeping posture determination system, comprising:
a sensor unit that is mounted on bedding and that receives a load of a body of a user of the bedding placed on the bedding and outputs a waveform corresponding to the load;
a sleeping posture determination unit that determines a sleeping posture of the user based on the waveform output by the sensor unit; and
an advice generation unit that generates advice for improving the sleeping posture of the user based on the sleeping posture determined by the sleeping posture determination unit.

6. A non-transitory computer-readable storage medium storing a sleep state improvement program, the program causing a computer to execute:
a step of determining a sleeping posture of a user of bedding based on a waveform indicating a load of the user output by a sensor unit mounted on the bedding; and
a step of generating advice for improving the sleeping posture of the user based on the sleeping posture determined by the step of determining.

7. A bruxism detection system that detects bruxism of a user from a sleep onset time to an awakening time, comprising:
a sensor unit that acquires vibration information, which is information indicating vibration of the user, without coming into contact with the user; and
a detection unit that extracts sign information indicating a sign occurring before on an onset of the bruxism from the vibration information, and that detects the bruxism from the vibration information and the sign information.

8. A non-transitory computer-readable storage medium storing a bruxism detection program, the program causing a computer to execute:
a step of extracting sign information indicating a sign occurring before an onset of bruxism from vibration information indicating vibration of a user acquired by a sensor unit; and
a step of detecting the bruxism from the vibration information and the sign information.
